# EUROPEAN PATENT SPECIFICATION

(11) **EP 3 957 733 B1**
(45) Date of publication and mention of the grant of the patent: **15.01.2025**
(21) Application number: 20791659.4
(22) Date of filing: 16.04.2020
(51) Int. Cl.: C12N 15/09, C12N 15/11, C12Q 1/6834, C12Q 1/6839, C12Q 1/6853, C12Q 1/6869, C12Q 1/6876, C12Q 1/70, G01N 33/50, G01N 33/53, G01N 33/569, C12Q 1/682

(54) **SIMPLE METHOD FOR DETECTING NUCLEIC ACID SEQUENCE, ETC**
EINFACHES VERFAHREN ZUM NACHWEIS VON NUKLEINSÄURESEQUENZEN ETC.
PROCÉDÉ SIMPLE POUR LA DÉTECTION D'UNE SÉQUENCE D'ACIDE NUCLÉIQUE, ETC

(30) Priority: 19.04.2019 JP 2019080312; 07.06.2019 JP 2019107358; 29.11.2019 JP 2019217075
(43) Date of publication of application: 23.02.2022
(73) Proprietor: Nihon University, Tokyo 102-8275 (JP)
(72) Inventor: KUWAHARA, Masayasu, Tokyo 102-8275 (JP); FUJITA, Hiroto, Tokyo 102-8275 (JP)
(74) Representative: Gill Jennings & Every LLP
(86) International application number: PCT/JP2020/016793
(87) International publication number: WO 2020/213700

(56) References cited:
- WO-A1-2016/152936
- WO-A1-2016/152936
- WO-A1-2017/086245
- WO-A1-2018/020831
- WO-A1-2018/020831
- WO-A1-2018/168895
- WO-A1-2018/168895
- JP-A- 2010 193 884

## Description

### TECHNICAL FIELD

The present invention relates to kits for simply and efficiently detecting nucleic acids or short chain target nucleic acids, and detection methods using the kits. The present invention also relates to kits for simply and efficiently detecting a target molecule, and a detection method using the kit. The present invention also relates to a ThT-PEG-ThT compound.

### BACKGROUND ART

In recent years, development of methods targeting RNA molecules such as mRNAs and miRNAs (microRNAs) for detection of diseases and stresses is attracting attention. As methods for quantification and detection of RNA, methods using real-time PCR are known. However, their use for simple tests at clinics or for self-medication is difficult since they require expensive devices and high usage cost, as well as complicated operation.

On the other hand, a method in which RNA is detected by the rolling circle amplification method has been disclosed in Patent Document 1 (JP 2012-080871). However, this method enables only detection of a sequence at the 3'-end since the method uses analyte RNA as a primer. This method is insufficient also from the viewpoint of the amplification efficiency and the detection efficiency.

Under such a technical background, the present inventors reported a simple detection method for RNA sequences (Patent Document 2; WO 2016/152936). WO 2016/152936 describes a method and a kit for detecting a target nucleic acid involving RCA amplification of a single-stranded circular DNA and a primer. More specifically, the method detects a target RNA by hybridizing the target RNA with a single-stranded circular DNA and a primer to form a ternary complex, performing amplification from the primer by the rolling circle amplification (RCA) method, and then detecting a detection reagent-binding sequence (for example, guanine-quadruplex-containing sequence) contained in the amplification product using a detection reagent such as a thioflavin T (ThT) derivative.

The present inventors also reported a detection method for a target molecule (Patent Document 3, WO 2018/168895). More specifically, the method efficiently detects a non-nucleic acid molecule by the rolling circle amplification (RCA) method using a single-stranded circular DNA, a capture oligonucleotide, and a primer, wherein aptamer sequences that bind to the target non-nucleic acid molecule are included in the sequences of the capture oligonucleotide and the primer. WO 2018/168895 describes kits for detecting a target nucleic acid and methods for doing so involving the said kits. The kits and methods disclosed therein comprise a first and second single-stranded circular DNA as well as a first and second primer.

WO 2018/020831 relates to a method for detecting a gene mutation, comprising the steps of: hybridizing a target polynucleotide that contains a first region and a second region adjacent to the 3' side of the first region and having a mutation with single-stranded cyclic DNA and a primer, wherein the method carries out a nucleic acid amplification reaction based on the formation of a complex of the target polynucleotide, the primer and the single-stranded cyclic DNA.

### SUMMARY OF THE INVENTION

### PROBLEMS TO BE SOLVED BY THE INVENTION

The rolling circle amplification (RCA) methods (also called the SATIC methods) described in Patent Documents 2 and 3 using the single-stranded circular DNA, the capture oligonucleotide, and the primer have enabled efficient detection of a target nucleic acid or a target molecule, respectively, but further improvement has been demanded. Accordingly, an object of the present invention is to provide a kit and a method for more efficiently detecting a target nucleic acid or a target molecule by a simple method.

### MEANS FOR SOLVING THE PROBLEMS

In order to solve the above problem, the present inventors intensively studied. In both methods described in Patent Documents 2 and 3, two kinds of primers present in the free state in a solution are used. The present inventors discovered that the detection sensitivity for the target nucleic acid or the target molecule can be remarkably increased by improvement of these methods by using a primer(s) and/or a capture oligonucleotide bound to a carrier and controlling their ratios, thereby completing the present invention.

The invention is set out in the appended set of claims. More specifically, the present invention provides a nucleic acid detection kit comprising:
(i) a first single-stranded circular DNA containing:
   a sequence of 10 to 30 bases complementary to a first site of a target nucleic acid;
   a first-primer-binding sequence of 7 to 8 bases adjacent to the 5'-side of this sequence; and
   a sequence complementary to a sequence that binds to a second single-stranded circular DNA;
(ii) a first oligonucleotide primer containing:
   a sequence of 8 to 15 bases complementary to a second site adjacent to the 3'-side of the first site of the target nucleic acid; and
   a sequence of 7 to 8 bases adjacent to the 3'-side of this sequence and complementary to the first-primer-binding site of the first single-stranded circular DNA;
(iii) a second single-stranded circular DNA containing:
   the same sequence as the sequence in the first single-stranded circular DNA, complementary to the sequence that binds to the second single-stranded circular DNA; and
   a second-primer-binding sequence adj acent to the 5'-side of this sequence; and
(iv) a second oligonucleotide primer containing:
   the same sequence as the site in the first single-stranded circular DNA, adjacent to the 5'-side of the sequence complementary to the sequence that binds to the second single-stranded circular DNA, wherein said same sequence as the region in the first single-stranded circular DNA is 8 to 15 bases; and
   a sequence of 7 to 8 bases adjacent to the 3'-side of this sequence and complementary to the second-primer-binding sequence of the second single-stranded circular DNA,
   wherein
   the first oligonucleotide primer is bound to a carrier through the 5'-end thereof, and
   the second oligonucleotide primer is bound, through the 5'-end thereof, to the carrier to which the first oligonucleotide primer is bound.

In a preferred embodiment,
the first oligonucleotide primer is modified with biotin at the 5'-end thereof, and bound, through the biotin, to a carrier on which avidin is immobilized; and
the second oligonucleotide primer is modified with biotin at the 5'-end thereof, and bound, through the biotin, to the carrier to which the first oligonucleotide primer is bound.

In a preferred embodiment, the ratio between the first oligonucleotide primer and the second oligonucleotide primer bound to the carrier is 1:10 to 1:30 in terms of the molar ratio.

In a preferred embodiment, the nucleic acid detection kit comprises (v) a detection reagent, wherein the second single-stranded circular DNA contains a sequence complementary to a detection reagent-binding sequence.

In a preferred embodiment, the detection reagent-binding sequence is a guanine-quadruplex-forming sequence, and the detection reagent is a guanine-quadruplex-binding reagent.

In a preferred embodiment, the sequence complementary to the guanine-quadruplex-forming sequence contains a C₃N₁₋₁₀C₃N₁₋₁₀C₃N₁₋₁₀C₃ sequence.

In a preferred embodiment, the guanine-quadruplex-binding reagent contains a compound represented by the following General Formula (I): 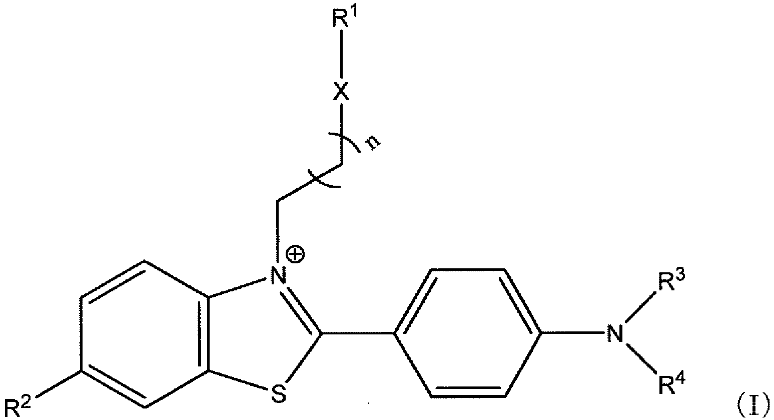 wherein
R¹ represents hydrogen, or a hydrocarbon group optionally containing one or more selected from O, S, and N,
R², R³, and R⁴ each independently represent a C₁-C₅ hydrocarbon group,
n represents an integer of 0 to 5, and
X represents O, S, or NH.

In a preferred embodiment, the compound represented by General Formula (I) is represented by the following Formula (II) or (III).

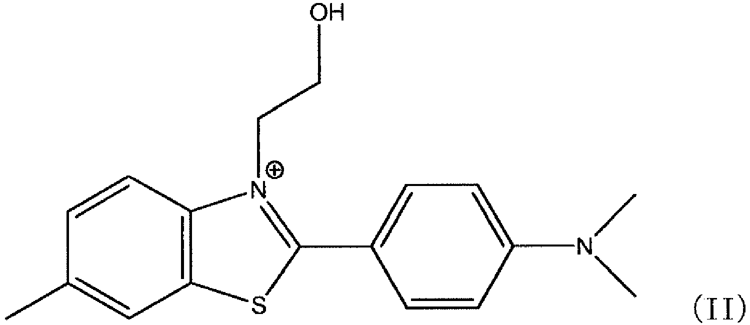

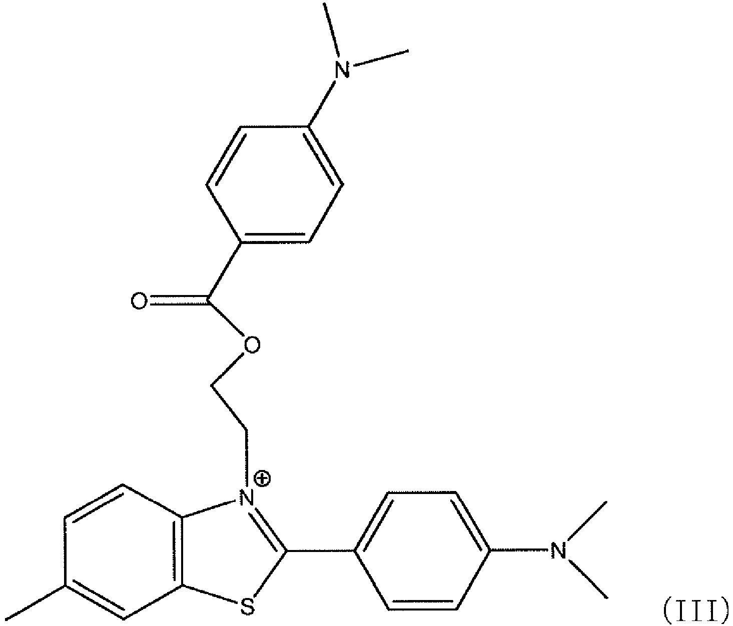

In a preferred embodiment, the guanine-quadruplex-binding reagent is the following ThT-PEG. Here, the PEG chain may have a branched structure. The ThT-PEG may be immobilized on a carrier together with a polyethylene glycol chain. 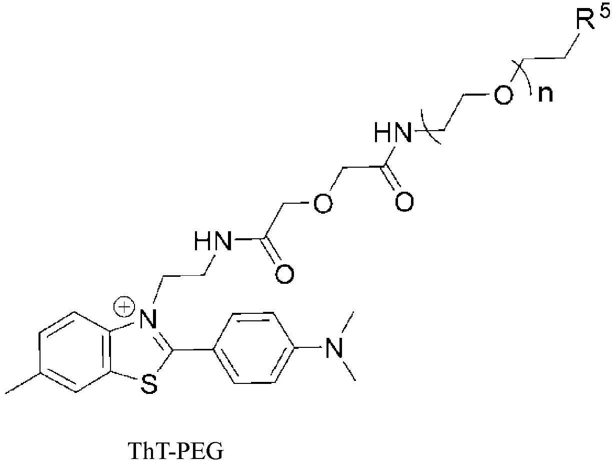 (wherein R⁵ represents an amino group, a hydroxyl group, an alkyl group, or a carboxyl group, and n represents an integer of 4 to 50).

In another preferred embodiment, the guanine-quadruplex-binding reagent is the following ThT-PEG-ThT. The ThT-PEG-ThT may have a branched structure in its PEG-chain moiety, and may be immobilized on a carrier together with a polyethylene glycol chain. In one embodiment, the compound is immobilized on a carrier together with a polyethylene glycol chain. A compound containing a spermine linker may be used instead of the PEG linker.

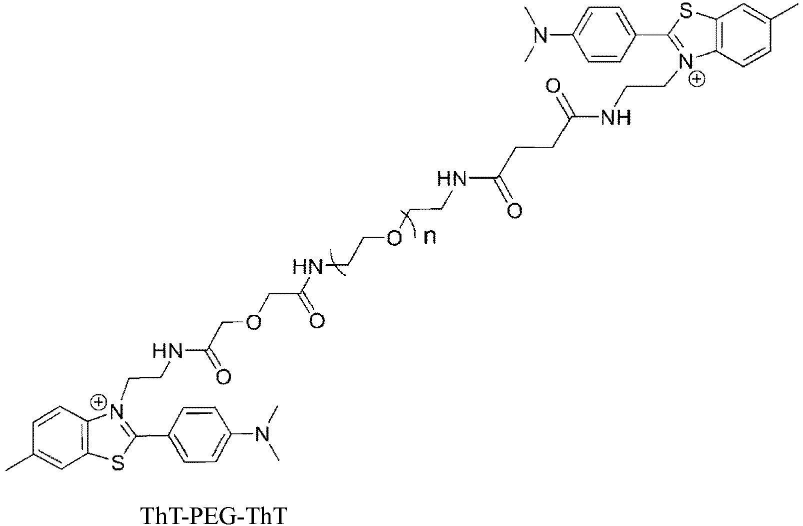

In a preferred embodiment, the nucleic acid detection kit comprises a crown ether.

In a preferred embodiment of the nucleic acid detection kit, the crown ether is 18-crown-6 or 15-crown-5.

In a preferred embodiment, the nucleic acid detection kit comprises a nonionic surfactant.

In a preferred embodiment of the nucleic acid detection kit, the nonionic surfactant is polyoxyethylene sorbitan monolaurate or octylphenol ethoxylate.

In a preferred embodiment of the nucleic acid detection kit, the target nucleic acid is viral RNA.

The present invention also provides a method of detecting a target nucleic acid using the kit, the method comprising the steps of:
hybridizing the first single-stranded circular DNA and the first oligonucleotide primer with the target nucleic acid;
performing a nucleic acid amplification reaction based on the target nucleic acid by rolling circle amplification from the first oligonucleotide primer;
hybridizing the second single-stranded circular DNA and the second oligonucleotide primer with the obtained amplification product;
performing a nucleic acid amplification reaction based on the amplification product by rolling circle amplification from the second oligonucleotide primer; and
detecting an amplified nucleic acid.

The present invention also provides a nucleic acid detection kit comprising:
a short-chain target nucleic acid containing:
a first region; and
a second region in the 3'-side of the first region, the second region containing a mutation;
   (i) a first single-stranded circular DNA containing:
      a region that binds to the short-chain target nucleic acid, the region being complementary to the second region of the short-chain target nucleic acid;
      a second region in the 3'-side thereof; and
      a sequence complementary to a sequence that binds to a second single-stranded circular DNA;
   (ii) a capture oligonucleotide containing:
      a template-binding sequence complementary to the second region of the single-stranded circular DNA; and
      a sequence that binds to the short-chain target nucleic acid, the sequence being complementary to the first region of the short-chain target nucleic acid;
   (iii) a second single-stranded circular DNA containing:
      the same sequence as the sequence in the first single-stranded circular DNA, complementary to the sequence that binds to the second single-stranded circular DNA;
      a second-primer-binding sequence adj acent to the 5'-side of this sequence; and
      a sequence complementary to a detection reagent-binding sequence; and
   (iv) a second oligonucleotide primer containing:
      the same sequence as the region in the first single-stranded circular DNA, adjacent to the 5'-side of the sequence complementary to the sequence that binds to the second single-stranded circular DNA, wherein said same sequence as the region in the first single-stranded circular DNA is 8 to 15 bases; and
      a sequence of 7 to 8 bases adjacent to the 3'-side of this sequence and complementary to the second-primer-binding sequence of the second single-stranded circular DNA,
wherein
the capture oligonucleotide is bound to a carrier through the 5'-end thereof, and
the second oligonucleotide primer is bound, through the 5'-end thereof, to the carrier to which the capture oligonucleotide is bound.

The present invention also provides a method of detecting a short-chain target nucleic acid using the kit, the method comprising the steps of:
hybridizing the first single-stranded circular DNA and the capture polynucleotide with the short-chain target nucleic acid containing: the first region; and the second region adjacent to the 3'-side of the first region and containing the mutation;
performing a nucleic acid amplification reaction by rolling circle amplification based on the formation of a complex of the short-chain target nucleic acid, the capture oligonucleotide, and the first single-stranded circular DNA;
hybridizing the second single-stranded circular DNA and the second oligonucleotide primer with an extended chain generated by the nucleic acid amplification reaction, and performing a nucleic acid amplification reaction based on the formation of a complex of the extended chain, the second primer, and the second single-stranded circular DNA; and
detecting an amplified nucleic acid.

The present invention also provides a kit for detecting a target molecule, the kit comprising:
(i) a first single-stranded circular DNA containing:
   a first region;
   a second region linked to the 3'-side thereof; and
   a sequence complementary to a sequence that binds to a second single-stranded circular DNA;
(ii) a first oligonucleotide primer containing:
   a first aptamer sequence which binds to the target molecule; and
   a sequence linked to the 3'-side thereof and complementary to the first region of the first single-stranded circular DNA;
(iii) a capture oligonucleotide containing:
   a sequence complementary to the second region of the first single-stranded circular DNA; and
   a second aptamer sequence linked to the 3'-side thereof, which binds to the target molecule,
(iv) a second single-stranded circular DNA containing:
   the same sequence as the sequence in the first single-stranded circular DNA, complementary to the sequence that binds to the second single-stranded circular DNA; and
   a sequence which is adjacent to the 5'-side of this sequence and which binds to the second oligonucleotide primer; and
(v) a second oligonucleotide primer containing:
   the same sequence as the region in the first single-stranded circular DNA, adjacent to the 5'-side of the sequence complementary to the sequence that binds to the second single-stranded circular DNA, wherein said same sequence as the site in the first single-stranded circular DNA is 8 to 15 bases; and
   a sequence of 7 to 8 bases adjacent to the 3'-side of this sequence and complementary to the sequence in the second single-stranded circular DNA, that binds to the second oligonucleotide primer,
wherein
the capture oligonucleotide and/or the first oligonucleotide primer is/are bound to a carrier through the 5'-end(s) thereof, and
the second oligonucleotide primer is bound, through the 5'-end thereof, to the carrier to which the capture oligonucleotide and/or the first oligonucleotide primer is/are bound.

The present invention also provides a method of detecting a target molecule using the kit, the method comprising the steps of:
forming a first complex containing the target molecule, the capture oligonucleotide, the first oligonucleotide primer, and the first single-stranded circular DNA;
performing a nucleic acid amplification reaction by rolling circle amplification based on the formation of the first complex;
hybridizing the second single-stranded circular DNA and the second oligonucleotide primer with an extended chain generated by the nucleic acid amplification reaction, to form a second complex containing the extended chain, the second oligonucleotide primer, and the second single-stranded circular DNA;
performing a nucleic acid amplification reaction by rolling circle amplification based on the formation of the second complex; and
detecting an amplified nucleic acid.

Also provided is a compound represented by the following formula: 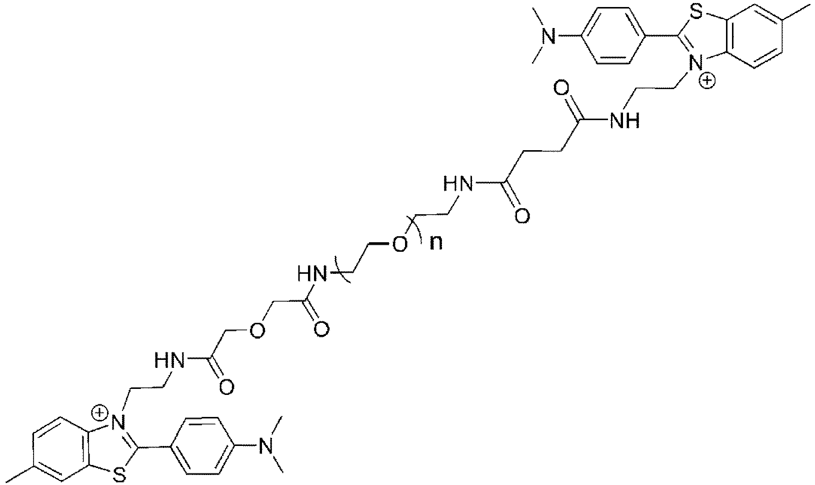 (wherein n represents an integer of 4 to 50).

### EFFECT OF THE INVENTION

According to the present invention, in the presence of a target nucleic acid sequence or a target molecule, a first amplification product is generated by RCA. An oligonucleotide primer then hybridizes with a complex of the first amplification product and a second single-stranded circular DNA. Subsequently, a second amplification product, for example, a DNA strand containing detection reagent-binding sequences such as guanine-quadruplex-containing sequences linearly linked together, is generated by RCA. By staining the resulting DNA strand with a detection reagent such as ThT (derivative), the nucleic acid sequence can be specifically detected.

In any case, immobilization of a primer(s) and/or a capture oligonucleotide on a carrier enabled remarkable improvement of the detection sensitivity.

Moreover, by using ThT-PEG or ThT-PEG-ThT as the detection reagent, the presence or absence of the amplification product can be visually observed, so that a test can be simply carried out. Moreover, by using a detection reagent in which ThT-PEG or ThT-PEG-ThT, and a PEG chain, are immobilized on a carrier, or using a detection reagent in which ThT-PEG or ThT-PEG-ThT is combined therewith, remarkable improvement of the detection sensitivity can be achieved.

### BRIEF DESCRIPTION OF THE DRAWINGS

Fig. 1 is a schematic diagram for a nucleic acid detection method according to the present invention.
Fig. 2 is a diagram (drawing-substituting photographs) illustrating the result of Example 1 of the present invention.
Fig. 3-1 is a diagram (drawing-substituting photographs) illustrating the result of Example 2 of the present invention.
Fig. 3-2 is a diagram (drawing-substituting photographs) illustrating the result of Example 2 of the present invention.
Fig. 4-1 is a diagram (drawing-substituting photographs) illustrating the result of Example 3 of the present invention.
Fig. 4-2 is a diagram (drawing-substituting photographs) illustrating the result of Example 3 of the present invention.
Fig. 4-3 is a diagram (drawing-substituting photographs) illustrating the result of Example 3 of the present invention.
Fig. 5 is a diagram (drawing-substituting photographs) illustrating the result of Example 3 of the present invention.
Fig. 6 is a diagram (drawing-substituting photographs) illustrating the result of Comparative Example 1 of the present invention.
Fig. 7 is a diagram (drawing-substituting photographs) illustrating the result of Comparative Example 1 of the present invention.
Fig. 8-1 is a diagram (drawing-substituting photographs) illustrating the result of Reference Example 1 of the present invention. (A): Use of a target RNA (40-mer); (B): use of a target RNA (full-length).
Fig. 8-2 is a diagram (drawing-substituting photographs) illustrating the result of Reference Example 1 of the present invention.
Fig. 8-3 is a diagram illustrating the result of Reference Example 1 of the present invention.
Fig. 9-1 is a diagram (drawing-substituting photographs) illustrating the result of Example 4 of the present invention.
Fig. 9-2 is a diagram illustrating the result of Reference Example 2 of the present invention.
Fig. 10 is a schematic diagram for a target molecule detection method according to the present invention.
Fig. 11-1 is a diagram (drawing-substituting photographs) illustrating the result of Example 5 of the present invention.
Fig. 11-2 is a diagram (drawing-substituting photographs) illustrating the result of Reference Example 3 of the present invention.
Fig. 11-3 is a diagram illustrating the result of Reference Example 3 of the present invention.
Fig. 12-1 is a diagram (drawing-substituting photographs) illustrating the result of Example 6 of the present invention.
Fig. 12-2 is a diagram (drawing-substituting photographs) illustrating the result of Reference Example 4 of the present invention.
Fig. 12-3 is a diagram illustrating the result of Reference Example 4 of the present invention.
Fig. 13-1 is a diagram (drawing-substituting photographs) illustrating the result of Example 7 of the present invention.
Fig. 13-2 is a diagram (drawing-substituting photographs) illustrating the result of Reference Example 5 of the present invention.
Fig. 13-3 is a diagram illustrating the result of Reference Example 5 of the present invention.
Fig. 14-1 is a diagram (drawing-substituting photographs) illustrating the result of Example 8 of the present invention.
Fig. 14-2 is a diagram (drawing-substituting photographs) illustrating the result of Reference Example 6 of the present invention.
Fig. 14-3 is a diagram illustrating the result of Reference Example 6 of the present invention.
Fig. 15 is a diagram illustrating the result of Example 9 of the present invention.
Fig. 16 is a schematic diagram for a nucleic acid detection method according to the present invention.
Fig. 17 is a schematic diagram for a nucleic acid detection method according to the present invention.
Fig. 18 is a diagram (drawing-substituting photograph) illustrating the result of Example 10 of the present invention.
Fig. 19 is a diagram (drawing-substituting photograph) illustrating the result of Example 11 of the present invention.
Fig. 20 is a diagram (drawing-substituting photograph) illustrating the result of Example 12 of the present invention.
Fig. 21 is a diagram (drawing-substituting photograph) illustrating the result of Example 13 of the present invention.
Fig. 22 is a diagram (drawing-substituting photograph) illustrating the result of Example 14 of the present invention.
Fig. 23 is a diagram (drawing-substituting photograph) No. 1 illustrating the result of Example 15 of the present invention.
Fig. 24 is a diagram (drawing-substituting photograph) No. 2 illustrating the result of Example 15 of the present invention.
Fig. 25 is a diagram (drawing-substituting photograph) illustrating the result of Example 16 of the present invention.
Fig. 26 is a diagram (drawing-substituting photographs) illustrating the result of Example 17 of the present invention.
Fig. 27 is a diagram (drawing-substituting photograph) illustrating the result of Example 18 of the present invention.
Fig. 28 is a diagram (drawing-substituting photograph) illustrating the result of Example 19 of the present invention.
Fig. 29 is a diagram (drawing-substituting photograph) illustrating the result of Example 20 of the present invention.
Fig. 30 is a diagram (drawing-substituting photograph) illustrating the result of Example 21 of the present invention.
Fig. 31 is a diagram (drawing-substituting photographs) illustrating the result of Example 22 of the present invention.
Fig. 32 is a diagram (drawing-substituting photograph) illustrating the result of Example 23 of the present invention.
Fig. 33 is a diagram (drawing-substituting photographs) illustrating the result of Example 24 of the present invention.
Fig. 34 is a diagram (drawing-substituting photographs) illustrating the result of Example 25 of the present invention.
Fig. 35 is a diagram (drawing-substituting photographs) illustrating the result of Example 26 of the present invention. Lane 1: Compound 2; lane 2: Compound 5; lane 3: ThT-PEG and PEG chain, immobilized on a carrier; lane 4: Compound 2 + Compound 5; lane 5: Compound 2 + ThT-PEG and PEG chain, immobilized on a carrier; lane 6: Compound 5 + ThT-PEG and PEG chain, immobilized on a carrier; lane 7: Compound 2 + Compound 5 + ThT-PEG and PEG chain, immobilized on a carri er.
Fig. 36 is a diagram (drawing-substituting photograph) illustrating the result of Example 27 of the present invention.
Fig. 37 is a diagram (drawing-substituting photographs) illustrating the result of Example 28 of the present invention.
Fig. 38 is a diagram (drawing-substituting photographs) illustrating the result of Example 29 of the present invention.
Fig. 39 is a diagram (drawing-substituting photographs) illustrating the result of Example 30 of the present invention.
Fig. 40 is a diagram (drawing-substituting photographs) illustrating the result of Example 31 of the present invention.
Fig. 41 is a diagram (drawing-substituting photographs) illustrating the result of Example 32 of the present invention.

### MODE FOR CARRYING OUT THE INVENTION

### <First Mode>

### <Target Nucleic Acid Detection Kit>

The target nucleic acid detection kit according to a first embodiment in a first mode of the present invention is
a nucleic acid detection kit comprising:
(i) a first single-stranded circular DNA containing:
   a sequence of 10 to 30 bases complementary to a first site of a target nucleic acid;
   a first-primer-binding sequence of 7 to 8 bases adjacent to the 5'-side of this sequence; and
   a sequence complementary to a sequence that binds to a second single-stranded circular DNA;
(ii) a first oligonucleotide primer containing:
   a sequence of 8 to 15 bases complementary to a second site adjacent to the 3'-side of the first site of the target nucleic acid; and
   a sequence of 7 to 8 bases adjacent to the 3'-side of this sequence and complementary to the first-primer-binding site of the first single-stranded circular DNA;
(iii) a second single-stranded circular DNA containing:
   the same sequence as the sequence in the first single-stranded circular DNA, complementary to the sequence that binds to the second single-stranded circular DNA; and
   a second-primer-binding sequence adj acent to the 5'-side of this sequence; and
(iv) a second oligonucleotide primer containing:
   the same sequence as the site in the first single-stranded circular DNA, adjacent to the 5'-side of the sequence complementary to the sequence that binds to the second single-stranded circular DNA, wherein said same sequence as the site in the first single-stranded circular DNA is 8 to 15 bases; and
   a sequence of 7 to 8 bases adjacent to the 3'-side of this sequence and complementary to the second-primer-binding sequence of the second single-stranded circular DNA,
wherein
the first oligonucleotide primer is bound to a carrier through the 5'-end thereof, and
the second oligonucleotide primer is bound, through the 5'-end thereof, to the carrier to which the first oligonucleotide primer is bound.

### <Target Nucleic Acid>

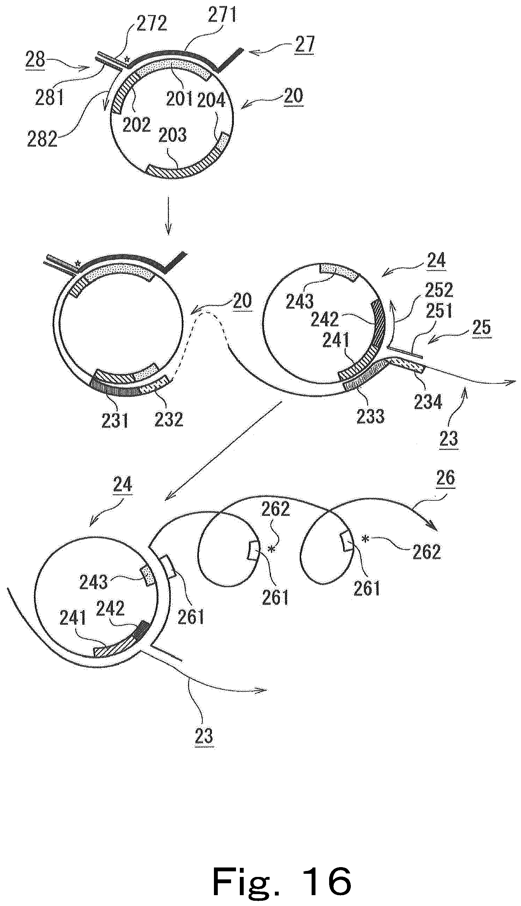

The target nucleic acid is not limited as long as it hybridizes with the first single-stranded circular DNA and the first oligonucleotide primer. The target nucleic acid may be a sequence containing a gene mutation such as an SNP, and, in this case, the gene mutation may be contained in the second site of the target nucleic acid (see Fig. 16; the asterisk in Fig. 16 represents the mutation). Examples of the target nucleic acid include DNA, RNA, DNA/RNA hybrid, and DNA/RNA chimera. The target nucleic acid may be composed only of natural bases, nucleotides, and/or nucleosides, or may partially contain a non-natural base, nucleotide, and/or nucleoside.

The DNA is not limited and any kind of DNA including cDNA, genomic DNA, and synthetic DNA may be detected as a target. The DNA may be in either a linear form or a circular form. Examples of the DNA include DNAs derived from DNA viruses and pathogens that cause diseases such as infections, and toxicoses.

The RNA is not limited and any kind of RNA such as mRNA, ribosomal RNA (rRNA), or transfer RNA (tRNA) may be detected as a target. The mRNA may or may not have a poly(A) sequence. The RNA may be a non-coding RNA such as siRNA, miRNA, piRNA, rasiRNA, rRNA, or tRNA, or it may be genomic RNA of a virus or the like. The RNA may be in either a linear form or a circular form. Examples of the RNA include an RNA expressed specifically in a disease, an RNA whose expression level varies among diseases, and an RNA derived from an RNA virus (such as an influenza virus) or a pathogen that causes a disease such as an infection or causes a toxicosis.

The concentration of the target nucleic acid in the amplification reaction (in use) is, for example, not less than 0.1 aM, not less than 1 aM, not less than 10 aM, or not less than 50 aM regarding the lower limit, and, for example, not more than 1000 aM, not more than 500 aM, not more than 200 aM, or not more than 100 aM regarding the upper limit.

### <First Single-Stranded Circular DNA>

The first single-stranded circular DNA contains:
a sequence of 10 to 30 bases complementary to a first site of a target nucleic acid;
a primer-binding sequence of 7 to 8 bases adjacent to the 5'-side of this sequence; and
a sequence complementary to a sequence that binds to a second single-stranded circular DNA.

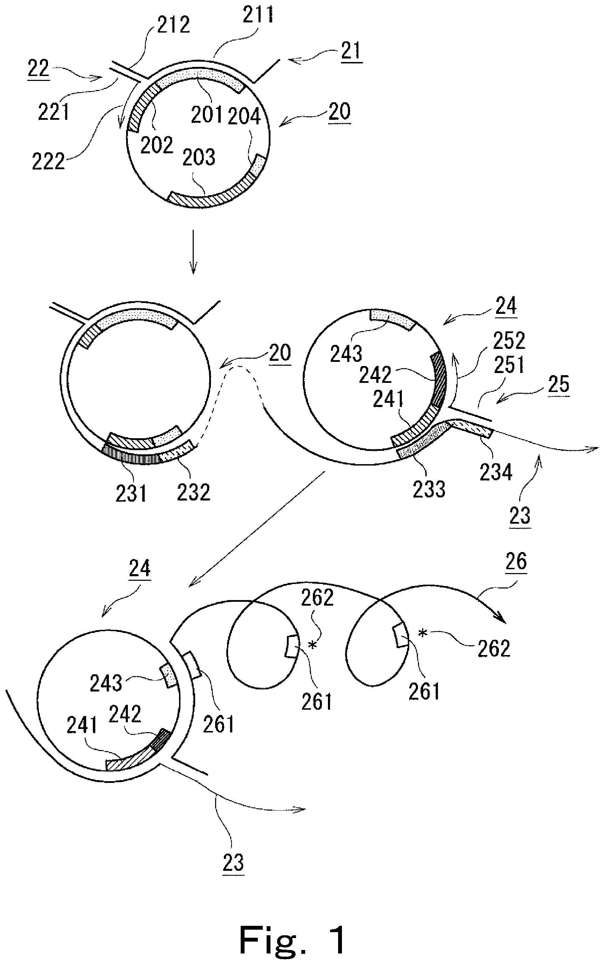

A description is given below with reference to Fig. 1 (see Fig. 16 for a case where the gene mutation is included in the second site of the target nucleic acid, wherein the reference numerals 21, 211, 212, 22, 221, and 222 in Fig. 1 correspond to the reference numerals 27, 271, 272, 28, 281, and 282 in Fig. 16, respectively). The single-stranded circular DNA is illustrated in the 5' -> 3' clockwise direction. For convenience, the carrier is not presented.

The first single-stranded circular DNA 20 contains: a sequence 201 complementary to a first site 211 of a target nucleic acid 21; a primer-binding sequence 202 linked to its 5'-side; and a sequence 203 complementary to a sequence that binds to a second single-stranded circular DNA.

The sequence 201 has a length of usually 10 to 30 bases, preferably 15 to 25 bases, and a GC content of preferably 30 to 70%. The sequence 202 has a length of 7 bases or 8 bases. The sequence is not limited and has a GC content of preferably 30 to 70%. The total length of the first single-stranded circular DNA 20 is preferably 35 to 100 bases.

The first single-stranded circular DNA 20 can be obtained by circularization of a single-stranded DNA (ssDNA). The circularization of the single-stranded DNA can be carried out by arbitrary means. It can be carried out by using, for example, CircLigase (registered trademark), CircLigase II (registered trademark), ssDNA Ligase (Epicentre), or ThermoPhage ligase (registered trademark) single-stranded DNA (Prokzyme).

### <First Oligonucleotide Primer>

The first oligonucleotide primer 22 contains:
a sequence 221 of 8 to 15 bases complementary to a second site 212 adjacent to the 3'-side of the first site 211 of the target nucleic acid 21; and
a sequence 222 of 7 to 8 bases linked to the 3'-side thereof and complementary to the primer-binding site 202 of the first single-stranded circular DNA 20.

The first oligonucleotide primer 22 is bound also to a carrier through its 5'-end.

The mode of each of the first oligonucleotide primer 22, its 5'-end, and the carrier is not limited as long as the first oligonucleotide primer 22 can be bound to the carrier through its 5'-end, and as long as a nucleic acid amplification reaction based on the target nucleic acid 21 can be carried out by the later-described rolling circle amplification (RCA) method using the first oligonucleotide primer 22.

Examples of the 5'-end of the first oligonucleotide primer 22 include those modified with biotin, an amino group, an aldehyde group, or an SH group. Examples of the carrier include carriers capable of binding to each of these, such as a carrier on which avidin (including its derivative, such as streptavidin or NeutrAvidin) is immobilized, and a carrier whose surface is treated with a silane coupling agent containing an amino group, an aldehyde group, an epoxy group, or the like. The immobilization may be carried out according to a conventional method.

The carrier is preferably a carrier capable of immobilizing the first oligonucleotide primer 22 and the later-described second oligonucleotide primer 25 closely to each other. This is because, in cases where these are positioned closely to each other, the step of amplification of the first amplification product 23 from the first oligonucleotide primer 22 and the step of amplification of the second amplification product 26 from the second oligonucleotide primer 25 can be more efficiently carried out compared to a method using two kinds of primers in the free state in a solution, such as the method described in Patent Document 2, so that the detection sensitivity can be remarkably improved as a result.

Preferred examples of the carrier include beads, and planar carriers such as substrates for use in sensors. The beads are insoluble carriers having a particle shape with an average particle size of, for example, 10 nm to 100 µm, preferably 30 nm to 10 µm, more preferably 30 nm to 1 µm, still more preferably 30 nm to 500 nm. The material of the beads is not limited. Examples of the material include magnetic bodies (iron oxides such as ferrite and magnetite; and magnetic materials such as chromium oxide and cobalt), silica, agarose, and sepharose. A magnetic-body bead is called "magnetic bead" in some cases. A metal colloid particle such as a gold colloid particle may also be used.

### <Second Single-Stranded Circular DNA>

The second single-stranded circular DNA 24 contains:
the same sequence 241 as the sequence 203 in the first single-stranded circular DNA 20, complementary to the sequence that binds to the second single-stranded circular DNA; and
a second-primer-binding sequence 242 adjacent to the 5'-side of this sequence.

The sequence 203 has a length of usually 10 to 30 bases, preferably 15 to 25 bases, and a GC content of preferably 30 to 70%. The sequence 242 has a length of 7 bases or 8 bases. The sequence is not limited, and has a GC content of preferably 30 to 70%. The total length of the second single-stranded circular DNA 24 is preferably 35 to 100 bases. The second single-stranded circular DNA 24 can be obtained by circularization of a single-stranded DNA (ssDNA) by the method described above.

The second single-stranded circular DNA 24 preferably contains a sequence complementary to a detection reagent-binding sequence. Examples of the detection reagent-binding sequence include a guanine-quadruplex-forming sequence.

The guanine-quadruplex-forming sequence may be, for example, a sequence described in Nat. Rev. Drug Discov. 2011 Apr; 10(4): 261-275, and can be represented as G₃N₁₋₁₀G₃N₁₋₁₀G₃N₁₋₁₀G₃. Specific examples of the sequence include a sequence described in Patent Document 2. Accordingly, the sequence 243 complementary to the guanine-quadruplex-forming sequence may be, for example, C₃N₁₋₁₀C₃N₁₋₁₀C₃N₁₋₁₀C₃. In other words, in the sequence, three consecutive C's are repeated four times via spacers each having a sequence composed of 1 to 10 (preferably 1 to 5) arbitrary bases (N = A, T, G, or C).

The sequence 243 complementary to the guanine-quadruplex-forming sequence may have arbitrary sequences before and after it, that is, between the sequence 243 and the same sequence 241 as the sequence 203 complementary to the sequence that binds to the second single-stranded circular DNA, and between the sequence 243 and the second-primer-binding sequence 242.

Although Fig. 1 illustrates a case where the second single-stranded circular DNA 24 contains a sequence 243 complementary to a guanine-quadruplex-forming sequence, this is merely one example of the case where the second single-stranded circular DNA 24 contains a sequence complementary to a detection reagent-binding sequence, and where the sequence complementary to a detection reagent-binding sequence is a sequence 243 complementary to a guanine-quadruplex-forming sequence. Alternatively, for example, the detection may be carried out using, as the detection reagent-binding sequence, an aptamer sequence or a sequence that binds to a molecular beacon (hairpin-shaped oligonucleotide having a fluorescent group (donor) and a quenching group (acceptor) that cause FRET), and using, as the detection reagent, an aptamer-binding coloring molecule or the molecular beacon (ChemBioChem 2007, 8, 1795-1803; J. Am. Chem. Soc. 2013, 135, 7430-7433).

Even in cases where the second single-stranded circular DNA 24 does not contain a sequence complementary to the detection reagent-binding sequence, the detection is possible by a known detection method capable of detecting the second amplification product 26. In other words, detection is possible by a known detection method without using a detection reagent that binds to a detection reagent-binding sequence. Examples of the detection method include a method in which the second amplification product 26 is labeled with, for example, a fluorescent reagent which does not bind to the first amplification product 23, but which specifically binds to the second amplification product 26, and the fluorescence intensity is measured.

### <Second Oligonucleotide Primer>

The second oligonucleotide primer 25 contains:
the same sequence 251 (preferably a sequence of 8 to 15 bases) as the site 204 adjacent to the 5'-side of the sequence 203 in the first single-stranded circular DNA 20, complementary to the sequence that binds to the second single-stranded circular DNA; and
a sequence 252 (preferably a sequence of 7 to 8 bases) adjacent to the 3'-side of this sequence and complementary to the second-primer-binding sequence 242 of the second single-stranded circular DNA 24.

The second oligonucleotide primer 25 is bound, through its 5'-end, to the carrier to which the first oligonucleotide primer 22 is bound.

The description in the section for the first oligonucleotide primer is applied to the mode of each of the second oligonucleotide primer 25, its 5'-end, and the carrier. Their modes are preferably the same as the modes of the first oligonucleotide primer 22, its 5'-end, and the carrier, respectively.

Thus, preferably, for example, the first oligonucleotide primer 22 is modified with biotin at the 5'-end thereof, and bound, through the biotin, to a carrier on which avidin is immobilized, and the second oligonucleotide primer 25 is modified with biotin at the 5'-end thereof, and bound, through the biotin, to the carrier to which the first oligonucleotide primer 22 is bound.

### <Ratio between Amounts of First Oligonucleotide Primer and Second Oligonucleotide Primer>

The ratio of the amount between the first oligonucleotide primer 22 and the second oligonucleotide primer 25 immobilized on the carrier reflects the concentration ratio at the time of immobilization of the primers. For example, as in the later-described Examples, in a case where the carrier is washed, and the supernatant is removed, followed by adding a mixture containing the first oligonucleotide primer 22 at a concentration of 1 µM and the second oligonucleotide primer 25 at a concentration of 20 µM to the carrier and immobilizing the primers to the carrier, the ratio of the amount (molar ratio) between the first oligonucleotide primer 22 and the second oligonucleotide primer 25 immobilized on the carrier can be regarded as 1:20.

The ratio of the amount between the first oligonucleotide primer 22 and the second oligonucleotide primer 25 immobilized on the carrier in terms of the molar ratio is preferably 1:10 to 1:30, more preferably 1:10 to 1:25, still more preferably 1:10 to 1:20, still more preferably 1:10 to 1:15.

The concentration of the first oligonucleotide primer 22 in the amplification reaction (in use) in terms of the molar ratio is preferably not less than 0.0025 pmol/µL, more preferably not less than 0.005 pmol/µL, and is preferably not more than 0.04 pmol/µL, more preferably not more than 0.02 pmol/µL.

The concentration of the second oligonucleotide primer 25 in the amplification reaction (in use) in terms of the molar ratio is preferably not less than 0.0125 pmol/µL, more preferably not less than 0.025 pmol/µL, and is preferably not more than 0.8 pmol/µL, more preferably not more than 0.4 pmol/L.

### <Relationship between Amounts of First Single-Stranded Circular DNA and Second Single-Stranded Circular DNA>

The ratio of the amount between the first single-stranded circular DNA 20 and the second single-stranded circular DNA 24 in the amplification reaction (in use) in terms of the molar ratio is preferably 1:2 to 1: 1000, more preferably 1:3 to 1:500, still more preferably 1:4 to 1:400.

The concentration of the first single-stranded circular DNA 20 in the amplification reaction (in use) is, for example, not less than 0.1 nM, not less than 1 nM, not less than 10 nM, or not less than 50 nM regarding the lower limit, and, for example, not more than 500 nM, not more than 200 nM, or not more than 100 nM regarding the upper limit.

The concentration of the second single-stranded circular DNA 24 in the amplification reaction (in use) is, for example, not less than 20 nM, not less than 40 nM, not less than 100 nM, or not less than 200 nM regarding the lower limit, and, for example, not more than 1000 nM, not more than 500 nM, or not more than 400 nM regarding the upper limit.

### <Amplification Method>

As illustrated in Fig. 1, after hybridizing the first single-stranded circular DNA 20 and the primer 22 with the target nucleic acid 21 to allow formation of a ternary complex, a nucleic acid amplification reaction based on the target nucleic acid 21 is carried out using the rolling circle amplification (RCA) method.

Those skilled in the art can appropriately set the conditions for the hybridization taking into account the combination of the single-stranded circular DNA 20, the target nucleic acid 21, and the primer.

The RCA method is described in, for example, Lizardi et al., Nature Genet. 19: 225-232 (1998); US 5,854,033 B; US 6,143,495 B; and WO 97/19193. The RCA method can be carried out using, for example, a mesophilic chain-substituting DNA synthetase such as phi29 polymerase, Klenow DNA Polymerase (5'-3', 3'-5' exo minus), Sequenase (registered trademark) Version 2.0 T7 DNA Polymerase (USB), Bsu DNA Polymerase, or Large Fragment (NEB); or a heat-resistant chain-substituting DNA synthetase such as Bst DNA Polymerase (Large Fragment), Bsm DNA Polymerase, Large Fragment (Fermentas), BcaBEST DNA polymerase (TakaraBio), Vent DNA polymerase (NEB), Deep Vent DNA polymerase (NEB), or DisplaceAce (registered trademark) DNA Polymerase (Epicentre).

The extension reaction of DNA by RCA does not require use of a thermal cycler, and is carried out, for example, at a constant temperature within the range of 25°C to 65°C. The reaction temperature is appropriately set according to an ordinary procedure based on the optimum temperature of the enzyme and the denaturation temperature (the temperature range in which binding (annealing) of the primer to, or dissociation of the primer from, the DNA occurs), which is dependent on the primer chain length. The reaction may also be carried out at a constant, relatively low temperature. For example, in cases where phi29DNA polymerase is used as a chain-substituting DNA synthetase, the reaction is carried out preferably at 25°C to 42°C, more preferably at about 30 to 37°C.

By the RCA, a first amplification product 23 is amplified dependently on the target nucleic acid 21 from the primer 22 along the first single-stranded circular DNA 20.

Since the amplification product 23 contains a sequence 233 complementary to the sequence 203 in the first single-stranded circular DNA 20, complementary to the sequence that binds to the second single-stranded circular DNA, the second single-stranded circular DNA 24, which contains the same sequence 241 as the sequence 203, hybridizes with the sequence 233 of the first amplification product 23 via the sequence 241.

With the thus formed complex of the first amplification product 23 and the second single-stranded circular DNA 24, the second oligonucleotide primer 25 hybridizes to form a ternary complex.

More specifically, since the second oligonucleotide primer 25 contains the same sequence 251 as the site 204 adjacent to the 5'-side of the sequence 203 in the first single-stranded circular DNA 20, complementary to the sequence that binds to the second single-stranded circular DNA, the second oligonucleotide primer 25 hybridizes with the region 234 of the first amplification product 23, which region is complementary to the site 204 of the first single-stranded circular DNA 20, via the sequence 251.

Since the second oligonucleotide primer 25 contains, in the 3'-side of the sequence 251, the sequence 252 complementary to the second-primer-binding sequence 242 of the second single-stranded circular DNA 24, the second oligonucleotide primer 25 also hybridizes with the second single-stranded circular DNA 24 via the sequence 252.

By RCA, a second amplification product 26 is amplified from the resulting ternary complex of the first amplification product 23, the second single-stranded circular DNA 24, and the second oligonucleotide primer 25. Since the second amplification product 26 contains, for example, a sequence 261 containing a guanine quadruplex, it can be detected with a guanine quadruplex detection reagent 262. The second single-stranded circular DNA 24 hybridizes with each region 231 contained in the first amplification product 23, to cause the RCA reaction.

Since the first oligonucleotide primer 22 and the second oligonucleotide primer 25 are immobilized on the same carrier, the step of amplification of the first amplification product 23 from the first oligonucleotide primer 22 and the step of amplification of the second amplification product 26 from the second oligonucleotide primer 25 are carried out at positions close to each other, so that remarkable improvement of the detection sensitivity can be achieved relative to the method described in Patent Document 2 that uses two kinds of primers in the free state in a solution.

As exemplified in Fig. 16, in cases where the presence or absence of a gene mutation such as an SNP is detected using a sequence containing the mutation as a target nucleic acid 27, when the type of the mutation matches the type of the base arranged in the first oligonucleotide primer 28, the amplification reaction occurs, so that the mutation is detected with the detection reagent. On the other hand, in cases where the type of the mutation is different from the type of the base arranged in the first oligonucleotide primer 28, the amplification reaction hardly occurs, so that the mutation is not detected with the detection reagent.

Thus, the first oligonucleotide primer 28 preferably has a base that hybridizes with the mutated base present in the second site 272 of the target nucleic acid 27 such that the base is positioned closest to the 3'-side of the sequence 281 complementary to the second site 272 of the target nucleic acid 27.

For example, in cases where the gene mutation in the target nucleic acid 27 is an A/G single nucleotide polymorphism, and where the A is to be detected, the first oligonucleotide primer 28 is preferably designed such that T, corresponding to the A, is positioned closest to the 3'-side of the sequence 281 complementary to the second site.

In cases where a high concentration of salt, for example, sodium ion at a concentration of not less than about 150 mM, is present in the reaction system, the SATIC reaction hardly proceeds. However, in cases where a crown ether is present in the reaction system, the SATIC reaction can easily proceed even at a high salt concentration. Examples of the crown ether include 18-crown-6 and 15-crown-5. The final concentration of the crown ether in the reaction system is, for example, 180 to 280 mM, preferably 180 to 240 mM or 240 to 280 mM.

For improving wettability and stability of nucleic acid and/or protein, the reaction system may contain a nonionic surfactant. Examples of such a case include cases in which a biological sample is treated under stable conditions. Examples of the nonionic surfactant include polyoxyethylene sorbitan monolaurate (Tween 20) and octylphenol ethoxylate (Triton X-100 and Nonidet P-40). The final concentration of polyoxyethylene sorbitan monolaurate in the reaction system is preferably not more than 2 v/v%, more preferably not more than 1 v/v%. The final concentration of octylphenol ethoxylate in the reaction system is preferably not more than 0.8 v/v%, more preferably not more than 0.5 v/v%.

### <Detection Method>

The second amplification product 26 obtained by RCA can be detected by a known detection method as described above. The second single-stranded circular DNA 24 preferably contains a sequence complementary to a detection reagent-binding sequence so as to include the detection reagent-binding sequence in the second amplification product 26 obtained by RCA.

In cases where the detection reagent-binding sequence is a guanine-quadruplex-forming sequence or the like, the amplification product obtained by RCA can be detected using a guanine-quadruplex-binding reagent. Examples of the guanine-quadruplex-binding reagent include the following reagents.
[1] Thioflavin T (ThT) or a derivative thereof 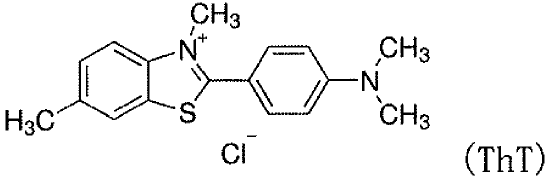
[2] H-aggregate "Yan, J. W.; Ye, W. J.; Chen, S. B.; Wu, W. B.; Hou, J. Q.; Ou, T. M.; Tan, J. H.; Li, D.; Gu, L. Q.; Huang, Z. S. Anal. Chem. 2012, 84, 6288-6292." 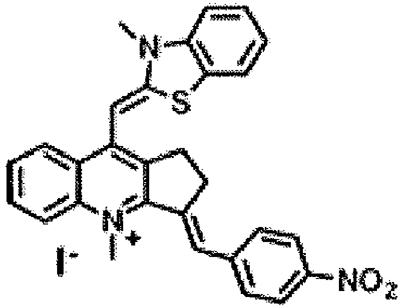
[3] TMPyP4 "Yaku, H.; Fujimoto, T.; Murashima, T.; Miyoshi, D.; Sugimoto, N. Chem. Commun. 2012, 48, 6203-6216." 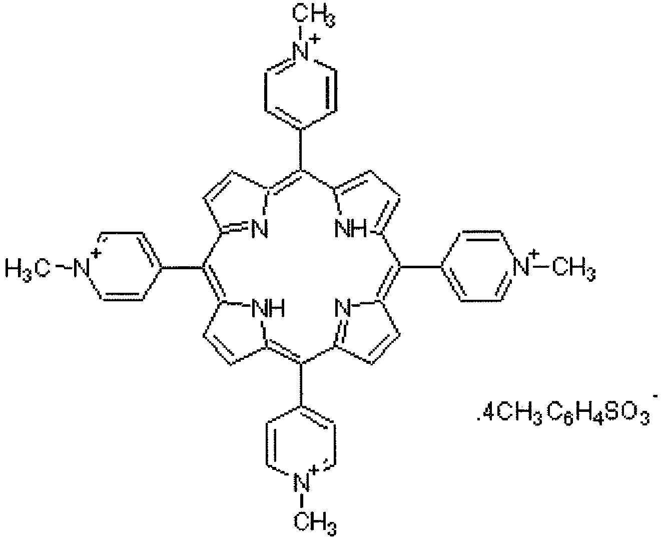
[4] PPIX "Li, T.; Wang, E.; Dong, S. Anal. Chem. 2010, 82, 7576-7580." 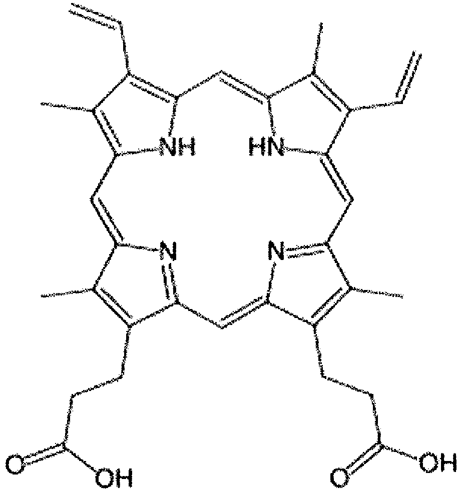
[5] BPBC "Jin, B.; Zhang, X.; Zheng, W.; Liu, X.; Qi, C.; Wang, F.; Shangguan, D. Anal. Chem. 2014, 86, 943-952."
[6] APD "Nikan, M.; Di Antonio, M.; Abecassis, K.; McLuckie, K.; Balasubramanian, S. Angew. Chem., Int. Ed. 2013, 52, 1428-1431." 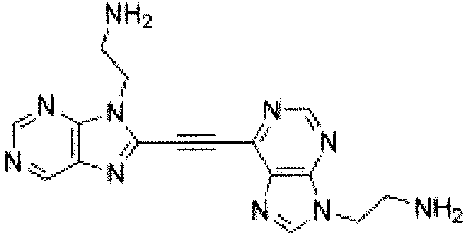
[7] Thiazole orange (TO)
   "Nakayama S.; Kelsey I.; Wang J.; Roelofs K.; Stefane B.; Luo Y; Lee V.T.; Sintim H. O. J. Am. Chem. Soc. 2011, 133, 4856-4864."
[8] Malachite green
   "Li X.; Zheng F.; Ren R. Chem Commun, 2015, 51, 11976-11979. "

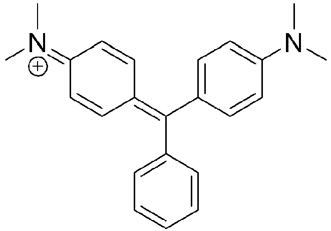

Preferably, the Malachite Green or a ThT derivative represented by the following General Formula (I) may be used (Anal. Chem. 2014, 86, 12078-12084).

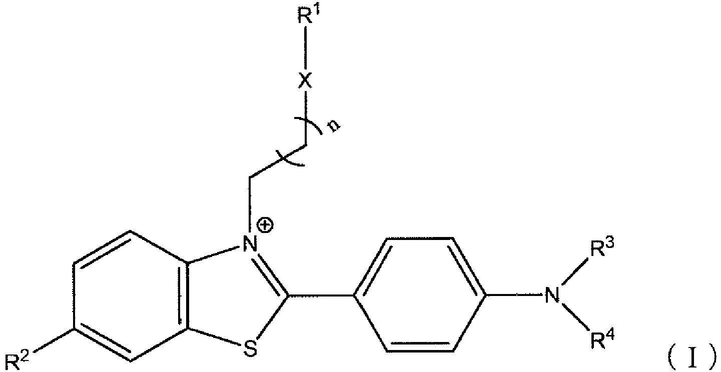

In this formula, R¹ represents hydrogen, or a C₁-C₁₀ (preferably C₁-C₅) hydrocarbon group which optionally contains one or more selected from O, S, and N. The hydrocarbon group may be either linear or branched, or either saturated or unsaturated. The hydrocarbon group may be an aliphatic hydrocarbon group such as an alkyl group, or may be an aromatic hydrocarbon group such as an aryl group or an arylalkyl group. The term "optionally contains one or more selected from O, S, and N" means that the hydrocarbon group may contain a functional group containing a nitrogen atom, an oxygen atom, a sulfur atom, or the like, such as an amino group (-NR₂) (wherein each R independently represents hydrogen or a C₁-C₅ alkyl group), a nitro group (-NO₂), a cyano group (-CN), an isocyanate group (-NCO), a hydroxyl group (-OH), an aldehyde group (-CHO), a carboxyl group (-COOH), a mercapto group (-SH), or a sulfonic acid group (-SO₃H), or that a linking group containing a nitrogen atom, an oxygen atom, a sulfur atom, or the like, such as an ether group (-O-), an imino group (-NH-), a thioether group (-S-), a carbonyl group (-C(=O)-), an amide group (-C(=O)-NH-), an ester group (-C(=O)-O-), or a thioester group (-C(=O)-S-), may be contained in the inside or at a terminus of the carbon backbone of the hydrocarbon group.

R², R³, and R⁴ each independently represent a C₁-C₅ (aliphatic) hydrocarbon group, more preferably a C₁-C₃ hydrocarbon group, especially preferably a methyl group. The C₁-C₅ hydrocarbon group may be either linear or branched, or either saturated or unsaturated.

n represents an integer of 0 to 5, more preferably an integer of 0 to 3, especially preferably 1.

X represents O, S, or NH, more preferably O.

Specific examples of the compound include the following.

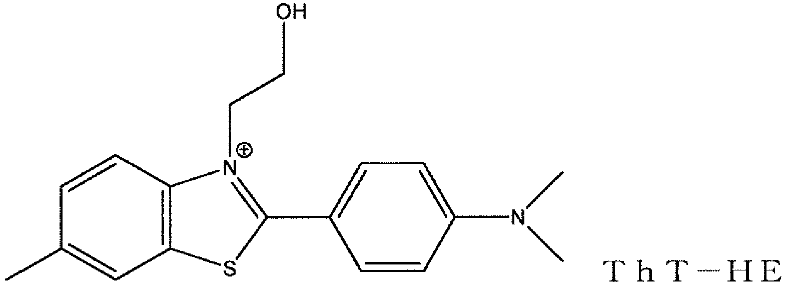

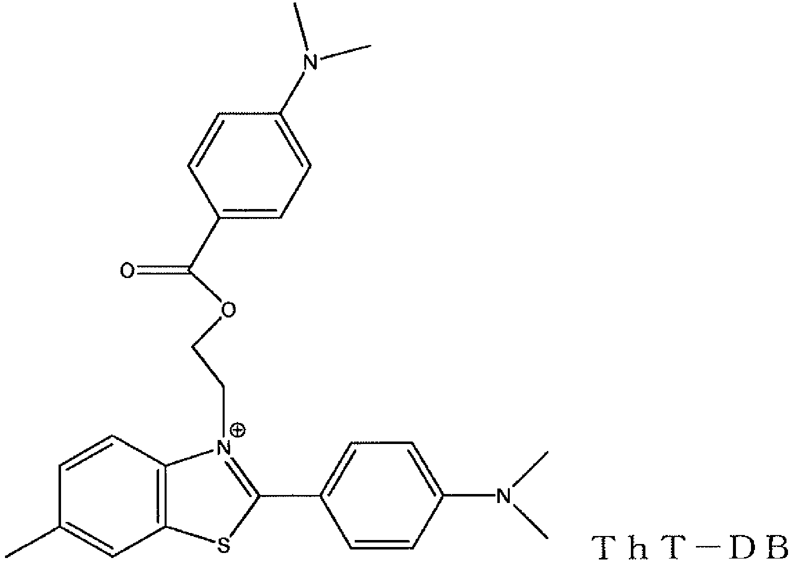

The following ThT derivatives containing a PEG chain (ThT-PEG) may also be used.

In this formula, R⁵ represents an amino group, a hydroxyl group, an alkyl group, or a carboxyl group, and n represents an integer of 4 to 50, preferably an integer of 5 to 20, more preferably an integer of 8 to 15, especially preferably 11. The ThT-PEG is more preferably a compound wherein R⁵ represents an amino group.

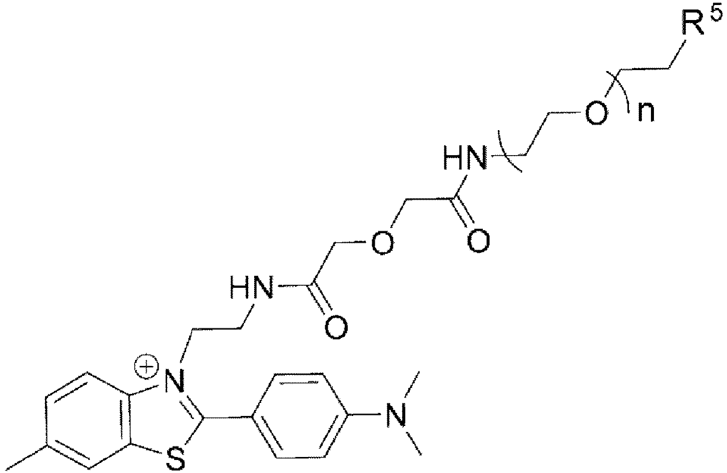

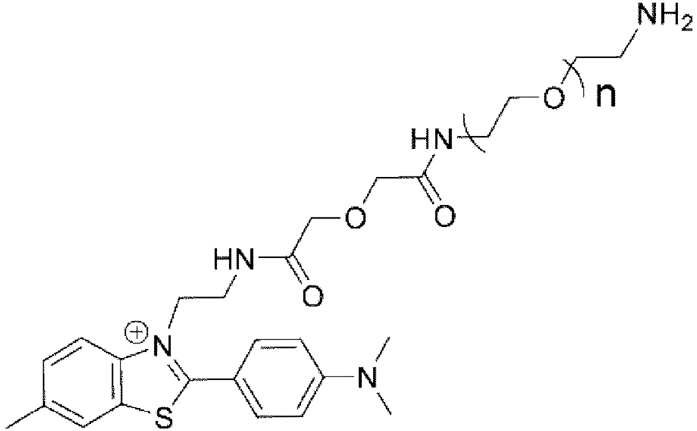

Also provided is the following ThT derivative containing ThT's linked through a PEG chain (ThT-PEG-ThT). In this formula, n represents an integer of 4 to 50, preferably an integer of 5 to 20, more preferably an integer of 8 to 15, especially preferably 11. The PEG chain of the ThT-PEG-ThT may be replaced with a spermine linker. Accordingly, provided is a compound represented by the following formula: 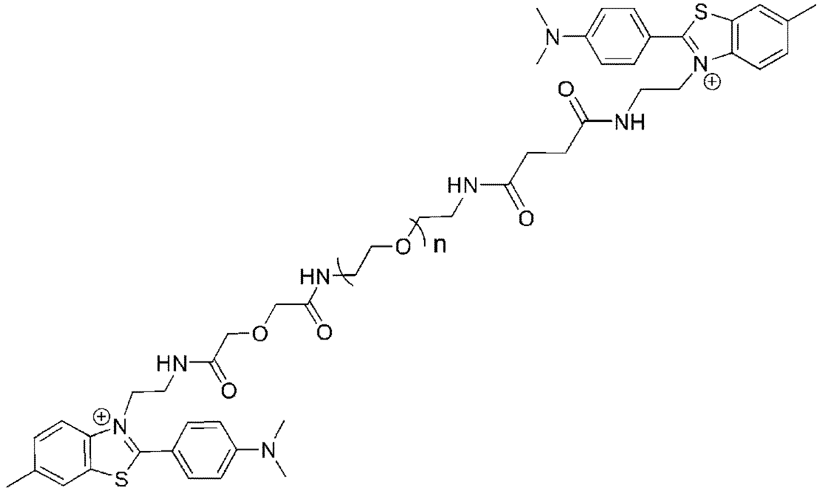 (wherein n represents an integer of 4 to 50).

The detection of the guanine quadruplex structure in the test DNA can be carried out by, for example, bringing a compound represented by General Formula (I) or a salt thereof into contact with a sample containing the RCA product, and detecting the compound bound to the guanine quadruplex structure based on fluorescence emitted from the compound. The detection operation itself is the same as a known method except that the compound represented by General Formula (I) or a salt thereof is used. The detection operation can be carried out by bringing a solution prepared by dissolving the compound in a buffer into contact with a sample containing a test DNA, incubating the resulting mixture, carrying out washing, and then detecting fluorescence from the fluorescent dye bound to the test DNA after the washing.

In the method of the present invention, in cases where ThT-PEG or ThT-PEG-ThT is used as the guanine-quadruplex-binding reagent, binding of the ThT-PEG or ThT-PEG-ThT to the RCA product causes specific aggregation, so that the presence or absence of the RCA amplification can be simply investigated by visual observation even without using a fluorescence detection apparatus. ThT-PEG and ThT-PEG-ThT may be used at the same time.

In cases where the presence or absence of the RCA amplification is investigated by visual observation, the aggregation may be allowed to occur quickly by carrying out the following operation as a post-reaction treatment after the RCA amplification.

For example, in cases where beads are used, a magnet may be applied to the reaction solution to accumulate the beads; the beads may be uniformly distributed by shaking a reaction container such as a tube; or the beads may be left to stand as they are. Preferably, magnetic beads are accumulated by, for example, application of a magnet to the reaction solution.

After the post-reaction treatment, the beads are preferably left to stand for a predetermined period of time at a predetermined temperature. Thereafter, the beads may be accumulated by, for example, shaking the reaction container such as a tube to uniformly distribute the beads, and then applying a magnet thereto, or may be accumulated by, for example, simple application of the magnet.

The predetermined time described above is preferably not more than 10 minutes, more preferably not more than 5 minutes, still more preferably not more than 3 minutes, still more preferably not more than 1 minute, and is preferably not less than 30 seconds. The predetermined temperature described above is preferably not more than 10°C, more preferably not more than 5°C, still more preferably not more than 2°C, still more preferably not more than -10°C, especially preferably not more than -20°C, and is preferably not less than -30°C.

In cases where ThT-PEG-ThT is used as the guanine-quadruplex-binding reagent, a PEG may be present therewith. The PEG is, for example, PEG 800 or higher, preferably PEG 900 or higher, and is, for example, PEG 4000 or lower, preferably PEG 2000 or lower, more preferably PEG 1500 or lower, still more preferably PEG 1200 or lower.

In cases where ThT-PEG-ThT and PEG are used, the final concentration of the PEG in the reaction system is, for example, not less than 8 w/v%, preferably not less than 10 w/v%, and is, for example, not more than 30 w/v%, preferably not more than 25 w/v%.

The molar ratio between the ThT-PEG-ThT and the PEG is preferably 1: 10,000 to 1:25,000.

In cases where ThT-PEG-ThT and PEG are used, the reaction time is, for example, not less than 15 minutes, preferably not less than 20 minutes, and is, for example, not more than 3 hours.

In cases where ThT-PEG or ThT-PEG-ThT is used as the guanine-quadruplex-binding reagent, the ThT-PEG or ThT-PEG-ThT added to the reaction product may be in a form in which it is immobilized on a carrier. As the carrier, a bead such as a magnetic bead; a gold colloid; or the like may be used. Its average particle size is, for example, 10 nm to 100 µm, preferably 30 nm to 10 µm, more preferably 30 nm to 1 µm, still more preferably 30 nm to 100 nm. The immobilization of the ThT-PEG or ThT-PEG-ThT on the carrier may be carried out by, for example, adding biotin to the ThT-PEG or ThT-PEG-ThT, and reacting the biotin with streptavidin introduced to the carrier. Preferably, in cases where the ThT-PEG-ThT is immobilized on the carrier, a branched chain is provided in the PEG-chain moiety, and biotin is added thereto for reacting the biotin with the streptavidin introduced to the carrier. In cases where the ThT-PEG or ThT-PEG-ThT is immobilized on the carrier, a PEG chain ((CH₂CH₂O)ₙ; n = 4 to 50) is also preferably immobilized on the carrier. The immobilization of the PEG chain on the carrier may also be carried out by, for example, adding biotin to the PEG chain similarly to the case of the ThT-PEG or ThT-PEG-ThT, and reacting the biotin with the streptavidin introduced to the carrier.

In cases where the ThT-PEG or ThT-PEG-ThT, and the PEG chain, are immobilized on the carrier to provide a detection reagent, the ratio between the ThT-PEG or ThT-PEG-ThT, and the PEG chain, is preferably 3:7 to 9: 1.

The ThT-PEG or ThT-PEG-ThT, and the PEG chain, immobilized on the carrier may be used in combination with ThT-PEG and/or ThT-PEG-ThT.

A synthesis example of a ThT derivative as one example of a guanine quadruplex detection reagent that may be used in the method of the present invention, and an experimental example for detection of a guanine quadruplex using the ThT derivative, are known and described in Patent Document 2.

ThT-PEG is described as ThT-P42 in Examples of JP 2018-154564 A. A synthesis method for ThT-PEG-ThT is described in the Examples below.

For example, the nucleic acid detection kit may use, as the first oligonucleotide primer, a short-chain target nucleic acid such as a miRNA, preferably a short-chain target nucleic acid containing a mutation, and a capture oligonucleotide that captures the nucleic acid. Provided herein is a method of detecting a target nucleic acid using a kit of the invention.

Examples of such a miRNA include miR-21CA and miR-13b.

The kit according to the present embodiment is described below for the case of a miRNA containing a mutation.

The kit uses, as a short-chain target nucleic acid,
a miRNA containing:
   a first region; and
   a second region in the 3'-side thereof, the second region containing a mutation;
and comprises:
   (i) a first single-stranded circular DNA containing:
      a miRNA-binding region complementary to the second region of the miRNA;
      a second region in the 3'-side thereof; and
      a sequence complementary to a sequence that binds to a second single-stranded circular DNA;
   (ii) a capture oligonucleotide containing:
      a template-binding sequence complementary to the second region of the single-stranded circular DNA; and
      a miRNA-binding sequence complementary to the first region of the miRNA;
   (iii) a second single-stranded circular DNA containing:
      the same sequence as the sequence in the first single-stranded circular DNA, complementary to the sequence that binds to the second single-stranded circular DNA;
      a second-primer-binding sequence adj acent to the 5'-side of this sequence; and
      a sequence complementary to a detection reagent-binding sequence; and
   (iv) a second oligonucleotide primer containing:
      the same sequence as the region in the first single-stranded circular DNA, adjacent to the 5'-side of the sequence complementary to the sequence that binds to the second single-stranded circular DNA, wherein said same sequence as the region in the first single-stranded circular DNA is 8 to 15 bases; and
      a sequence of 7 to 8 bases adjacent to the 3'-side of this sequence and complementary to the second-primer-binding sequence of the second single-stranded circular DNA,
wherein
the capture oligonucleotide is bound to a carrier through the 5'-end thereof, and
the second oligonucleotide primer is bound, through the 5'-end thereof, to the carrier to which the capture oligonucleotide is bound.

The method of detecting a target nucleic acid uses this kit. In this case, the miRNA functions as the first primer, and an extended chain is generated therefrom. The second single-stranded circular DNA and the second oligonucleotide primer hybridize with the extended chain to allow the amplification reaction to proceed.

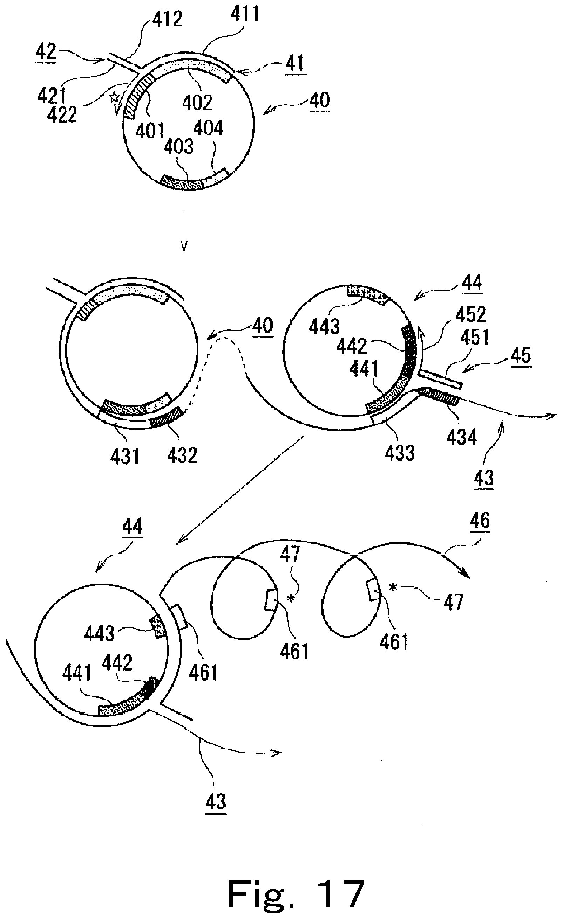

A description is given below showing an example with reference to Fig. 17. The single-stranded circular DNA is illustrated in the 5' -> 3' clockwise direction. For convenience, the carrier is not presented.

A single-stranded circular DNA 40 contains: a sequence (miRNA-binding region) 401 complementary to a second region 422 of a target miRNA 42; a second region 402 linked to the 3'-side thereof; and a sequence 403 complementary to a sequence that binds to a second single-stranded circular DNA.

The sequence 401 has a length of preferably 7 bases or 8 bases. The sequence is not limited and has a GC content of preferably 30 to 70%. The sequence 402 has a length of usually 10 to 30 bases, preferably 15 to 25 bases, and a GC content of preferably 30 to 70%. The total length of the first single-stranded circular DNA 40 is preferably 35 to 100 bases. The first single-stranded circular DNA 40 can be obtained by circularization of a single-stranded DNA (ssDNA) by the method described above.

### <Second Single-Stranded Circular DNA>

A second single-stranded circular DNA 44 contains: the same sequence 441 as the sequence 403 in the first single-stranded circular DNA 40, complementary to the sequence that binds to the second single-stranded circular DNA; a second-primer-binding sequence 442 adjacent to the 5'-side of this sequence; and a sequence 443 complementary to a guanine-quadruplex-forming sequence.

The sequence 441 has a length of usually 10 to 30 bases, preferably 15 to 25 bases, and a GC content of preferably 30 to 70%. The sequence 442 has a length of preferably 7 bases or 8 bases. The sequence is not limited and has a GC content of preferably 30 to 70%. To the sequence 443 complementary to a guanine-quadruplex-forming sequence, the description on the first embodiment in the first mode similarly applies. The total length of the second single-stranded circular DNA 44 is preferably 35 to 100 bases. The second single-stranded circular DNA 44 can be obtained by circularization of a single-stranded DNA (ssDNA) by the method described above.

Although Fig. 17 describes a case where the detection reagent-binding sequence is a guanine-quadruplex-forming sequence, the detection may also be carried out using, as the detection reagent-binding sequence, an aptamer sequence or a sequence that binds to a molecular beacon (hairpin-shaped oligonucleotide having a fluorescent group (donor) and a quenching group (acceptor) that cause FRET), and using, as the detection reagent, an aptamer-binding coloring molecule or the molecular beacon (ChemBioChem 2007, 8, 1795-1803; J. Am. Chem. Soc. 2013, 135, 7430-7433). In the present mode, it is also possible to detect the amplified nucleic acid using, as the detection reagent, a nucleic acid staining reagent which non-sequence-specifically binds to DNA to emit fluorescence, such as Cyber Gold (trade name). Therefore, in the second single-stranded circular DNA, the presence of the sequence complementary to the detection reagent-binding sequence is not indispensable.

### <Capture Oligonucleotide>

The capture oligonucleotide 41 is bound to a carrier through its 5'-end. The mode of each of the capture oligonucleotide 41, its 5'-end, and the carrier is not limited as long as the capture oligonucleotide 41 can be bound to the carrier through its 5'-end, can hybridize with the target miRNA 42, and allows a nucleic acid amplification reaction based on the target miRNA 42 by the rolling circle amplification (RCA) method using the target miRNA 42. To the 5'-end of the capture oligonucleotide 41, and the carrier, the description on the first oligonucleotide primer 22 in the first embodiment in the first mode similarly applies.

### <Second Oligonucleotide Primer>

To the 5'-end of the second oligonucleotide primer 45, and the carrier, the description on the second oligonucleotide primer 25 in the first embodiment in the first mode similarly applies.

The second oligonucleotide primer 45 contains: the same sequence 451 (preferably a sequence of 8 to 15 bases) as a region 404 in the first single-stranded circular DNA 40, adjacent to the 5'-side of the sequence 403 complementary to the sequence that binds to the second single-stranded circular DNA; and a sequence 452 (preferably a sequence of 7 to 8 bases) adjacent to the 3'-side of this sequence and complementary to the second-primer-binding sequence 442 of the second single-stranded circular DNA.

### <Amplification Method>

As illustrated in Fig. 17, after hybridizing the capture oligonucleotide 41 and the first single-stranded circular DNA 40 with the target miRNA 42 to allow formation of a ternary complex, a nucleic acid amplification reaction based on the target polynucleotide is carried out using the rolling circle amplification (RCA) method. The reaction conditions and the like are the same as those for the first embodiment in the first mode.

By the RCA, a first amplification product 43 is amplified dependently on the target miRNA 42 along the first single-stranded circular DNA 40.

The amplification product 43 contains a sequence 431 complementary to the sequence 403 in the first single-stranded circular DNA 40, complementary to the sequence that binds to the second single-stranded circular DNA. Therefore, the second single-stranded circular DNA 44, which contains the same sequence 441 as the sequence 403, hybridizes with the sequence 431 of the first amplification product 43 via the sequence 441.

With the thus formed complex of the first amplification product 43 and the second single-stranded circular DNA, the second oligonucleotide primer 45 hybridizes to form a ternary complex.

More specifically, since the second oligonucleotide primer 45 contains the same sequence 451 as the region 404 in the first single-stranded circular DNA 40, adjacent to the 5'-side of the sequence 403 complementary to the sequence that binds to the second single-stranded circular DNA, the second oligonucleotide primer 45 hybridizes with the region 432, in the first amplification product 43, complementary to the region 404 of the first single-stranded circular DNA 40, via the sequence 451.

Since the second oligonucleotide primer 45 contains, in the 3'-side of the sequence 451, the sequence 452 complementary to the second-primer-binding sequence 442 of the second single-stranded circular DNA 44, the second oligonucleotide primer 45 also hybridizes with the second single-stranded circular DNA 44 via the sequence 452.

By RCA, a second amplification product 46 (extended chain) is amplified from the resulting ternary complex of the first amplification product 43, the second single-stranded circular DNA 44, and the second oligonucleotide primer 45. Since the second amplification product 46 contains a sequence 461 containing a guanine quadruplex, it can be detected with a guanine quadruplex detection reagent 47. In the present embodiment, the second single-stranded circular DNA 44 hybridizes with each region 431 contained in the first amplification product 43, to cause the RCA reaction. Thus, remarkable improvement in the detection sensitivity can be achieved.

In cases where the sequence of the short-chain target nucleic acid hybridizes with the sequence of the capture oligonucleotide, the amplification reaction occurs, so that the short-chain target nucleic acid is detected with the detection reagent. On the other hand, in cases where the sequence of the short-chain target nucleic acid does not hybridize with the sequence of the capture oligonucleotide, the amplification reaction hardly occurs, so that the short-chain target nucleic acid is not detected with the detection reagent.

Thus, by the detection method of the present invention, the sequence, or the presence or absence, of the short-chain target nucleic acid can be identified.

In cases where the short-chain target nucleic acid contains a mutation, when the type of the mutation of the short-chain target nucleic acid matches the type of the base arranged in the capture oligonucleotide sequence, the amplification reaction occurs, so that the mutation is detected with the detection reagent. On the other hand, when the type of the mutation of the short-chain target nucleic acid is different from the type of the base arranged in the capture oligonucleotide sequence, the amplification reaction hardly occurs, so that the mutation is not detected with the detection reagent.

Thus, by the detection method of the present invention, the type of the mutation, or the presence or absence of the mutation, in the short-chain target nucleic acid can be identified.

### <Detection Reagent>

In the method of the present invention, a nucleic acid staining reagent which non-sequence-specifically binds to DNA to emit fluorescence, such as Cyber Gold (trade name), may be used as the detection reagent. However, for specific and highly sensitive detection, it is preferred to use a molecule which binds to a particular nucleic acid sequence (detection reagent-binding sequence) to cause luminescence or coloring. Examples of the detection reagent include guanine-quadruplex-binding reagents such as the ThT derivatives described above. It is preferred to use ThT-PEG or ThT-PEG-ThT as a ThT derivative since the amplification product can be visually observed in this case. For increasing the detection sensitivity, it is preferred to immobilize ThT-PEG or ThT-PEG-ThT on a carrier together with a PEG chain. Further, it is preferred to use ThT-PEG or ThT-PEG-ThT immobilized on a carrier together with a PEG chain, in combination with ThT-PEG and/or ThT-PEG-ThT.

Other details are the same as those in the first embodiment in the first mode.

### <Second Mode>

### <Method of Detecting Target Molecule>

A method of detecting a target molecule according to a second mode of the present invention is a method comprising the steps of:
forming a first complex containing a target molecule, a capture oligonucleotide, a first oligonucleotide primer, and a first single-stranded circular DNA;
performing a nucleic acid amplification reaction by rolling circle amplification based on the formation of the first complex;
hybridizing a second single-stranded circular DNA and a second oligonucleotide primer with an extended chain generated by the nucleic acid amplification reaction, to form a second complex containing the extended chain, the second oligonucleotide primer, and the second single-stranded circular DNA;
performing a nucleic acid amplification reaction by rolling circle amplification based on the formation of the second complex; and
detecting an amplified nucleic acid,
wherein
the first single-stranded circular DNA contains:
   a first region;
   a second region linked to the 3'-side thereof; and
   a sequence complementary to a sequence that binds to the second single-stranded circular DNA;
the first oligonucleotide primer contains:
   a first aptamer sequence which binds to the target molecule; and
   a sequence linked to the 3'-side thereof and complementary to the first region of the first single-stranded circular DNA;
the capture oligonucleotide contains:
   a sequence complementary to the second region of the first single-stranded circular DNA; and
   a second aptamer sequence linked to the 3'-side thereof, which binds to the target molecule;
the second single-stranded circular DNA contains:
   the same sequence as the sequence in the first single-stranded circular DNA, complementary to the sequence that binds to the second single-stranded circular DNA; and
   a sequence which is adjacent to the 5'-side of this sequence and which binds to the second oligonucleotide primer; and
the second oligonucleotide primer contains:
   the same sequence as the region in the first single-stranded circular DNA, adjacent to the 5'-side of the sequence complementary to the sequence that binds to the second single-stranded circular DNA; and
   a sequence adjacent to the 3'-side of this sequence and complementary to the sequence, in the second single-stranded circular DNA, that binds to the second oligonucleotide primer,
wherein
the capture oligonucleotide and/or the first oligonucleotide primer is/are bound to a carrier through the 5'-end(s) thereof, and
the second oligonucleotide primer is bound, through the 5'-end thereof, to the carrier to which the capture oligonucleotide and/or the first oligonucleotide primer is/are bound.

### <Target Molecule>

In the present description, the target molecule is not limited as long as it is a molecule capable of binding to the first aptamer sequence and the second aptamer sequence. The target molecule is preferably a non-nucleic acid molecule, and examples of the molecule include proteins, peptides, and low molecular weight compounds, and also include sugars, vitamins, hormones, and coenzymes.

Examples of the hormones include adrenaline, noradrenaline, angiotensin, atriopeptin, aldosterone, dehydroepiandrosterone, androstenedione, testosterone, dihydrotestosterone, calcitonin, calcitriol, calcidiol, corticotropin, cortisol, dopamine, estradiol, estrone, estriol, erythropoietin, follicle-stimulating hormone, gastrin, ghrelin, glucagon, gonadotropin-releasing hormone, growth hormone-releasing hormone, human chorionic gonadotropin, histamine, human placental lactogen, insulin, insulin-like growth factor, growth hormone, inhibin, leptin, leukotriene , lipotropin, melatonin, orexin, oxytocin, parathyroid hormone, progesterone, prolactin, prolactin-releasing hormone, prostaglandin (prostglandin), renin, serotonin, secretin, somatostatin, thrombopoietin, thyroid-stimulating hormone, thyrotropin-releasing hormone, thyroxine, triiodothyronine, and vasopressin.

Examples of the proteins include blood coagulation factors such as thrombin; virus-derived proteins; cytokines and growth factors (which may also correspond to the above-described hormones); and disease marker proteins such as tumor markers.

Examples of the antibiotics include streptomycin, ampicillin, kanamycin, actinomycin, amphotericin, antimycin, bafilomycin, bleomycin, carbenicillin, chloramphenicol, concanamycin, erythromycin, G418, gentamycin, hygromycin, mitomycin, neomycin, oligomycin, penicillin, puromycin, rapamycin, tetracycline, tobramycin, and valinomycin.

The target molecule may be an isolated molecule, or a molecule contained in a sample derived from an organism species. Examples of such a sample containing a target molecule include samples containing a virus, a prokaryote, or a eukaryote. In cases of vertebrates (including human), examples of the sample include excrements such as feces, urine, and sweat; and body fluids such as blood, semen, saliva, gastric juice, and bile. The sample may also be a tissue surgically removed from a body, or a tissue that has dropped from a body such as a body hair. The sample may also be a sample prepared from a processed product such as a food.

### <First Single-Stranded Circular DNA>

The first single-stranded circular DNA contains: a first region; a second region linked to the 3'-side thereof; and a sequence complementary to a sequence that binds to the second single-stranded circular DNA.

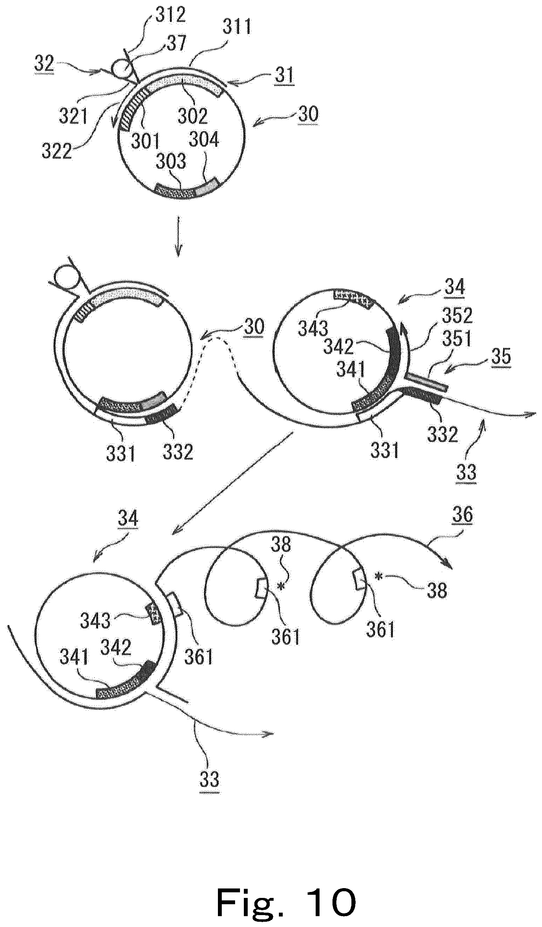

Adescription is given below with reference to Fig. 10. The single-stranded circular DNA is illustrated in the 5' -> 3' clockwise direction. For convenience, the carrier is not presented.

A first single-stranded circular DNA 30 contains: a first region 301 (primer-binding sequence); a second region 302 (sequence complementary to a first region 311 of a capture oligonucleotide 31); and a sequence 303 complementary to a sequence that binds to a second single-stranded circular DNA.

The first region 301 has a length of preferably 7 bases or 8 bases. Its sequence is not limited and has a GC content of preferably 30 to 70%. The second region 302 has a length of usually 10 to 30 bases, preferably 15 to 25 bases, and a GC content of preferably 30 to 70%. The sequence 303 complementary to a sequence that binds to a second single-stranded circular DNA has a length of usually 10 to 30 bases, preferably 15 to 25 bases, and a GC content of preferably 30 to 70%. The total length of the first single-stranded circular DNA 30 is preferably 35 to 100 bases. The first single-stranded circular DNA 30 can be obtained by circularization of a single-stranded DNA (ssDNA) by the method described above.

### <First Oligonucleotide Primer>

The first oligonucleotide primer contains: a first aptamer sequence which binds to a target molecule; and a sequence linked to the 3'-side thereof and complementary to the first region of the first single-stranded circular DNA. The sequence of the first oligonucleotide primer may be a DNA sequence, an RNA sequence, or a mixed sequence of DNA and RNA. As long as the aptamer-binding properties, the hybridization properties, and the extension properties are retained, the sequence may be a sequence further containing a modified nucleic acid or a nucleic acid analog.

In Fig. 10, a first oligonucleotide primer 32 contains: a first aptamer sequence 321 which binds to a target molecule 37; and a sequence 322 linked to the 3'-side thereof and complementary to the first region 301 of the first single-stranded circular DNA 30. The first aptamer sequence 321 has a length of usually 10 to 30 bases, preferably 15 to 25 bases, and a GC content of preferably 30 to 70%. The sequence 322, which is complementary to the first region 301 of the first single-stranded circular DNA 30 has a length of preferably 7 to 8 bases.

The first oligonucleotide primer 32 may be bound to a carrier through its 5'-end.

In this case, the mode of each of the first oligonucleotide primer 32, its 5'-end, and the carrier is not limited as long as the first oligonucleotide primer 32 can be bound to the carrier through its 5'-end and contains the first aptamer sequence 321 which binds to the target molecule 37, and as long as a nucleic acid amplification reaction based on the target molecule 37 can be carried out by the later-described rolling circle amplification (RCA) method using the first oligonucleotide primer 32.

In cases where the first oligonucleotide primer 32 is bound to the carrier through its 5'-end, the description on the first embodiment in the first mode similarly applies to the 5'-end of the first oligonucleotide primer 32, and the carrier.

The first aptamer sequence 321 is a sequence that binds to the target molecule 37 described above. The first aptamer sequence 321 may be a sequence known as an aptamer sequence of the target molecule 37 (for example, a sequence described in the aptamer database described in Nucleic Acids Res (2004) 32 (suppl_1): D95-D100.), or may be a sequence selected using SELEX (Stoltenburg, R. et al. (2007), Biomolecular Engineering 24, pp. 381-403; Tuerk, C. et al., Science 249, pp. 505 to 510; Bock, L. C. et al. (1992), Nature 355, pp. 564-566) or non-SELEX (Berezovski, M. et al. (2006), Journal of the American Chemical Society 128, pp. 1410-1411).

Two kinds of aptamer sequences that bind to the target molecule 37 may be used as the first aptamer sequence 321 and the later-described second aptamer sequence 312.

As the first aptamer sequence 321 and the later-described second aptamer sequence 312, two kinds of sequences may be separately selected. Alternatively, an aptamer sequence which forms a stem-loop structure, bulge-loop structure, or the like and which binds to the target molecule at two sites, may be cleaved in a loop portion to obtain a split aptamer, and the split aptamer may be used as the first aptamer sequence 321 and the second aptamer sequence 312.

### <Capture Oligonucleotide>

The capture oligonucleotide contains: a sequence complementary to the second region of the single-stranded circular DNA; and a second aptamer sequence linked to the 3'-side thereof, which binds to the target molecule. The sequence of the capture oligonucleotide may be a DNA sequence, an RNA sequence, or a mixed sequence of DNA and RNA. As long as the hybridization properties and the aptamer-binding properties are retained, the sequence may be a sequence further containing a modified nucleic acid or a nucleic acid analog.

As illustrated in Fig. 10, the capture oligonucleotide 31 contains: a sequence 311 complementary to the second region 302 of the first single-stranded circular DNA 30; and a second aptamer sequence 312 which is linked to the 3'-side thereof and which binds to the target molecule 37.

The lengths of the sequence 311 complementary to the second region 302 and the second aptamer sequence 312 are usually 10 to 30 bases, preferably 15 to 25 bases, and their GC contents are preferably 30 to 70%.

For preventing occurrence of non-specific extension reaction from the second aptamer sequence 312, the 3'-end of the second aptamer sequence 312 is preferably modified with a phosphate group or the like.

The capture oligonucleotide 31 may be bound to a carrier through its 5'-end.

In this case, the mode of each of the capture oligonucleotide 31, its 5'-end, and the carrier is not limited as long as the capture oligonucleotide 31 can be bound to the carrier through its 5'-end and contains the second aptamer sequence 312 which binds to the target molecule 37, and as long as a nucleic acid amplification reaction based on the target molecule 37 can be carried out by the later-described rolling circle amplification (RCA) method using the first oligonucleotide primer 32.

In cases where the capture oligonucleotide 31 is bound to the carrier through its 5'-end, the description on the first embodiment in the first mode similarly applies to the 5'-end of the capture oligonucleotide 31, and the carrier.

### <Second Single-Stranded Circular DNA>

The second single-stranded circular DNA 34 contains:
the same sequence 341 as the sequence 303 in the first single-stranded circular DNA 30, complementary to the sequence that binds to the second single-stranded circular DNA; and
a second-primer-binding sequence 342 adjacent to the 5'-side of this sequence.

The sequence 303 has a length of usually 10 to 30 bases, preferably 15 to 25 bases, and a GC content of preferably 30 to 70%. The sequence 342 has a length of 7 bases or 8 bases. The sequence is not limited and has a GC content of preferably 30 to 70%. The total length of the second single-stranded circular DNA 34 is preferably 35 to 100 bases. The second single-stranded circular DNA 34 can be obtained by circularization of a single-stranded DNA (ssDNA) by the method described above.

The second single-stranded circular DNA 24 preferably contains a sequence complementary to a detection reagent-binding sequence. To the detection reagent-binding sequence, the description on the first embodiment in the first mode similarly applies.

### <Second Oligonucleotide Primer>

The second oligonucleotide primer 35 contains: the same sequence 351 (preferably a sequence of 8 to 15 bases) as a region 304 in the first single-stranded circular DNA 30, adjacent to the 5'-side of the sequence 303 complementary to the sequence that binds to the second single-stranded circular DNA; and a sequence 352 (preferably a sequence of 7 to 8 bases) adjacent to the 3'-side of this sequence and complementary to the second-primer-binding sequence 342 of the second single-stranded circular DNA 34. The sequence of the second oligonucleotide primer 35 may be a DNA sequence, an RNA sequence, or a mixed sequence of DNA and RNA. As long as the hybridization properties and the extension properties are retained, the sequence may be a sequence further containing a modified nucleic acid or a nucleic acid analog.

The second oligonucleotide primer 35 is bound, through its 5'-end, to the carrier to which the first oligonucleotide primer 32 is bound.

To each of the second oligonucleotide primer 35, its 5'-end, and the carrier, the description on the first embodiment in the first mode similarly applies.

### <Amplification Method>

As illustrated in Fig. 10, in the presence of the target molecule 37, a quaternary complex of the target molecule 37, the capture oligonucleotide 31, the first single-stranded circular DNA 30, and the first oligonucleotide primer 32 is formed, and, as a result, a nucleic acid amplification reaction by the rolling circle amplification (RCA) method occurs. The reaction conditions and the like are the same as those for the first embodiment in the first mode. By the RCA, a first amplification product 33 is amplified dependently on the target molecule 37 from the first oligonucleotide primer 32 along the first single-stranded circular DNA 30.

The first amplification product 33 contains a sequence 331 complementary to the sequence 303 in the first single-stranded circular DNA 30, complementary to the sequence that binds to the second single-stranded circular DNA. Therefore, the second single-stranded circular DNA 34, which contains the same sequence 341 as the sequence 303, hybridizes with the sequence 331 of the first amplification product 33 via the sequence 341.

With the thus formed complex of the first amplification product 33 and the second single-stranded circular DNA 34, the second oligonucleotide primer 35 hybridizes to form a ternary complex.

More specifically, since the second oligonucleotide primer 35 contains the same sequence 351 as the region 304 in the first single-stranded circular DNA 30, adjacent to the 5'-side of the sequence 303 complementary to the sequence that binds to the second single-stranded circular DNA, the second oligonucleotide primer 35 hybridizes with the region 332, in the first amplification product 33, complementary to the region 304 of the first single-stranded circular DNA 30, via the sequence 351.

Since the second oligonucleotide primer 35 contains, in the 3'-side of the sequence 351, the sequence 352 complementary to the second-primer-binding sequence 342 of the second single-stranded circular DNA 34, the second oligonucleotide primer 35 also hybridizes with the second single-stranded circular DNA 34 via the sequence 352.

By RCA, a second amplification product 36 (extended chain) is amplified from the resulting ternary complex of the first amplification product 33, the second single-stranded circular DNA 34, and the second oligonucleotide primer 35. Since the second amplification product 36 contains, for example, a sequence 361 containing a guanine quadruplex, it can be detected with a guanine quadruplex detection reagent 38. The second single-stranded circular DNA 34 hybridizes with each region 331 contained in the first amplification product 33 to cause the RCA reaction. Thus, remarkable improvement in the detection sensitivity can be achieved.

In the presence of a target molecule, a quaternary complex of the target molecule, the capture oligonucleotide, the first single-stranded circular DNA, and the first oligonucleotide primer is formed, and, as a result, amplification reaction occurs to allow detection of the amplification product. On the other hand, in the absence of the target molecule, the amplification reaction does not occur, so that the amplification product is not detected. Accordingly, the detection method of the present invention enables detection and quantification of the target molecule.

### <Detection Reagent>

As described above, the combination of the detection reagent-binding sequence and the detection reagent may be arbitrarily set. Examples of the combination include combinations of an aptamer sequence and an aptamer-binding coloring molecule, combinations of a molecular beacon-binding sequence and a molecular beacon, and combinations of a specific sequence and a labeled probe that hybridizes therewith. The combination is preferably the combination of a guanine quadruplex and a guanine-quadruplex-binding reagent. Examples of the guanine-quadruplex-binding reagent include the reagents described for the first embodiment in the first mode.

Another mode of the present invention is a kit for detecting a target molecule.

According to the invention, the kit for detecting a target molecule comprises the following:
(i) a first single-stranded circular DNA containing:
   a first region;
   a second region linked to the 3'-side thereof; and
   a sequence complementary to a sequence that binds to a second single-stranded circular DNA;
(ii) a first oligonucleotide primer containing:
   a first aptamer sequence which binds to the target molecule; and
   a sequence linked to the 3'-side thereof and complementary to the first region of the first single-stranded circular DNA;
(iii) a capture oligonucleotide containing:
   a sequence complementary to the second region of the first single-stranded circular DNA; and
   a second aptamer sequence linked to the 3'-side thereof, which binds to the target molecule,
(iv) a second single-stranded circular DNA containing:
   the same sequence as the sequence in the first single-stranded circular DNA, complementary to the sequence that binds to the second single-stranded circular DNA; and
   a sequence which is adjacent to the 5'-side of this sequence and which binds to a second oligonucleotide primer; and
(v) a second oligonucleotide primer containing:
   the same sequence as the region in the first single-stranded circular DNA, adjacent to the 5'-side of the sequence complementary to the sequence that binds to the second single-stranded circular DNA, wherein said same sequence as the site in the first single-stranded circular DNA is 8 to 15 bases; and
   a sequence of 7 to 8 bases adjacent to the 3'-side of this sequence and complementary to the sequence, in the second single-stranded circular DNA, that binds to the second oligonucleotide primer,
wherein
the capture oligonucleotide and/or the first oligonucleotide primer is/are bound to a carrier through the 5'-end(s) thereof; and
the second oligonucleotide primer is bound, through the 5'-end thereof, to the carrier to which the capture oligonucleotide and/or the first oligonucleotide primer is/are bound.

The second single-stranded circular DNA may contain a sequence complementary to a detection reagent-binding sequence such as a guanine-quadruplex-forming sequence.

The target-molecule detection kit of the present invention may also contain a detection reagent such as a guanine-quadruplex-binding reagent.

The target-molecule detection kit of the present invention may also contain the above-described crown ether or nonionic surfactant.

Also provided is a compound represented by the following formula: 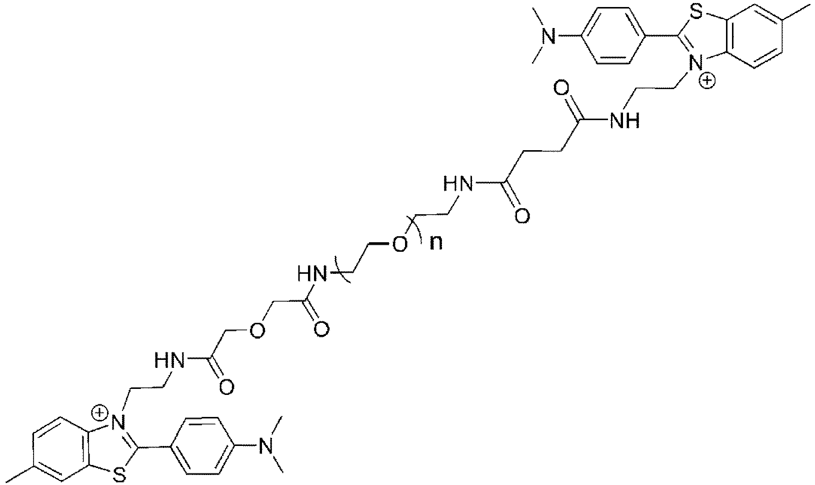 (wherein n represents an integer of 4 to 50).

### EXAMPLES

The present invention is described below concretely by way of Examples. However, the present invention is not limited to these Examples.

### [Example 1]

### (Preparation of Primer-Immobilized Beads)

### (a) Preparation of Biotinylated Primers

A biotinylated primer (P1) and a biotinylated primer (P2) were dissolved in 1×ϕ29 DNA polymerase reaction buffer, to prepare a P1-P2 mixed solution of P1 (1.25 µM) and P2 (5 µM).

The DNA sequence of the primer (P1) is the sequence of SEQ ID NO: 1.

The DNA sequence of the primer (P2) is the sequence of SEQ ID NO:2.

### (b) Providing and Washing of Magnetic Beads before Use

FG beads (FG beads streptavidin) were stirred by vortexing well to obtain uniform particles. Four microliters of the resulting beads were scooped up and placed in a 1.5-mL tube manufactured by Eppendorf. The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting. The above operation was further carried out twice.

### (c) Immobilization of Primers on Magnetic Beads, and Washing

The tube was placed in a magnetic rack (a tube stand with a magnet), to separate the magnetic beads from the supernatant (5 minutes). The supernatant was removed. Four microliters of the P1-P2 mixed solution were added to the beads. Incubation was carried out at 25°C for 30 minutes. During the incubation, vortexing was carried out at 5-minute intervals. The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting. The above operation was further carried out twice. The tube was placed in a magnetic rack (a tube stand with a magnet), to separate the magnetic beads from the supernatant (5 minutes). After removing the supernatant, 40 µL of water was added to the beads, followed by pipetting. The above operation was further carried out twice. The prepared beads were stored under refrigeration until use.

### (Reaction Using Primer-Immobilized Beads)

Two microliters of the primer (P1-P2)-immobilized FG beads were scooped up and placed in a 0.5-mL tube manufactured by Eppendorf. The tube was placed in a magnetic rack (a tube stand with a magnet), to separate the magnetic beads from the supernatant (1 minute). The supernatant was removed. After adding 18 µL of SATIC reagent thereto, 2 µL (10,000, 1000, or 100 fM) of CidR_ 40 (40-mer) as a target RNA or ArfR_39 (39-mer) as a non-target RNA was further added thereto. The reaction was allowed to proceed at 37°C for 2 hours.

The DNA sequence of the first single-stranded circular DNA (cT1) is the sequence of SEQ ID NO:3, which is circularized by binding both ends to each other.

The DNA sequence of the second single-stranded circular DNA (cT2) is the sequence of SEQ ID NO:4, which is circularized by binding both ends to each other.

The RNA sequence of the target RNA CidR_40 (40-mer) is the sequence of SEQ ID NO: 5.

The RNA sequence of the non-target RNA ArfR_ 39 (39-mer) is the sequence of SEQ ID NO:6.

### (Observation under Fluorescence Microscope)

On a slide glass, 20 µL of the reaction solution was placed, and a cover glass was placed thereon. Fluorescence observation was carried out using a fluorescence microscope (Keyence BZ-700) (lens, ×60 magnification; imaging speed, 1/2.3 second; excitation light wavelength (420 nm ± 30 nm), cut-off filter wavelength, 480 nm).

**[Table 1]**

| Table 1. Final concentrations of the SATIC reagent and the primers in the reaction solution | |
|---|---|
| Component of the reaction solution | Concentration |
| cT1 | 10 nM |
| cT2 | 40 nM |
| P1 | 0.25 pmol/tube |
| P2 | 1.0 pmol/tube |
| *φ* 29 DNA polymerase reaction buffer | 1 × |
| BSA | 0. 1 mg/ *µ* L |
| *φ* 29 DNA polymerase | 0.2 U/ *µ* L |
| ThT-HE | 10 *µ*M |

### (Results)

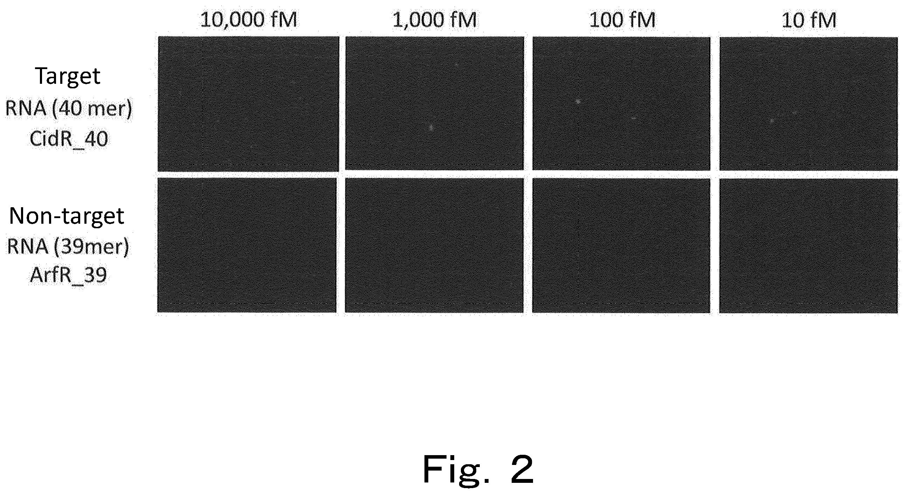

The results are shown in Fig. 2. Detection was successful even in the case where the concentration of the target RNA CidR_40 (40-mer) was 10 fM. More sensitive detection was possible compared to the case of Comparative Example 1, which is described later. On the other hand, no fluorescence was found for the non-target RNAArfR_39 (39-mer).

### [Example 2]

### <Study on Ratio of Amount of Immobilization on Beads>

### (Preparation of Primer-Immobilized Beads)

### (a) Preparation of Biotinylated Primers

P1-P2 mixed solutions of biotinylated primers having the concentrations described in Table 2 were prepared. The biotinylated primers were dissolved in 1×ϕ29 DNA polymerase reaction buffer.

**[Table 2]**

| Table 2. Concentrations of the biotinylated primers | | | | | | | |
|---|---|---|---|---|---|---|---|
| Condition | I | II | III | IV | V | VI | VII |
| P1 | 1 *µ*M | 1 *µ*M | 1 *µ*M | 20 *µ*M | 5 *µ*M | 1 *µ*M | 5 *µ*M |
| P2 | 20 *µ*M | 10 *µ*M | 5 *µ*M | 1 *µ*M | 1 *µ*M | 1 *µ*M | 5 *µ*M |

### (b) Providing and Washing of Magnetic Beads before Use

The same operation as in Example 1 was carried out.

### (c) Immobilization of Primers on Magnetic Beads, and Washing

The tube was placed in a magnetic rack (a tube stand with a magnet), to separate the magnetic beads from the supernatant (5 minutes). After removing the supernatant, the same operation as in Example 1 was carried out except that 4 µL of each of the P1-P2 mixed solutions (I to VII) was added to the beads.

### (Reaction Using Primer-Immobilized Beads)

The same operation as in Example 1 was carried out except that the non-target RNA ArfR_ 39 (39-mer) was not used.

### (Observation under Fluorescence Microscope)

The same operation as in Example 1 was carried out.

**[Table 3]**

| Table 3. Final concentrations of the SATIC reagent and the primers in the reaction solution | |
|---|---|
| Component of the reaction solution | Concentration |
| cT1 | 10 nM |
| cT2 | 40 nM |
| P1 | As shown in Table 4 |
| P2 | As shown in Table 4 |
| *φ* 29 DNA polymerase reaction buffer | 1 × |
| BSA | 0.1 mg/ *µ* L |
| *φ* 29 DNA polymerase | 0.2 U/ *µ* L |
| ThT-HE | 10 *µ* M |

**[Table 4]**

| Table 4 | | | | | | | |
|---|---|---|---|---|---|---|---|
| Condition | A | B | C | D | E | F | G |
| P1 | 0.20 | 0.20 | 0.20 | 4.0 | 1.0 | 0.20 | 1.0 |
| P2 | 4.0 | 2.0 | 1.0 | 0.20 | 0.20 | 0.20 | 1.0 |

| | | | | | | | |
|---|---|---|---|---|---|---|---|
| Unit: pmol/tube | | | | | | | |

### (Results)

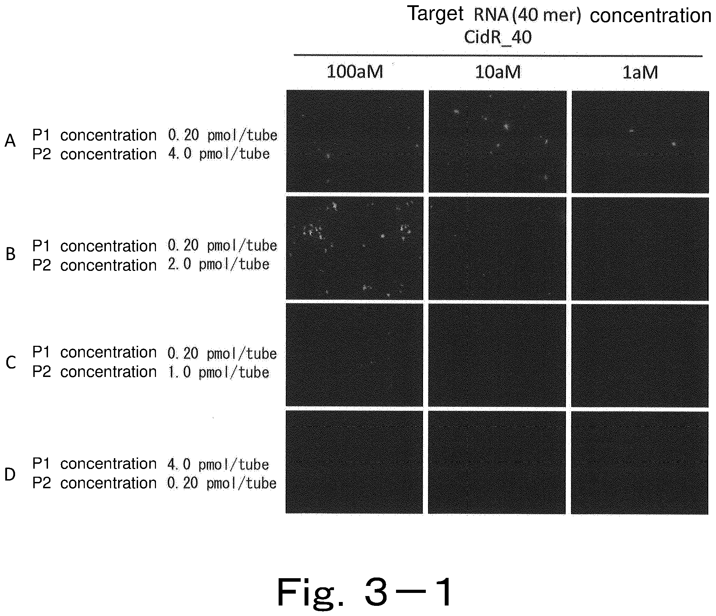

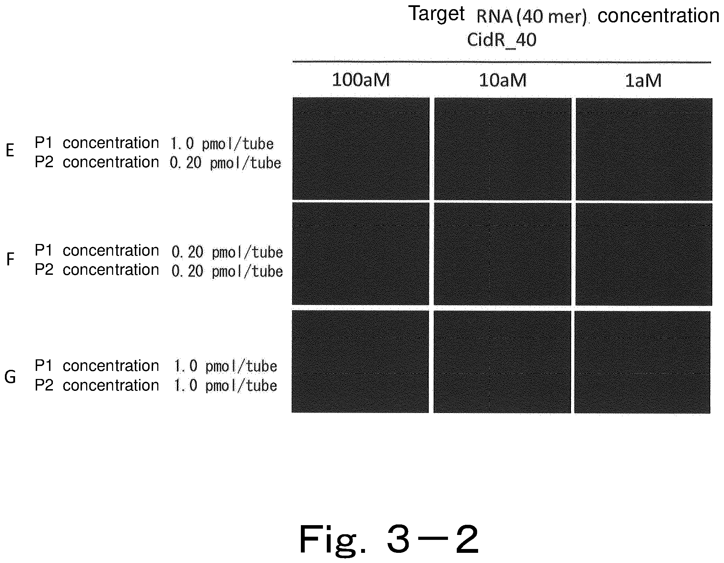

The results are shown in Fig. 3-1 and Fig. 3-2. Under Condition A, the target RNA CidR_40 (40-mer) could be detected at a concentration of as low as 1 aM. Under Condition B, the target RNA CidR_40 (40-mer) could be detected at a concentration of 10 aM.

### [Example 3]

### <Study on Template Concentration Ratio>

### (Preparation of Primer-Immobilized Beads)

Primer-immobilized FG beads prepared using a biotinylated primer P1-P2 mixed solution by employing Condition A in Table 4 in Example 2 were used for the reaction.

### (Reaction Using Primer-Immobilized Beads)

The same operation as in Example 1 was carried out except that the non-target RNAArfR _39 (39-mer) was not used, and that a target RNA CidR_1298 (full length) or a non-target RNA ArfR_2642 (full length) was additionally used.

The RNA sequence of the target RNA CidR_1298 (full length) is the sequence of SEQ ID NO:7.

The RNA sequence of the non-target RNA ArfR_2642 (full length) is the sequence of SEQ ID NO:8.

### (Observation under Fluorescence Microscope)

The same operation as in Example 1 was carried out.

**[Table 5]**

| Table 5. Final concentrations of the SATIC reagent and the primers in the reaction solution | |
|---|---|
| Component of the reaction solution | Concentration |
| cT1 | As shown in Table 6 |
| cT2 | As shown in Table 6 |
| P1 | 0.20 pmol/tube |
| P2 | 4.0 pmol/tube |
| *φ* 29 DNA polymerase reaction buffer | 1 × |
| BSA | 0.1 mg/ *µ* L |
| *φ* 29 DNA polymerase | 0.2 U/ *µ* L |
| ThT-HE | 10 *µ* M |

**[Table 6]**

| Table 6 | | | | | | | | | |
|---|---|---|---|---|---|---|---|---|---|
| Condition | H | I | J | K | L | M | N | 0 | P |
| cT1 | 100 nM | 10 nM | 1 nM | 100 nM | 10 nM | 1 nM | 100 nM | 10 nM | 1 nM |
| cT2 | 400 nM | 400 nM | 400 nM | 40 nM | 40 nM | 40 nM | 4 nM | 4 nM | 4 nM |

### (Results)

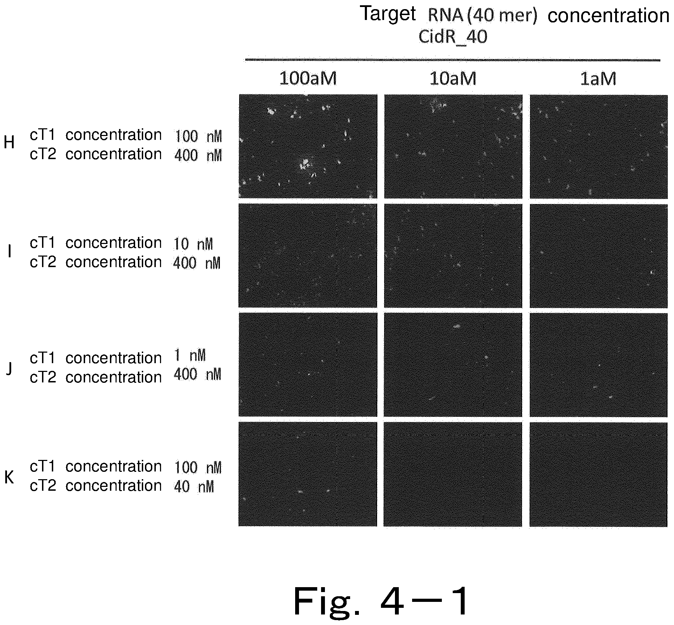

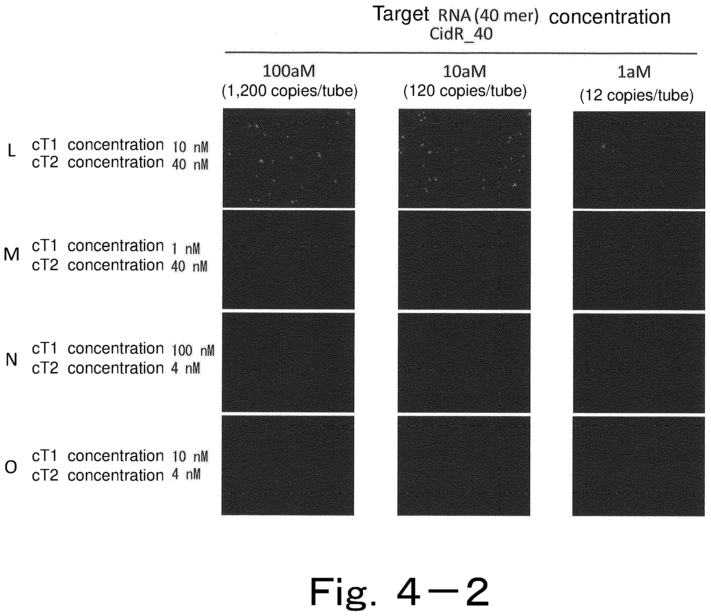

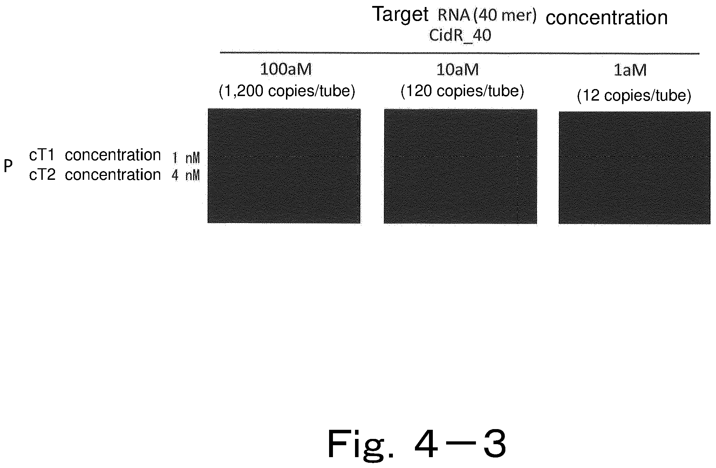

The results are shown in Fig. 4-1, Fig. 4-2, and Fig. 4-3. The strongest fluorescence was found in the case of Condition H. Under Conditions H, I, and J, when the cT2 concentration was 400 nM, the target RNA CidR_40 (40-mer) could be detected at a concentration of as low as 1 aM even in the cases where the cT1 concentration varied within the range of 1 to 100 nM.

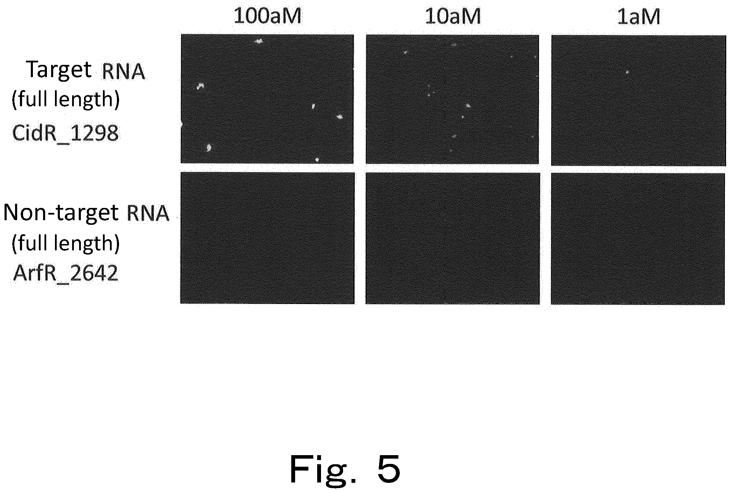

Moreover, as shown in Fig. 5, under Condition H, the target RNA CidR_1298 (full length) could be distinguished from the non-target RNA ArfR_2642 (full length).

In the conventional SATIC method, in which the reaction is carried out in a solution, the detection limit is 1 pM. In contrast, by using Condition H, the present method succeeded in detection of the target RNA CidR_ 40 (40-mer) at 1 aM. Thus, the present method can be said to have a million times higher detection sensitivity relative to that of the conventional method.

### [Comparative Example 1]

### (Preparation of Primer-Immobilized Beads)

### (a) Preparation of Biotinylated Primer

The biotinylated primer (P2) was dissolved in 1×ϕ29 DNA polymerase reaction buffer, to prepare a 5-µM solution.

### (b) Providing and Washing of Magnetic Beads before Use

The same operation as in Example 1 was carried out.

### (c) Immobilization of Primers on Magnetic Beads, and Washing

The tube was placed in a magnetic rack (a tube stand with a magnet), to separate the magnetic beads from the supernatant (5 minutes). After removing the supernatant, the same operation as in Example 1 was carried out except that 4 µL of 5 µM biotinylated primer (P2) was added to the beads.

### (Reaction Using Primer-Immobilized Beads)

The same operation as in Example 1 was carried out except that 2 µL of the primer (P2)-immobilized FG beads were scooped up and placed in a 0.5-mL tube manufactured by Eppendorf.

### (Observation under Fluorescence Microscope)

The same operation as in Example 1 was carried out.

**[Table 7]**

| Table 7. Final concentrations of the SATIC reagent and the primers in the reaction solution | |
|---|---|
| Component of the reaction solution | Concentration |
| cT1 | 10 nM |
| cT2 | 40 nM |
| P1 | 12 nM |
| P2 | 1.0 pmol/tube |
| *φ* 29 DNA polymerase reaction buffer | 1 × |
| BSA | 0.1 mg/*µ*L |
| *φ* 29 DNA polymerase | 0.2 U/ *µ* L |
| ThT-HE | 10 *µ*M |

### (Results)

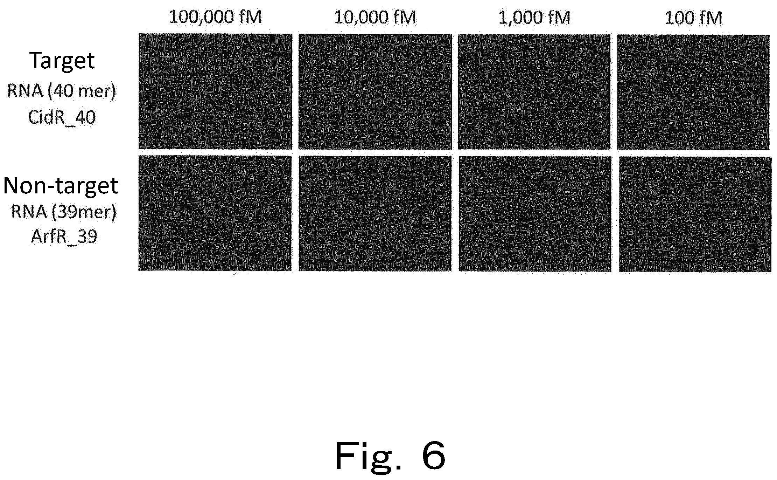

The results are shown in Fig. 6. Fluorescence was found in the case where the concentration of the target RNA CidR_40 (40-mer) was 10,000 fM. On the other hand, no fluorescence was found for the non-target RNA ArfR_ 39 (39-mer).

### [Comparative Example 2]

### (Preparation of Primer-Immobilized Beads)

### (a) Preparation of Biotinylated Primer

The biotinylated primer (P1) was dissolved in 1×ϕ29 DNA polymerase reaction buffer, to prepare a 1.25-µM solution. Further, the biotinylated primer (P2) was dissolved in 1×ϕ29 DNA polymerase reaction buffer, to prepare a 5-µM solution.

### (b) Providing and Washing of Magnetic Beads before Use

The same operation as in Example 1 was carried out.

### (c) Immobilization of Primers on Magnetic Beads, and Washing

The tube was placed in a magnetic rack (a tube stand with a magnet) to separate the magnetic beads from the supernatant (5 minutes). After removing the supernatant, the same operation as in Example 1 was carried out except that 4 µL of 1.25 µM biotinylated primer (P1) or 4 µL of 5 µM biotinylated primer (P2) was added to the beads.

### (Reaction Using Primer-Immobilized Beads)

The same operation as in Example 1 was carried out except that 2 µL of the primer (P1)-immobilized FG beads and 2 µL of the primer (P2)-immobilized FG beads were scooped up and placed in a 0.5-mL tube manufactured by Eppendorf.

### (Observation under Fluorescence Microscope)

The same operation as in Example 1 was carried out.

**[Table 8]**

| Table 8. Final concentrations of the SATIC reagent and the primers in the reaction solution | |
|---|---|
| Component of the reaction solution | Concentration |
| cT1 | 10 nM |
| cT2 | 40 nM |
| P1 | 0.25 pmol/tube |
| P2 | 1.0 pmol/tube |
| *φ* 29 DNA polymerase reaction buffer | 1 × |
| BSA | 0.1 mg/ *µ*L |
| *φ* 29 DNA polymerase | 0.2 U/*µ*L |
| ThT-HE | 10 *µ*M |

### (Results)

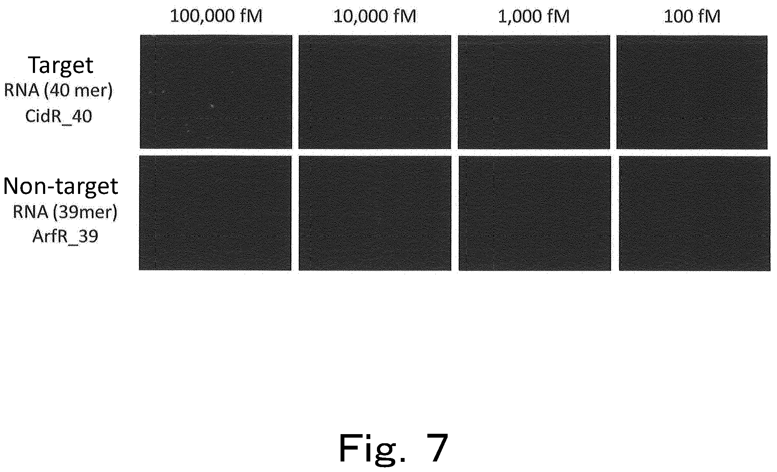

The results are shown in Fig. 7. Fluorescence was found in the case where the concentration of the target RNA CidR_40 (40-mer) was 100,000 fM. On the other hand, no fluorescence was found for the non-target RNA ArfR_39 (39-mer). The detection sensitivity was lower than that in Comparative Example 1.

### [Reference Example 1]

### <Preparation of Calibration Curves for Detection System>

### (Preparation of Primer-Immobilized Beads)

Primer-immobilized FG beads prepared using a biotinylated primer P1-P2 mixed solution by employing Condition A in Table 4 in Example 2 were used for the reaction.

### (Reaction Using Primer-Immobilized Beads)

The same operation as in Example 1 was carried out except that 2 µL (10,000, 1000, 100, or 10 aM) of the target RNA (40-mer) or the non-target RNA (39-mer), or the target RNA (full length) or the non-target RNA (full-length) was used.

### (Observation under Fluorescence Microscope)

On a 96-well plate (V-bottom plate, IWAKI MICROPLATE 3420-096), 3 µL of each reaction solution was placed, and the beads were collected using a magnet. Fluorescence observation was carried out using a fluorescence microscope (Keyence BZ-700) (lens, ×4 magnification; imaging speed, 1/8.5 second; excitation light wavelength (420 nm ± 30 nm), cut-off filter wavelength, 480 nm).

**[Table 9]**

| Table 9. Final concentrations of the SATIC reagent and the primers in the reaction solution | |
|---|---|
| Component of the reaction solution | Concentration |
| cT1 | 100 nM |
| cT2 | 400 nM |
| P1 | 0.20 pmol/tube |
| P2 | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-HE | 10 *µ*M |

### (Results)

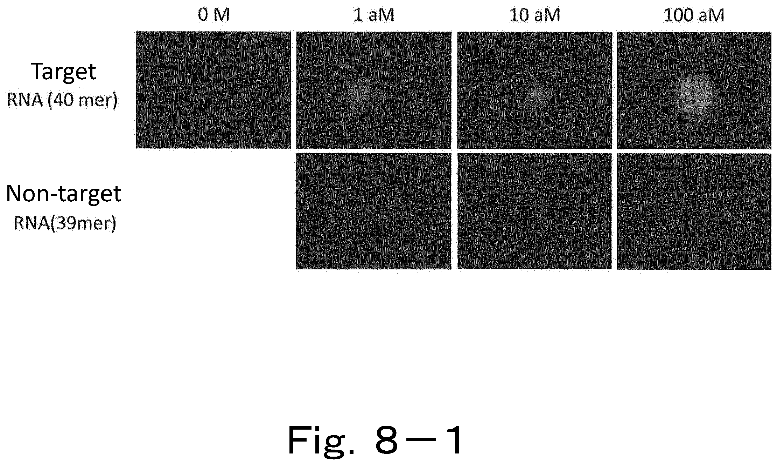

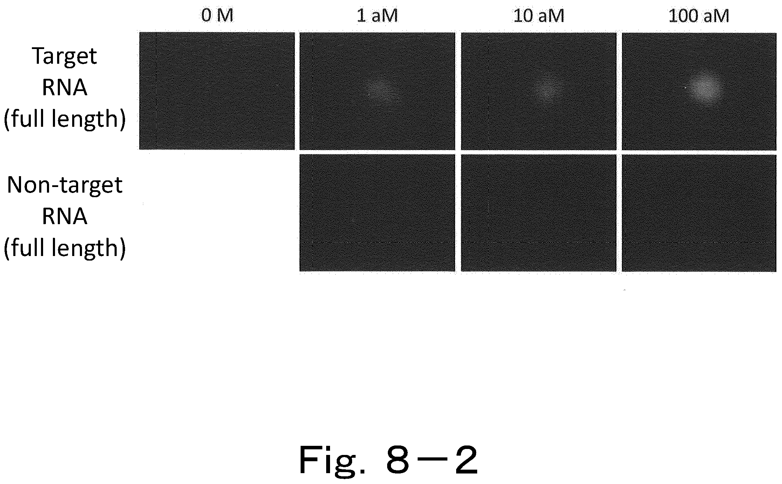

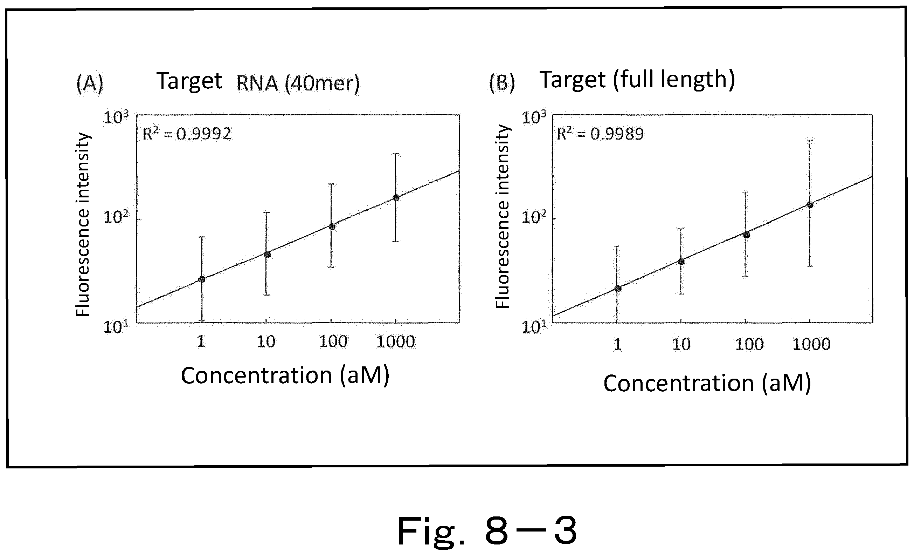

The results are shown in Fig. 8-1, Fig. 8-2, Fig. 8-3(A), and Fig. 8-3(B). By collecting the beads, quantitative measurement of the fluorescence intensity was made possible. More specifically, using the target RNA (40-mer) and the target RNA (full length), calibration curves within the range of 1 aM to 1000 aM could be prepared, and quantitative analysis was made possible therewith.

### [Example 4]

### (Preparation of Primer-Immobilized Beads)

Primer-immobilized FG beads prepared using a biotinylated primer P1-P2 mixed solution by employing Condition A in Table 4 in Example 2 were used for the reaction.

### (Reaction Using Primer-Immobilized Beads)

The same operation as in Example 1 was carried out except that 2 µL (10,000, 1000, 100, or 10 aM) of a target double-stranded DNA (40 bp) CidD_40 (SEQ ID NO:9) or a target double-stranded DNA (full length) CidD_1298 (SEQ ID NO:10), or a non-target double-stranded DNA (39 bp) ArfD_39 (SEQ ID NO:11) or a non-target double-stranded DNA (full length) ArfD_2642 (SEQ ID NO:12) was used instead of the target RNA CidR_40 (40-mer) or the non-target RNA ArfR_39 (39-mer).

The DNA sequence of the first single-stranded circular DNA (cT1-9bp) is the sequence of SEQ ID NO:13, which is circularized by binding both ends to each other.

The DNA sequence of the second single-stranded circular DNA (cT2) is the sequence of SEQ ID NO:4, which is circularized by binding both ends to each other.

### (Observation under Fluorescence Microscope)

On a 96-well plate (V-bottom plate, IWAKI MICROPLATE 3420-096), 3 µL of each reaction solution was placed, and the beads were collected using a magnet. Fluorescence observation was carried out using a fluorescence microscope (Keyence BZ-700) (lens, ×4 magnification; imaging speed, 1/8.5 seconds; excitation light wavelength (420 nm ± 30 nm), cut-off filter wavelength, 480 nm).

**[Table 10]**

| Table 10. Final concentrations of the SATIC reagent and the primers in the reaction solution | |
|---|---|
| Component of the reaction solution | Concentration |
| cT1-9bp | 100 nM |
| cT2 | 400 nM |
| P1 | 0.20 pmol/tube |
| P2 | 4.0 pmol/tube |
| *φ* 29 DNA polymerase reaction buffer | 1 × |
| BSA | 0. 1 mg/ *µ*L |
| dNTPs | 1 mM |
| *φ* 29 DNA polymerase | 0.2 U/ *µ*L |
| ThT-HE | 10 *µ*M |

### (Results)

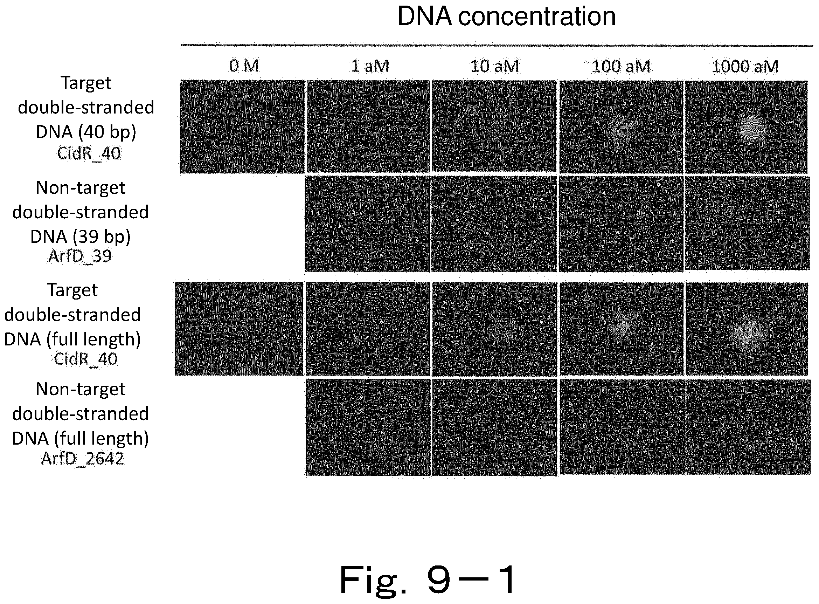

The results are shown in Fig. 9-1. By collecting the beads, quantitative measurement of the fluorescence intensity was made possible. For the target double-stranded DNA (40 bp) CidD_40 and the target double-stranded DNA (full length) CidD_1298, fluorescence could be found within the range of 1 aM to 1000 aM. On the other hand, fluorescence was found for neither the non-target double strand (39 bp) ArfD_39 nor the non-target double-stranded DNA (full length) ArfD_2642.

### [Reference Example 2]

Based on the results of Example 4, calibration curves were prepared for the detection system.

### (Results)

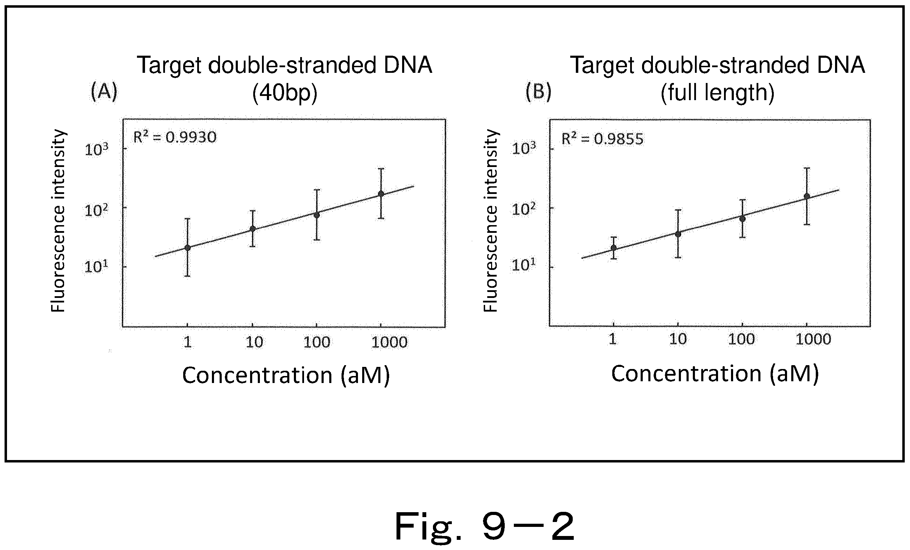

The results are shown in Fig. 9-2. Calibration curves within the range of 1 aM to 1000 aM could be prepared, and quantitative analysis was possible therewith.

### [Example 5]

### (Preparation of Beads on Which Capture Oligonucleotide and Primer Are Immobilized)

### (a) Preparation of Biotinylated Capture Oligonucleotide and Biotinylated Primer

A biotinylated capture oligonucleotide (CS-mir-21ca) and a biotinylated primer (P2) were dissolved in 1×ϕ29 DNA polymerase reaction buffer, to prepare a CS-mir-21ca-P2 mixed solution of CS-mir-21ca (1 µM) and P2 (20 µM).

The DNA sequence of the capture oligonucleotide (CS-mir-21ca) is the sequence of SEQ ID NO: 14. Its 3'-end is phosphorylated.

The DNA sequence of the primer (P2) is the sequence of SEQ ID NO:2.

### (b) Providing and Washing of Magnetic Beads before Use

The same operation as in Example 1 was carried out.

### (c) Immobilization of Capture Oligonucleotide and Primer on Magnetic Beads, and Washing

The tube was placed in a magnetic rack (a tube stand with a magnet), to separate the magnetic beads from the supernatant (5 minutes). After removing the supernatant, the same operation as in Example 1 was carried out except that 4 µL of the CS-mir-21ca-P2 mixed solution was added to the beads.

### (Reaction Using Beads on Which Capture Oligonucleotide and Primer Are Immobilized)

The same operation as in Example 1 was carried out except that 2 µL of the CS-mir-21ca-primer (P2)-immobilized FG beads were scooped up, and that 2 µL (10 nM) of a target miR-21CA (SEQ ID NO: 15), or a non-target miR-21(SEQ ID NO: 16) or miR-221 (SEQ ID NO: 17) was used.

The DNA sequence of the first single-stranded circular DNA (cT1-mir-21ca) is the sequence of SEQ ID NO: 18, which is circularized by binding both ends to each other.

The DNA sequence of the second single-stranded circular DNA (cT2) is the sequence of SEQ ID NO:4, which is circularized by binding both ends to each other.

The DNA sequence of the primer (P1-thr) is the sequence of SEQ ID NO: 19.

### (Observation under Fluorescence Microscope)

The same operation as in Example 4 was carried out.

**[Table 11]**

| Table 11. Final concentrations of the SATIC reagent and the primers in the reaction solution | |
|---|---|
| Component of the reaction solution | Concentration |
| cT1-mir-21ca | 100 nM |
| cT2 | 400 nM |
| CS-mir-21ca | 0.20 pmol/tube |
| P2 | 4.0 pmol/tube |
| *φ* 29 DNA polymerase reaction buffer | 1 × |
| BSA | 0.1 mg/ *µ*L |
| *φ* 29 DNA polymerase | 0.2 U/*µ*L |
| ThT-HE | 10 *µ*M |

### (Results)

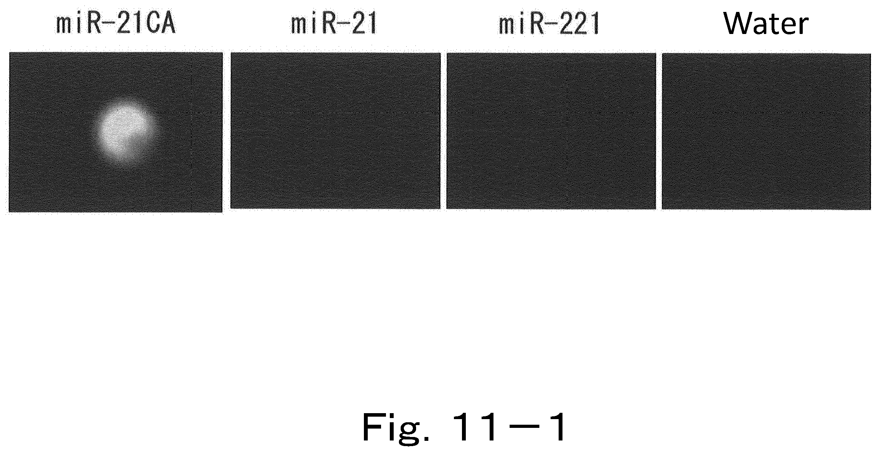

The results are shown in Fig. 11-1. In the case where the target miR-21CA was used, fluorescence was found. However, in the cases where the non-target miR-21 or miR-221 was used, no fluorescence was found. In other words, the presence of the target miR-21CA could be specifically detected.

### [Reference Example 3]

Beads on which a capture oligonucleotide and a primer are immobilized were prepared in the same manner as in Example 5. Separately, the following primer-immobilized beads were prepared.

### (Preparation of Primer-Immobilized Beads)

### (a) Preparation of Biotinylated Primer

The biotinylated primer (P2) was dissolved in 1×ϕ29 DNA polymerase reaction buffer, to prepare a 20-µM P2 solution.

### (b) Providing and Washing of Magnetic Beads before Use

The same operation as in Example 1 was carried out.

### (c) Immobilization of Primer on Magnetic Beads, and Washing

The tube was placed in a magnetic rack (a tube stand with a magnet), to separate the magnetic beads from the supernatant (5 minutes). After removing the supernatant, the same operation as in Example 1 was carried out except that 4 µL of the P2 solution was added to the beads.

### (Reaction Using Beads on Which Capture Oligonucleotide and Primer Are Immobilized, or Using Primer-Immobilized Beads)

The same operation as in Example 1 was carried out except that 2 µL of the CS-mir-21ca-primer (P2)-immobilized FG beads or 2 µL of the primer (P2)-immobilized FG beads were scooped up, and that 2 µL (1, 10, 100, or 1000 fM) of the target miR-21CA was used.

### (Observation under Fluorescence Microscope)

The same operation as in Example 5 was carried out.

**[Table 12]**

| Table 12. Final concentrations of the SATIC reagent and the primers in the reaction solution | |
|---|---|
| Component of the reaction solution | Concentration |
| cT1-mir-21ca | 100 nM |
| cT2 | 400 nM |
| CS-mir-21ca | 0.20 pmol/tube |
| P2 | 4.0 pmol/tube |
| *φ* 29 DNA polymerase reaction buffer | 1 × |
| BSA | 0.1 mg/ *µ*L |
| *φ* 29 DNA polymerase | 0.2 U/*µ*L |
| ThT-HE | 10 *µ*M |

### (Results)

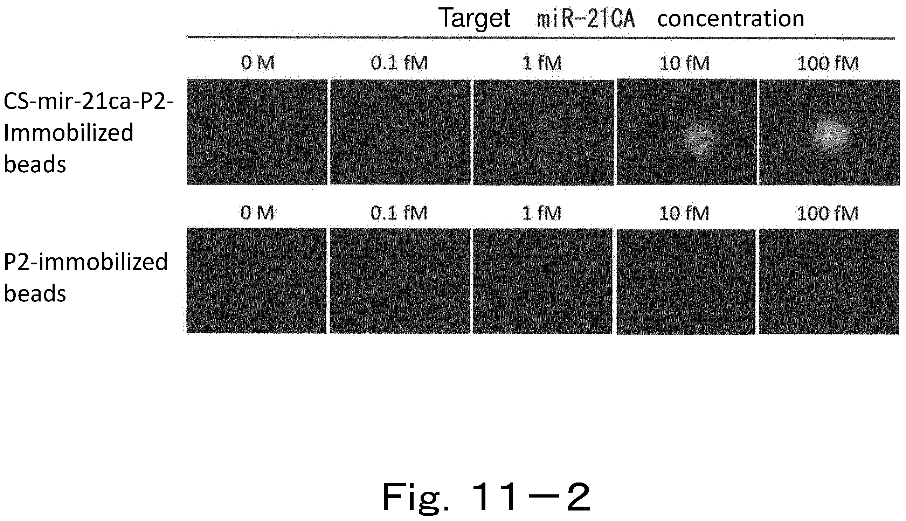

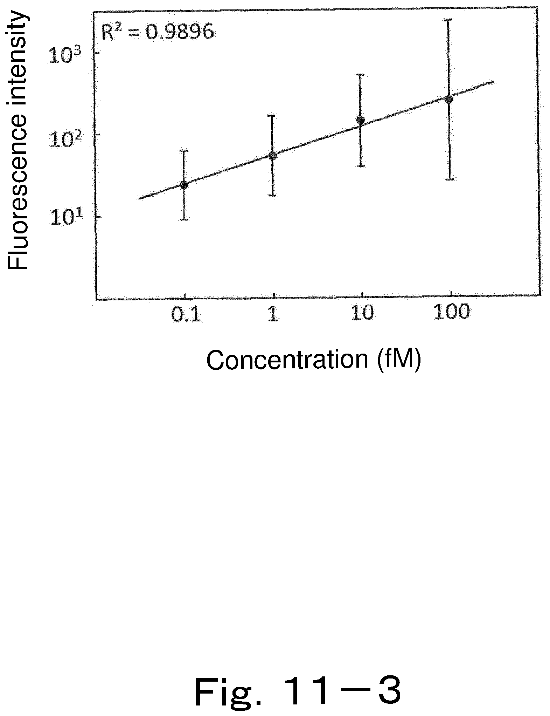

The results are shown in Fig. 11-2 and Fig. 11-3. By collecting the beads, quantitative measurement of the fluorescence intensity was made possible.

In the case where the CS-mir-21ca-primer (P2)-immobilized FG beads were used, a calibration curve within the concentration range of 0.1 fM to 100 fM could be prepared for the target miR-21CA, and quantitative analysis was possible therewith.

On the other hand, in the case where the primer (P2)-immobilized FG beads were used, no fluorescence could be found for the target miR-21CA after similarly performing the reaction at concentrations of 0.1 fM to 100 fM.

It is assumed that, by using the beads on which the capture oligonucleotide is immobilized, the first-stage reaction occurred in the vicinities of the beads, and therefor that the extension product of the first-stage reaction tended to be present in the vicinities of the beads (it is thought that, in the case where only P2 was immobilized, the first-stage reaction proceeded in the solution, and therefore that the extension product of the first-stage reaction was less likely to gather in the vicinities of the beads ). It is thus thought that the second-stage reaction proceeded more smoothly even without immobilization of the extension product of the first-stage reaction. The detection sensitivity was (about 5000 times) higher than that in the case by the conventional method using no beads (that is, the solution system), whose sensitivity was 500 fM.

### [Example 6]

### (Preparation of Beads on Which Capture Oligonucleotide and Primer Are Immobilized)

### (a) Preparation of Biotinylated Capture Oligonucleotide and Biotinylated Primer

A biotinylated capture nucleotide (CS-mir-13b) and a biotinylated primer (P2) were dissolved in 1×ϕ29 DNA polymerase reaction buffer, to prepare a CS-mir-13b-P2 mixed solution of CS-mir-13b (1 µM) and P2 (20 µM).

The DNA sequence of the capture nucleotide (CS-mir-13b) is the sequence of SEQ ID NO:20. Its 3'-end is phosphorylated.

The DNA sequence of the primer (P2) is the sequence of SEQ ID NO:2.

### (b) Providing and Washing of Magnetic Beads before Use

The same operation as in Example 1 was carried out.

### (c) Immobilization of Capture Oligonucleotide and Primer on Magnetic Beads, and Washing

The tube was placed in a magnetic rack (a tube stand with a magnet), to separate the magnetic beads from the supernatant (5 minutes). After removing the supernatant, the same operation as in Example 1 was carried out except that 4 µL of the CS-mir-13b-P2 mixed solution was added to the beads.

### (Reaction Using Beads on Which Capture Oligonucleotide and Primer Are Immobilized)

The same operation as in Example 1 was carried out except that 2 µL of the CS-mir-13b-primer (P2)-immobilized FG beads were scooped up, and that 2 µL (10 nM) of a target miR-13b (SEQ ID NO:21), or a non-target miR-13a (SEQ ID NO:22) or miR-221 (SEQ ID NO: 17) was used.

The DNA sequence of the first single-stranded circular DNA (cT 1-mir-13b) is the sequence of SEQ ID NO:23, which is circularized by binding both ends to each other.

The DNA sequence of the second single-stranded circular DNA (cT2) is the sequence of SEQ ID NO:4, which is circularized by binding both ends to each other.

### (Observation under Fluorescence Microscope)

The same operation as in Example 4 was carried out.

**[Table 13]**

| Table 13. Final concentrations of the SATIC reagent and the primers in the reaction solution | |
|---|---|
| Component of the reaction solution | Concentration |
| cT1-mir-13b | 100 nM |
| cT2 | 400 nM |
| CS-mir-13b | 0.20 pmol/tube |
| P2 | 4.0 pmol/tube |
| *φ* 29 DNA polymerase reaction buffer | 1 × |
| BSA | 0.1 mg/ *µ*L |
| *φ* 29 DNA polymerase | 0.2 U/*µ*L |
| ThT-HE | 10 *µ*M |

### (Results)

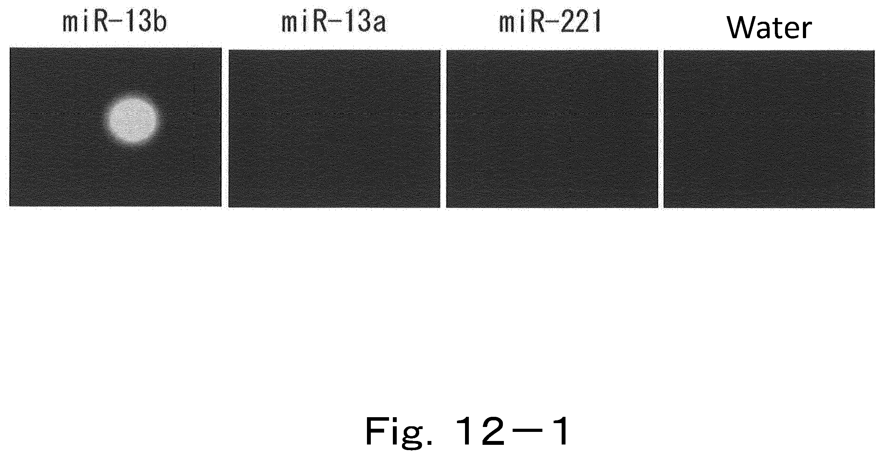

The results are shown in Fig. 12-1. In the case where the target miR-13b was used, fluorescence was found. However, in the cases where the non-target miR-13a or miR-221 was used, no fluorescence was found. It should be noted that only one base is different between the base sequence of miR-13b and the base sequence of miR-13a. In other words, the presence of the target miR-13b, including the one-base difference, could be specifically detected.

### [Reference Example 4]

Beads on which a capture oligonucleotide and a primer are immobilized were prepared in the same manner as in Example 6. In addition, primer (P2)-immobilized beads were prepared in the same manner as in Reference Example 3.

### (Reaction Using Beads on Which Capture Oligonucleotide and Primer Are Immobilized, or Using Primer-Immobilized Beads)

The same operation as in Example 1 was carried out except that 2 µL of the CS-mir-13b-primer (P2)-immobilized FG beads or 2 µL of the primer (P2)-immobilized FG beads were scooped up, and that 2 µL (1, 10, 100, or 1000 fM) of the target miR-13b was used.

### (Observation under Fluorescence Microscope)

The same operation as in Example 4 was carried out.

**[Table 14]**

| Table 14. Final concentrations of the SATIC reagent and the primers in the reaction solution | |
|---|---|
| Component of the reaction solution | Concentration |
| cT1-mir-13b | 100 nM |
| cT2 | 400 nM |
| CS-mir-13b | 0.20 pmol/tube |
| P2 | 4.0 pmol/tube |
| *φ* 29 DNA polymerase reaction buffer | 1 × |
| BSA | 0.1 mg/ *µ*L |
| *φ* 29 DNA polymerase | 0.2 U/*µ*L |
| ThT-HE | 10 *µ*M |

### (Results)

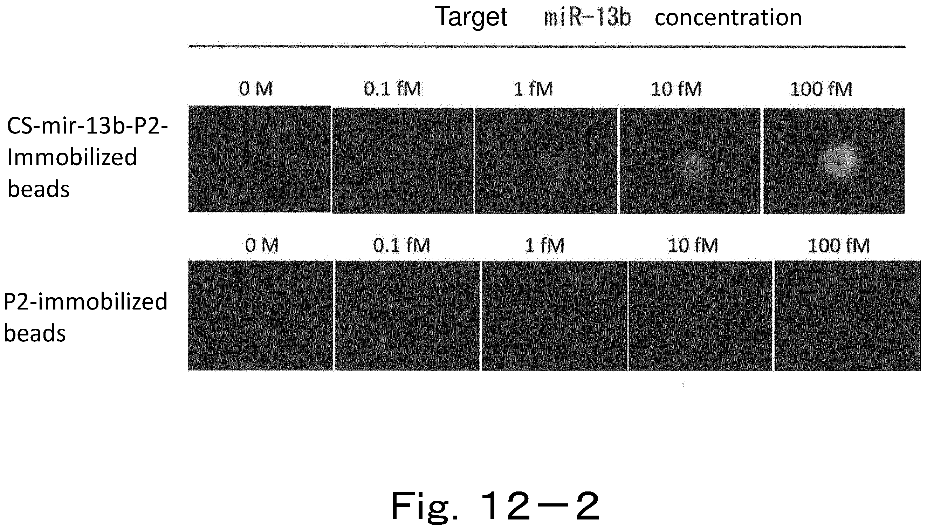

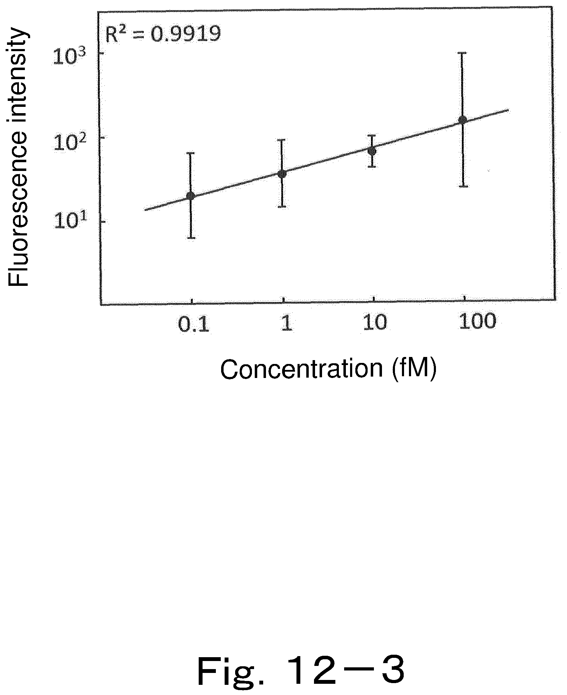

The results are shown in Fig. 12-2 and Fig. 12-3. By collecting the beads, quantitative measurement of the fluorescence intensity was made possible.

In the case where the CS-mir-13b-primer (P2)-immobilized FG beads were used, a calibration curve within the concentration range of 0.1 fM to 100 fM could be prepared for the target miR-13b, and quantitative analysis was possible therewith. On the other hand, in the case where the primer (P2)-immobilized FG beads were used, no fluorescence could be found for the target miR-13b after similarly performing the reaction at concentrations of 0.1 fM to 100 fM.

It is assumed that, by using the beads on which the capture oligonucleotide is immobilized, the first-stage reaction occurred in the vicinities of the beads, and therefor that the extension product of the first-stage reaction tended to be present in the vicinities of the beads (it is thought that, in the case where only P2 was immobilized, the first-stage reaction proceeded in the solution, and therefore that the extension product of the first-stage reaction was less likely to gather in the vicinities of the beads). It is thus thought that the second-stage reaction proceeded more smoothly even without immobilization of the extension product of the first-stage reaction. The detection sensitivity was (about 1000 times) higher than that in the case by the conventional method using no beads (that is, the solution system), whose sensitivity was 100 fM.

### [Example 7]

### (Preparation of Beads on Which Capture Oligonucleotide and Primer Are Immobilized)

### (a) Preparation of Biotinylated Capture Oligonucleotide and Biotinylated Primer

A biotinylated capture oligonucleotide (CS-thr) and a biotinylated primer (P2) were dissolved in 1×ϕ29 DNA polymerase reaction buffer, to prepare a CS-thr-P2 mixed solution of CS-thr (1 µM) and P2 (20 µM).

The DNA sequence of the capture oligonucleotide (CS-thr) is the sequence of SEQ ID NO:24.

The DNA sequence of the primer (P2) is the sequence of SEQ ID NO:2.

### (b) Providing and Washing of Magnetic Beads before Use

The same operation as in Example 1 was carried out.

### (c) Immobilization of Capture Oligonucleotide and Primer on Magnetic Beads, and Washing

The tube was placed in a magnetic rack (a tube stand with a magnet), to separate the magnetic beads from the supernatant (5 minutes). After removing the supernatant, the same operation as in Example 1 was carried out except that 4 µL of the CS-thr-P2 mixed solution was added to the beads.

### (Reaction Using Beads on Which Capture Oligonucleotide and Primer Are Immobilized)

The same operation as in Example 1 was carried out except that 2 µL of the CS-thr-primer (P2)-immobilized FG beads were scooped up, and that 2 µL (10 nM) of thrombin as a target, or lysozyme, lectin, or streptavidin as a non-target was used.

The DNA sequence of the first single-stranded circular DNA (cT1-thr) is the sequence of SEQ ID NO:25, which is circularized by binding both ends to each other.

The DNA sequence of the second single-stranded circular DNA (cT2) is the sequence of SEQ ID NO:4, which is circularized by binding both ends to each other.

The DNA sequence of the primer (P1-thr) is the sequence of SEQ ID NO: 19.

### (Observation under Fluorescence Microscope)

The same operation as in Example 4 was carried out.

**[Table 15]**

| Table 15. Final concentrations of the SATIC reagent and the primers in the reaction solution | |
|---|---|
| Component of the reaction solution | Concentration |
| cT1-thr | 100 nM |
| cT2 | 400 nM |
| CS-thr | 0.20 pmol/tube |
| P1-thr | 120 nM |
| P2 | 4.0 pmol/tube |
| *φ* 29 DNA polymerase reaction buffer | 1 × |
| BSA | 0.1 mg/ *µ*L |
| *φ* 29 DNA polymerase | 0.2 U/*µ*L |
| ThT-HE | 10 *µ*M |
| KCI | 10 mM |

### (Results)

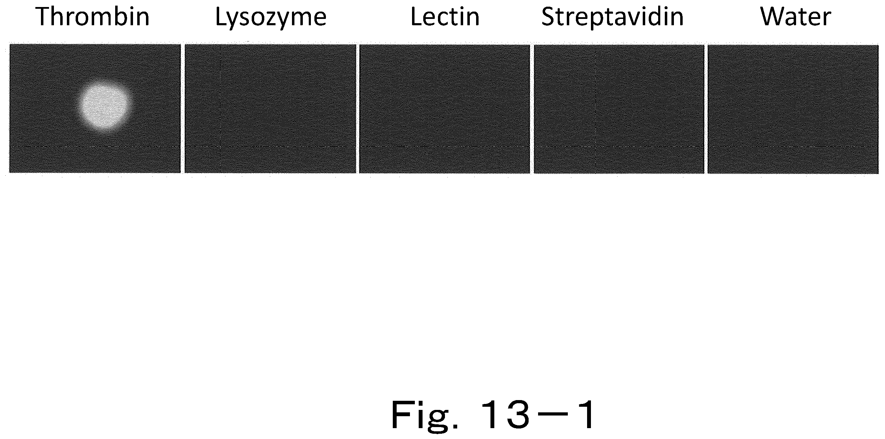

The results are shown in Fig. 13-1. In the case where thrombin as a target was used, fluorescence was found. However, in the cases where lysozyme, lectin, or streptavidin as a non-target was used, no fluorescence was found. In other words, the presence of thrombin as a target could be specifically detected.

### [Reference Example 5]

Beads on which a capture oligonucleotide and a primer are immobilized were prepared in the same manner as in Example 7. In addition, primer (P2)-immobilized beads were prepared in the same manner as in Reference Example 3.

### (Reaction Using Beads on Which Capture Oligonucleotide and Primer Are Immobilized, or Using Primer-Immobilized Beads)

The same operation as in Example 1 was carried out except that 2 µL of the CS-thr-primer (P2)-immobilized FG beads or 2 µL of the primer (P2)-immobilized FG beads were scooped up, and that 2 µL (10, 100, 1000, or 10,000 fM) of thrombin as a target was used.

### (Observation under Fluorescence Microscope)

The same operation as in Example 4 was carried out.

**[Table 16]**

| Table 16. Final concentrations of the SATIC reagent and the primers in the reaction solution | |
|---|---|
| Component of the reaction solution | Concentration |
| cT1-thr | 100 nM |
| cT2 | 400 nM |
| CS-thr | 0.20 pmol/tube |
| P1-thr | 120 nM |
| P2 | 4.0 pmol/tube |
| *φ* 29 DNA polymerase reaction buffer | 1 × |
| BSA | 0.1 mg/ *µ*L |
| *φ* 29 DNA polymerase | 0.2 U/*µ*L |
| ThT-HE | 10 *µ*M |
| KCI | 10 mM |

### (Results)

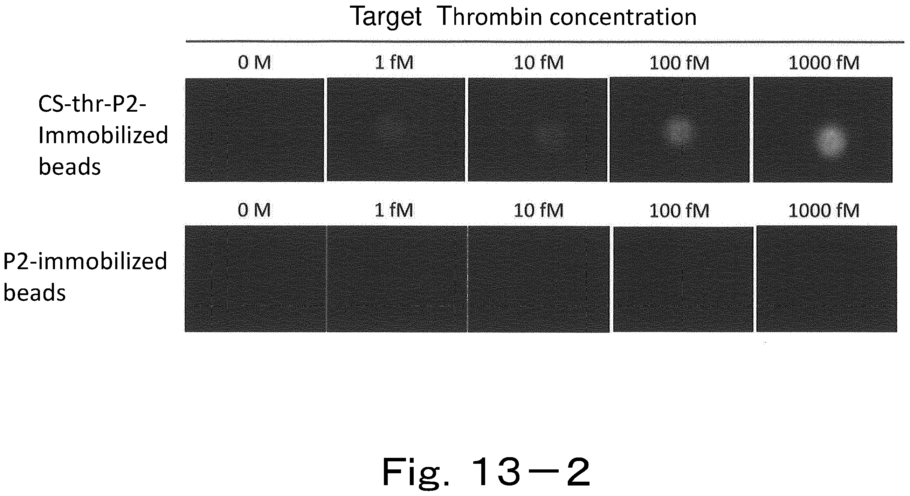

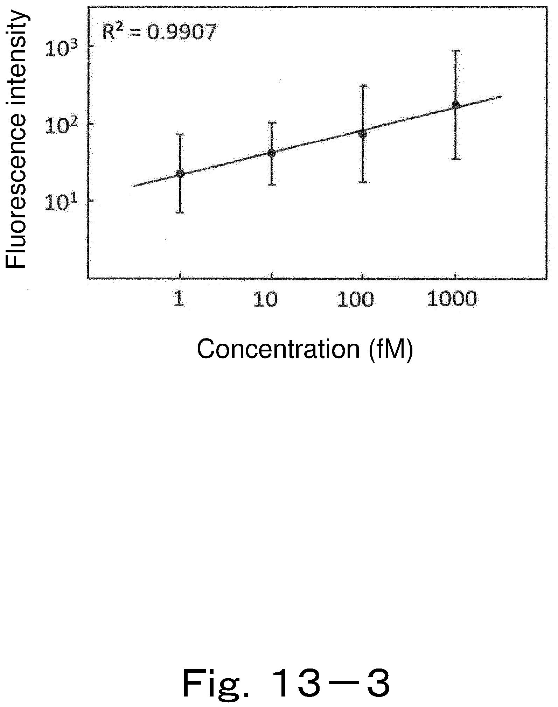

The results are shown in Fig. 13-2 and Fig. 13-3. By collecting the beads, quantitative measurement of the fluorescence intensity was made possible.

In the case where the CS-thr-primer (P2)-immobilized FG beads were used, a calibration curve within the concentration range of 1 fM to 1000 fM could be prepared for thrombin as the target, and quantitative analysis was possible therewith.

On the other hand, in the case where the primer (P2)-immobilized FG beads were used, no fluorescence could be found for the target thrombin after similarly performing the reaction at concentrations of 1 fM to 1000 fM.

It is assumed that, by using the beads on which the capture oligonucleotide is immobilized, the first-stage reaction occurred in the vicinities of the beads, and therefor that the P1 extension product tended to be present in the vicinities of the beads (it is thought that, in the case where only P2 was immobilized, the first-stage reaction proceeded in the solution, and therefore that the P1 extension product was less likely to gather in the vicinities of the beads). It is thus thought that the second-stage reaction proceeded more smoothly even without immobilization of P1. The detection sensitivity was (about 50,000 times) higher than that in the case by the conventional method using no beads (that is, the solution system), whose sensitivity was 50 pM.

### [Example 8]

### (Preparation of Beads on Which Capture Oligonucleotide and Primer Are Immobilized)

### (a) Preparation of Biotinylated Capture Oligonucleotide and Biotinylated Primer

A biotinylated capture oligonucleotide (CS-str) and a biotinylated primer (P2) were dissolved in 1×ϕ29 DNA polymerase reaction buffer, to prepare a CS-str-P2 mixed solution of CS-str (1 µM) and P2 (20 µM).

The DNA sequence of the capture oligonucleotide (CS-str) is the sequence of SEQ ID NO:26.

The DNA sequence of the primer (P2) is the sequence of SEQ ID NO:2.

### (b) Providing and Washing of Magnetic Beads before Use

The same operation as in Example 1 was carried out.

### (c) Immobilization of Capture Oligonucleotide and Primer on Magnetic Beads, and Washing

The tube was placed in a magnetic rack (a tube stand with a magnet), to separate the magnetic beads from the supernatant (5 minutes). After removing the supernatant, the same operation as in Example 1 was carried out except that 4 µL of the CS-str-P2 mixed solution was added to the beads.

### (Reaction Using Beads on Which Capture Oligonucleotide and Primer Are Immobilized)

The same operation as in Example 1 was carried out except that 2 µL of the CS-str-primer (P2)-immobilized FG beads were scooped up, and that 2 µL (10 µM) of streptomycin as a target, or ampicillin or kanamycin as a non-target was used.

The DNA sequence of the first single-stranded circular DNA (cT1-thr) is the sequence of SEQ ID NO:25, which is circularized by binding both ends to each other.

The DNA sequence of the second single-stranded circular DNA (cT2) is the sequence of SEQ ID NO:4, which is circularized by binding both ends to each other.

The DNA sequence of the primer (P1-str) is the sequence of SEQ ID NO:27.

### (Observation under Fluorescence Microscope)

The same operation as in Example 4 was carried out.

**[Table 17]**

| Table 17. Final concentrations of the SATIC reagent and the primers in the reaction solution | |
|---|---|
| Component of the reaction solution | Concentration |
| cT1-thr | 100 nM |
| cT2 | 400 nM |
| CS-str | 0.20 pmol/tube |
| P1-str | 120 nM |
| P2 | 4.0 pmol/tube |
| *φ* 29 DNA polymerase reaction buffer | 1 × |
| BSA | 0. 1 mg/ *µ*L |
| *φ* 29 DNA polymerase | 0.2 U/*µ*L |
| ThT-HE | 10 *µ*M |
| KCI | 10 mM |

### (Results)

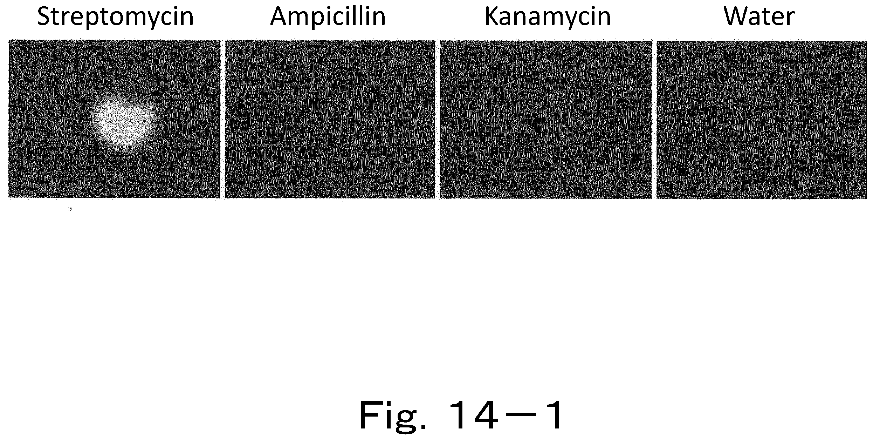

The results are shown in Fig. 14-1. In the case where streptomycin as the target was used, fluorescence was found. However, in the cases where ampicillin or kanamycin as the non-target was used, no fluorescence was found. In other words, the presence of streptomycin as the target could be specifically detected.

### [Reference Example 6]

Beads on which a capture oligonucleotide and a primer are immobilized were prepared in the same manner as in Example 8. In addition, primer (P2)-immobilized beads were prepared in the same manner as in Reference Example 3.

### (Reaction Using Beads on Which Capture Oligonucleotide and Primer Are Immobilized, or Using Primer-Immobilized Beads)

The same operation as in Example 1 was carried out except that 2 µL of the CS-str-primer (P2)-immobilized FG beads or 2 µL of the primer (P2)-immobilized FG beads were scooped up, and that 2 µL (0.1, 1, 10, or 100 nM) of streptomycin as a target was used.

### (Observation under Fluorescence Microscope)

The same operation as in Example 4 was carried out.

**[Table 18]**

| Table 18. Final concentrations of the SATIC reagent and the primers in the reaction solution | |
|---|---|
| Component of the reaction solution | Concentration |
| cT1-str | 100 nM |
| cT2 | 400 nM |
| CS-str | 0.20 pmol/tube |
| P1-str | 120 nM |
| P2 | 4.0 pmol/tube |
| *φ* 29 DNA polymerase reaction buffer | 1 × |
| BSA | 0.1 mg/*µ*L |
| *φ* 29 DNA polymerase | 0.2 U/*µ*L |
| ThT-HE | 10 *µ*M |
| KCI | 10 mM |

### (Results)

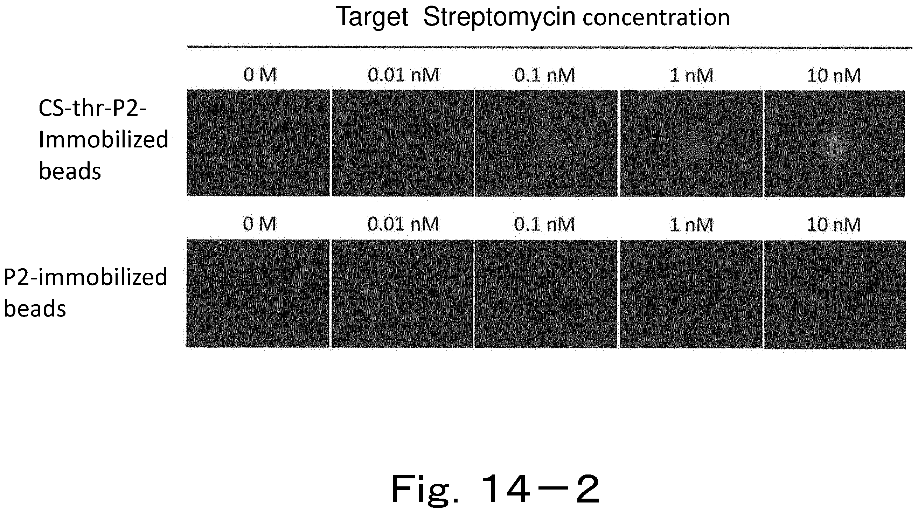

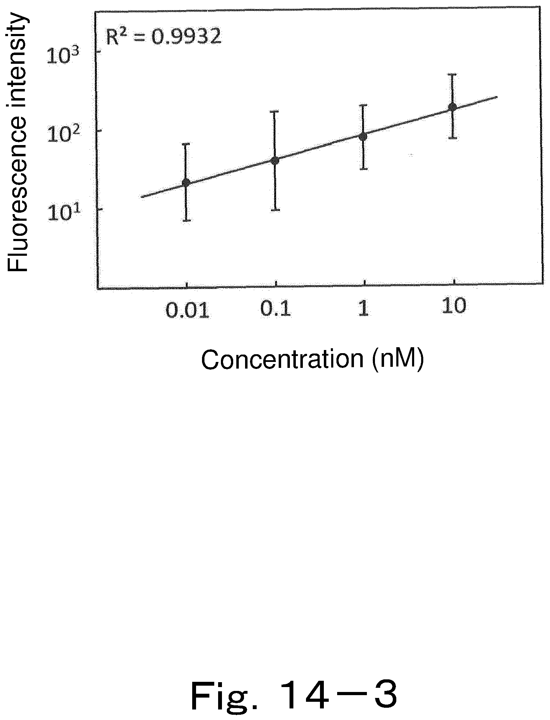

The results are shown in Fig. 14-2 and Fig. 14-3. By collecting the beads, quantitative measurement of the fluorescence intensity was made possible.

In the case where the CS-str-primer (P2)-immobilized FG beads were used, a calibration curve within the concentration range of 0.01 nM to 10 nM could be prepared for streptomycin as the target, and quantitative analysis was possible therewith.

On the other hand, in the case where the primer (P2)-immobilized FG beads were used, no fluorescence could be found for the target streptomycin after similarly performing the reaction at concentrations of 0.01 nM to 10 nM. It is assumed that, by using the beads on which the capture oligonucleotide is immobilized, the first-stage reaction occurred in the vicinities of the beads, and therefor that the P1 extension product tended to be present in the vicinities of the beads (it is thought that, in the case where only P2 was immobilized, the first-stage reaction proceeded in the solution, and therefore that the P1 extension product was less likely to gather in the vicinities of the beads). It is thus thought that the second-stage reaction proceeded more smoothly even without immobilization of P1. The detection sensitivity was (about 7500 times) higher than that in the case by the conventional method using no beads (that is, the solution system), whose sensitivity was 75 nM.

### [Example 9]

### (Preparation of Fluorescent Dye)

Malachite Green was dissolved in distilled water, to prepare a 200-µM solution.

### (Reaction in Solution System)

Eighteen microliters of SATIC reagent were added to the solution, and then 2 µL (10 nM) of a target RNA CidR_40 (40-mer) or a non-target RNA ArfR_39 (39-mer) was further added thereto, followed by allowing the reaction to proceed at 37°C for 2 hours.

As a control, water was used. As a reference, a double-stranded DNA (40 bp, 1 µM; which was, however, not subjected to SATIC reaction) was used.

The DNA sequence of the primer (P1) is the sequence of SEQ ID NO: 1.

The DNA sequence of the primer (P2) is the sequence of SEQ ID NO:2.

The DNA sequence of the first single-stranded circular DNA (cT1) is the sequence of SEQ ID NO:3, which is circularized by binding both ends to each other.

The DNA sequence of the second single-stranded circular DNA (cT2) is the sequence of SEQ ID NO:4, which is circularized by binding both ends to each other.

The RNA sequence of the target RNA CidR_40 (40-mer) is the sequence of SEQ ID NO:5.

The RNA sequence of the non-target RNA ArfR_ 39 (39-mer) is the sequence of SEQ ID NO:6.

### (Measurement Using Fluorescence Spectrophotometer)

In a cell for fluorescence measurement, 70 µL of the reaction solution was placed, and measurement was carried out using a fluorescence spectrophotometer. The measurement was carried out under the following conditions: excitation wavelength, 590 nm; measurement wavelength, 630 nm to 800 nm.

**[Table 19]**

| Table 19. Final concentrations of the SATIC reagent and the primers in the reaction solution | |
|---|---|
| Component of the reaction solution | Concentration |
| cT1 | 100 nM |
| cT2 | 400 nM |
| P1 | 120 nM |
| P2 | 480 nM |
| *φ* 29 DNA polymerase reaction buffer | 1 × |
| BSA | 0.1 mg/ *µ*L |
| dNTPs | 1 mM |
| *φ* 29 DNA polymerase | 0.1 U/*µ*L |
| Malachite green | 20 *µ*M |

### (Results)

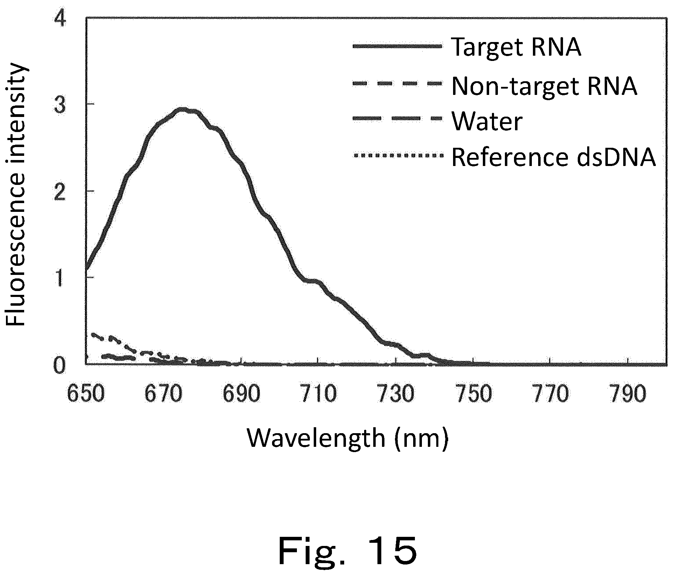

The results are shown in Fig. 15. In the case of the target RNA CidR_40 (40-mer), the fluorescence intensity increased. Since the excitation wavelength is near 600 nm, at which light absorption due to biological components is low, direct detection from biological components may be possible.

It could be confirmed that a guanine-quadruplex-binding reagent other than ThT-HE can be used for the present detection system.

### [Example 10]

### Effects of ThT Derivatives as Aggregation Promoters

### 1) Preparation of Primer-Immobilized Gold Colloid

### a) Preparation of Biotinylated Primers

A biotinylated primer (Pi) and a biotinylated primer (P₂) were dissolved in 1×ϕ29 DNA polymerase reaction buffer, to prepare a P₁-P₂ mixed solution of P₁ (1 mM) and P₂ (20 µM).

### b) Providing and Washing of Gold Colloid before Use

A gold colloid (30 nm) was vortexed well, and 20 µL of the gold colloid was scooped up and placed in a 1.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice.

### c) Immobilization of Primers to Gold Colloid, and Washing

By centrifugation in a centrifuge, the gold colloid was separated from the supernatant. The supernatant was removed.

To the washed gold colloid, 4 µL of the P₁-P₂ mixed solution was added.

Incubation was carried out at 25°C for 30 minutes. During the incubation, vortexing was carried out at 5-minute intervals.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice. The prepared gold colloid was stored under refrigeration until use.

### 2) Reaction Using Primer-Immobilized Gold Colloid and Coagulant

Two microliters of the P₁-P₂ gold colloid were scooped up and placed in a 0.5-mL tube manufactured by Eppendorf. Fourteen microliters of SATIC reagent were added to the colloid, and 2 µL of a target (40-mer CIDEC; 10 pM) were also added thereto.

As a coagulant, 2 µL of ThT-HE, ThT-PEG (R⁵=NH₂, n=11), ThT-spermine, or ThT was added.

The reaction was allowed to proceed at 37°C for 2 hours.

The above-described primers P₁ and P₂, and cT1 and cT2 were used.

CidR_40 was used as a target RNA, and ArfR_39 was used as an off-target RNA.

**[Table 20]**

| Table 20. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-HE, ThT-PEG, ThT-spermine, ThT | 10 µM |

### 3) Visual Observation

Changes in the gold colloid were visually observed.

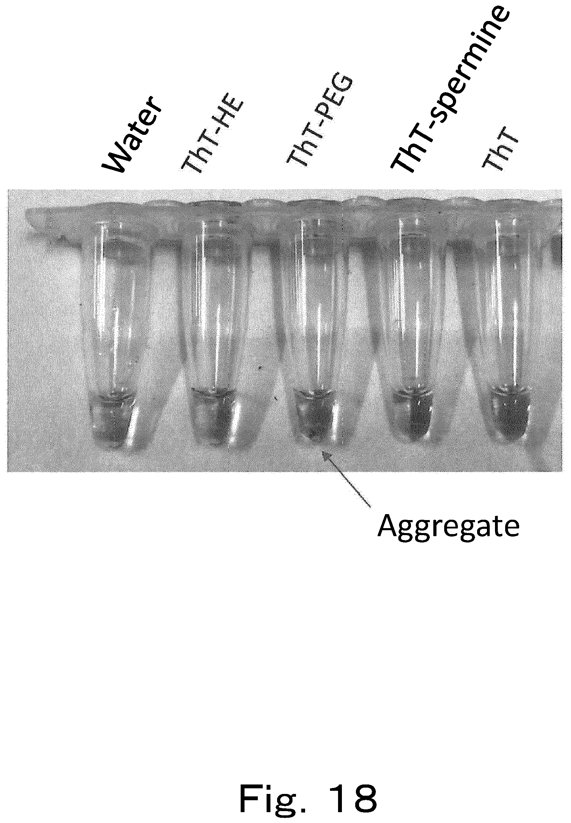

The results are shown in Fig. 18.

As a result of the addition of the ThT derivatives, an aggregate was found only in the case where ThT-PEG (Compound 2) was added.

### [Example 11]

### Study on Particle Size of Gold Colloid

### 1) Preparation of Primer-Immobilized Gold Colloid

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Gold Colloid before Use

A gold colloid (10, 15, or 30 nm) was vortexed well, and 10 µL of the gold colloid was scooped up and placed in a 0.5-mL tube manufactured by Eppendorf.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice.

### c) Immobilization of Primers to Gold Colloid, and Washing

By centrifugation in a centrifuge, the gold colloid was separated from the supernatant. The supernatant was removed.

To the washed gold colloid, 2 µL of the P₁-P₂ mixed solution was added.

Incubation was carried out at 25°C for 30 minutes. During the incubation, vortexing was carried out at 5-minute intervals.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice.

### 2) Visual Observation

Changes in the gold colloid were visually observed.

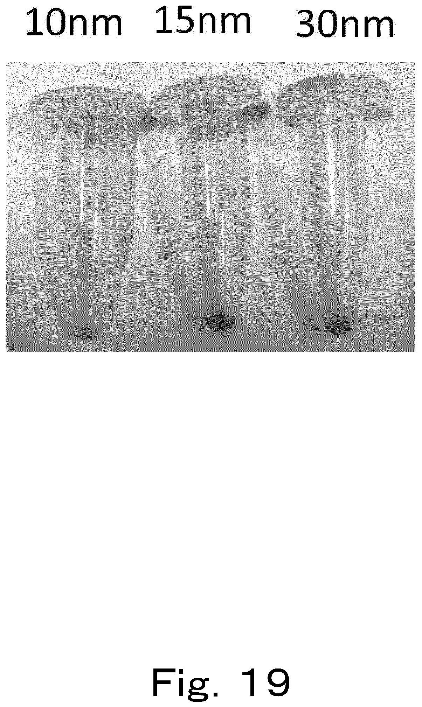

The results are shown in Fig. 19.

In the case where the particle size was not more than 10 nm, even the binding of the primers to the gold colloid caused aggregation.

### [Example 12]

### Study on Particle Size of Gold Colloid (Continued)

### 1) Preparation of Primer-Immobilized Gold Colloid

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Gold Colloid before Use

A gold colloid (15, 30, 40, or 60 nm) was vortexed well, and 10 µL of the gold colloid was scooped up and placed in a 0.5-mL tube manufactured by Eppendorf.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice.

### c) Immobilization of Primers to Gold Colloid, and Washing

By centrifugation in a centrifuge, the gold colloid was separated from the supernatant. The supernatant was removed.

To the washed gold colloid, 2 µL of the P₁-P₂ mixed solution was added.

Incubation was carried out at 25°C for 30 minutes. During the incubation, vortexing was carried out at 5-minute intervals.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice. The prepared gold colloid was stored under refrigeration until use.

### 2) Reaction Using Primer-Immobilized Gold Colloid and Coagulant

Two microliters of the P₁-P₂ gold colloid were scooped up and placed in a 0.5-mL tube manufactured by Eppendorf. Sixteen microliters of SATIC reagent were added to the colloid, and 2 µL of a target (40-mer CIDEC; 10 pM) were also added thereto.

The reaction was allowed to proceed at 37°C for 2 hours.

**[Table 21]**

| Table 21. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG | 10 µM |

### 3) Visual Observation

Changes in the gold colloid were visually observed.

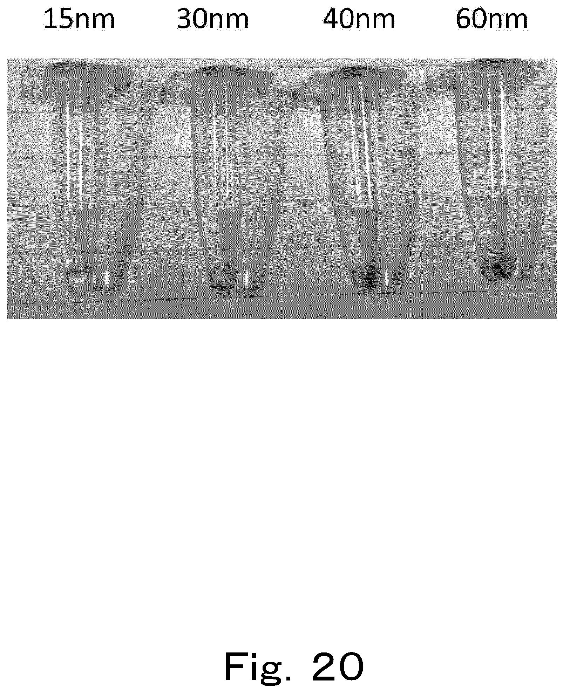

### The results are shown in Fig. 20.

As a result of the binding of P₁ and P₂ to the gold colloids having the various particle sizes, and carrying out the SATIC reaction, aggregation was found for the cases where the particle size was not less than 30 nm.

### [Example 13]

### Aggregation Effect by Addition of Polyethylene Glycol (PEG)

### 1) Preparation of Primer-Immobilized Gold Colloid

### a) Preparation of Biotinylated Primers

A biotinylated primer (Pi) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Gold Colloid before Use

A gold colloid (30 nm) was vortexed well, and 20 µL of the gold colloid was scooped up and placed in a 0.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice.

### c) Immobilization of Primers to Gold Colloid, and Washing

By centrifugation in a centrifuge, the gold colloid was separated from the supernatant. The supernatant was removed.

To the washed gold colloid, 4 µL of the P₁-P₂ mixed solution was added.

Incubation was carried out at 25°C for 30 minutes. During the incubation, vortexing was carried out at 5-minute intervals.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice. The prepared gold colloid was stored under refrigeration until use.

### 2) Reaction Using Primer-Immobilized Gold Colloid and Coagulant

Two microliters of the P₁-P₂ gold colloid were scooped up and placed in a 0.5-mL tube manufactured by Eppendorf. Twelve microliters of SATIC reagent were added to the colloid, and 2 µL of a target (40-mer CIDEC; 10 pM) were also added thereto.

Four microliters of a solution of PEG200, 6000, or 8000 were added thereto.

The reaction was allowed to proceed at 37°C for 2 hours.

**[Table 22]**

| Table 22. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Various PEG solutions | 0.1, 1, 10 w/v(%) |

### 3) Visual Observation

Changes in the gold colloid were visually observed.

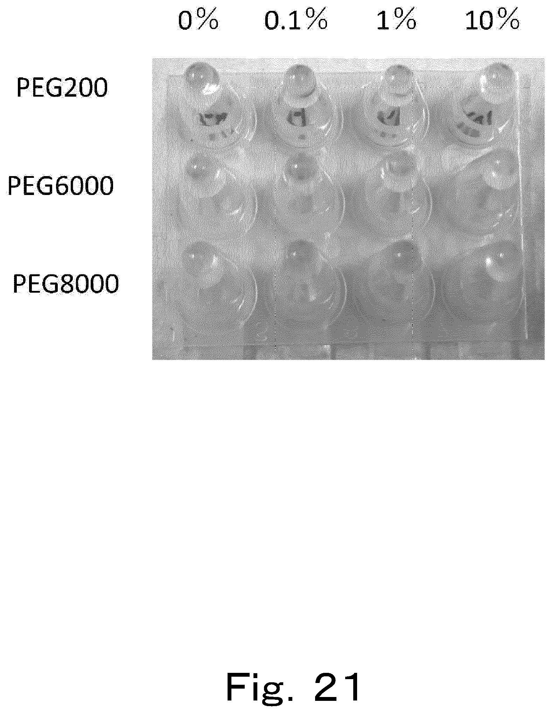

The results are shown in Fig. 21.

It could be confirmed that no aggregation can be seen even by adding each kind of PEG and performing the SATIC reaction. It could thus be confirmed that the addition of PEG does not have an aggregation effect.

### [Example 14]

### Study on Detection Sensitivity

### 1) Preparation of Primer-Immobilized Gold Colloid

### a) Preparation of Biotinylated Primers

A biotinylated primer (Pi) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Magnetic Beads before Use

A gold colloid (30 nm) was vortexed well, and 40 µL of the gold colloid was scooped up and placed in a 0.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice.

### c) Immobilization of Primers to Gold Colloid, and Washing

By centrifugation in a centrifuge, the gold colloid was separated from the supernatant. The supernatant was removed.

To the washed gold colloid, 4 µL of the P₁-P₂ mixed solution was added.

Incubation was carried out at 25°C for 30 minutes. During the incubation, vortexing was carried out at 5-minute intervals.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice. The prepared gold colloid was stored under refrigeration until use.

### 2) Reaction Using Primer-Immobilized Gold Colloid and Coagulant

Two microliters of the P₁-P₂ gold colloid were scooped up and placed in a 0.5-mL tube manufactured by Eppendorf.

Sixteen microliters of SATIC reagent were added to the colloid, and 2 µL of a target (40-mer CIDEC; 10 aM to 10 pM) was also added thereto.

The reaction was allowed to proceed at 37°C for 2 hours.

**[Table 23]**

| Table 23. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG | 10 µM |

### 3) Visual Observation

Changes in the gold colloid were visually observed.

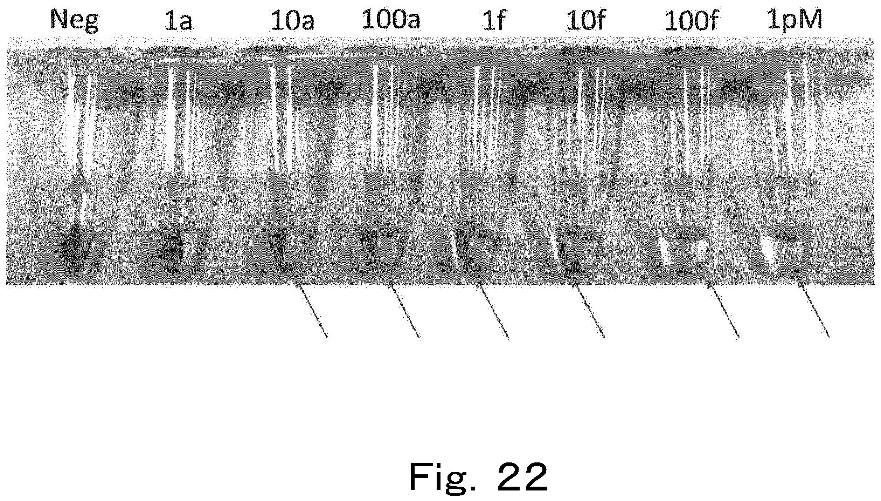

The results are shown in Fig. 22.

At a concentration of 10 aM (120 copies/tube), the 40-mer target RNA could be visually detected.

### [Example 15]

### Effects of ThT Derivatives as Aggregation Promoters (Nanoparticles: FG Beads; Particle Size: 180 nm)

### 1) Preparation of Primer-Immobilized Nanoparticles

### a) Preparation of Biotinylated Primers

A biotinylated primer (Pi) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 nL of water was added to the beads, followed by pipetting.

The above operation was further carried out twice. The prepared beads were stored under refrigeration until use.

### 2) Reaction Using Primer-Immobilized Nanoparticles

Two microliters of the P1-P2-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Sixteen microliters of SATIC reagent were added to the beads, and 2 µL of a target RNA (40-mer CIDEC; 10 pM) were also added thereto. Alternatively, 2 µL of an off-target RNA (39-mer Arf; 10 pM) were added thereto.

Two microliters of ThT-HE, ThT-PEG, ThT-spermine, or ThT were added thereto.

The reaction was allowed to proceed at 37°C for 2 hours.

**[Table 24]**

| Table 24. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-HE, ThT-PEG, ThT-spermine, ThT | 10 µM |

### 3) Visual Observation

Changes in the nanoparticles were visually observed.

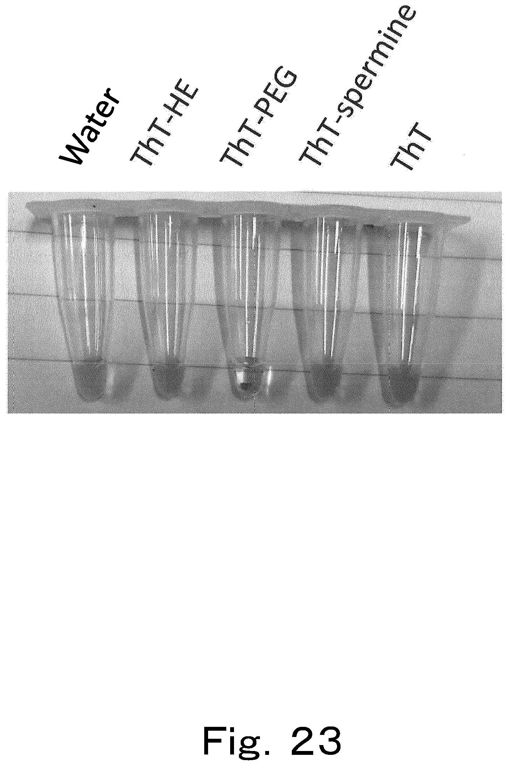

The results are shown in Fig. 23.

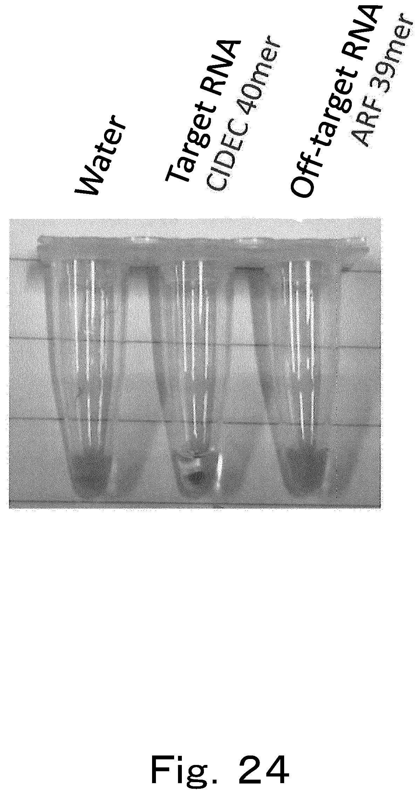

As a result of the addition of the ThT derivatives, an aggregate was found only in the case where ThT-PEG (Compound 2) was added. Further, as shown in Fig. 24, this aggregation was found to occur specifically to the target.

### [Example 16]

### Test of Detection Specificity and Detection Sensitivity

### 1) Preparation of Primer-Immobilized Nanoparticles

### a) Preparation of Biotinylated Primers

A biotinylated primer (Pi) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 pM) was also added thereto. Alternatively, 2 µL of an off-target RNA (39-mer Arf; 10 pM) was added thereto.

The reaction was allowed to proceed at 37°C for 2 hours.

**[Table 25]**

| Table 25. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG | 10 µM |

### 3) Visual Observation

Changes in the nanoparticles were visually observed.

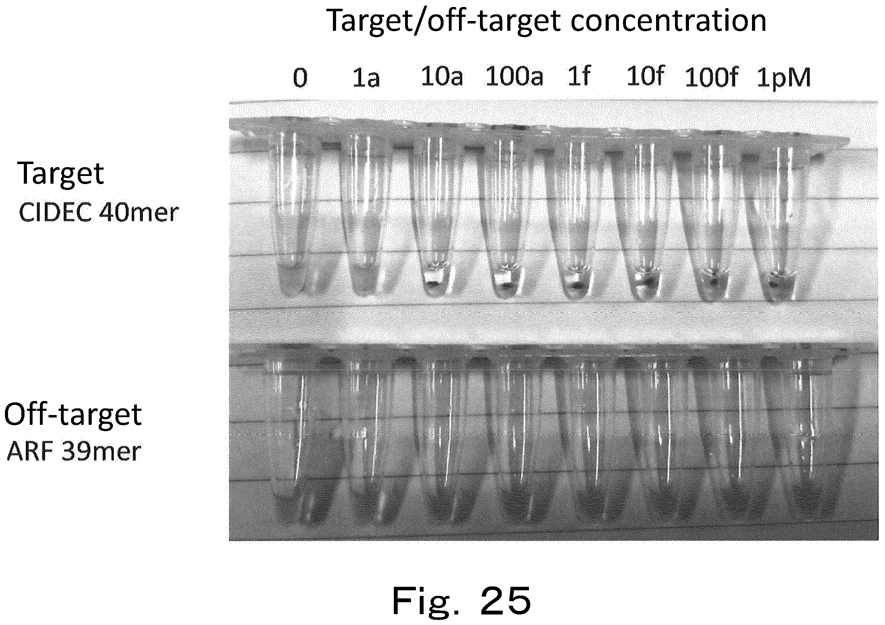

### The results are shown in Fig. 25.

At a concentration of 10 aM (120 copies/tube), the 40-mer target could be visually detected.

### [Example 17]

### Detection System Using Modified Gold Colloid as Coagulant

### 1) Preparation of Primer-Immobilized Nanoparticles

### a) Preparation of Biotinylated Primers

A biotinylated primer (Pi) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Preparation of Modified Gold Colloid

### a) Providing and Washing of Gold Colloid before Use

A gold colloid (30 nm) was vortexed well, and 20 µL of the gold colloid was scooped up and placed in a 0.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the beads, followed by pipetting.

The above operation was further carried out twice.

### b) Immobilization of ThT-Biotin (Compound 3) and PEG-Biotin (Compound 4) on Gold Colloid, and Washing

By centrifugation in a centrifuge, the gold colloid was separated from the supernatant. The supernatant was removed.

To the washed gold colloid, 2 µL of a mixed solution of ThT-biotin (100 µM) and PEG-biotin (100 µM) was added.

Incubation was carried out at 25°C for 30 minutes. During the incubation, vortexing was carried out at 5-minute intervals.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice. The prepared gold colloid was stored under refrigeration until use.

### 2-2-3) Reaction Using Primer-Immobilized Nanoparticles and Coagulant (Modified Gold Colloid)

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Sixteen microliters of SATIC reagent were added to the beads, and 2 µL of a target RNA (40-mer CIDEC; 10 pM) were also added thereto. Alternatively, 2 µL of an off-target RNA (39-mer Arf; 10 pM) were added thereto.

As a coagulant, 2 µL of the modified gold colloid was added.

The reaction was allowed to proceed at 37°C for 2 hours.

**[Table 26]**

| Table 26. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Compound 3 | 1µM |
| Compound 4 | 1µM |

### 4) Visual Observation

Changes in the nanoparticles were visually observed.

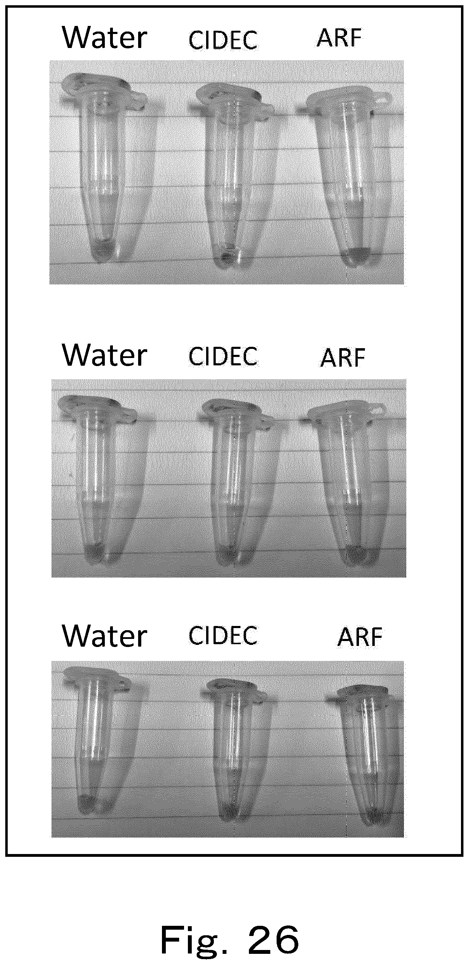

The results are shown in Fig. 26.

The SATIC reaction using, as a coagulant, the gold colloid on which Compounds 3 and 4 are immobilized together caused specific aggregation only in the presence of the target (CIDEC 40-mer).

### [Example 18]

### Optimization of Immobilization rates of Compounds 3 and 4 on Gold Colloid

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Preparation of Modified Gold Colloid

### a) Providing and Washing of Gold Colloid before Use

A gold colloid (30 nm) was vortexed well, and 20 µL of the gold colloid was scooped up and placed in a 0.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice.

### b) Immobilization of ThT-Biotin (Compound 3) and PEG-Biotin (Compound 4) on Gold Colloid, and Washing

By centrifugation in a centrifuge, the gold colloid was separated from the supernatant. The supernatant was removed.

To the washed gold colloid, 2 µL of a mixed solution of ThT-biotin (Compound 3, 100 µM) and PEG-biotin (Compound 4, 100 µM) was added. The preparation of the mixed solution was carried out such that Compound 3 was contained at 0, 10, 30, 50, 70, 90, or 100%.

Incubation was carried out at 25°C for 30 minutes. During the incubation, vortexing was carried out at 5-minute intervals.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice. The prepared colloid was stored under refrigeration until use.

### 3) Reaction Using Primer-Immobilized Nanoparticles and Coagulant (Modified Gold Colloid)

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Sixteen microliters of SATIC reagent were added to the beads, and 2 µL of a target RNA (40-mer CIDEC; 10 pM) were also added thereto. Alternatively, 2 µL of an off-target RNA (39-mer Arf; 10 pM) were added thereto.

As a coagulant, 2 µL of the modified gold colloid was added.

The reaction was allowed to proceed at 37°C for 2 hours.

**[Table 27]**

| Table 27. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Compound 3 | 1µM |
| Compound 4 | 1µM |

### 4) Visual Observation

Changes in the nanoparticles were visually observed.

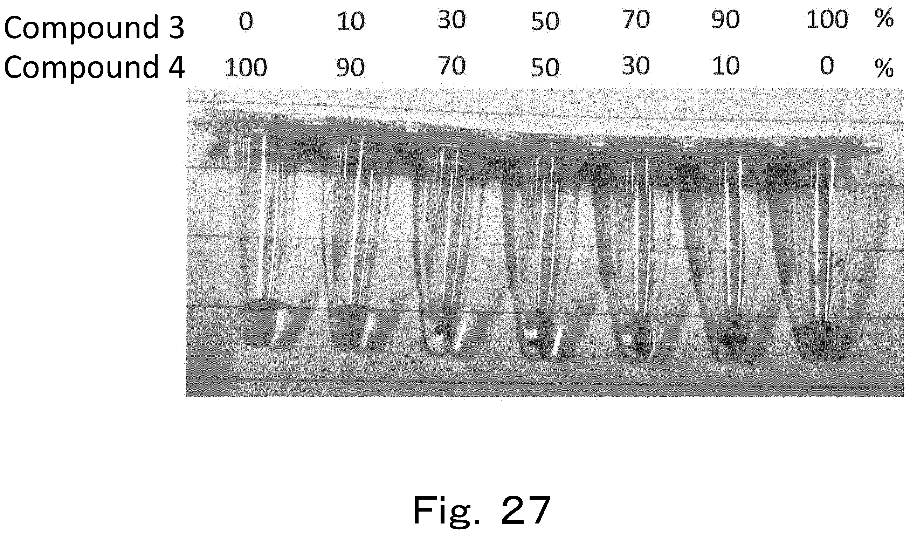

The results are shown in Fig. 27.

It was found that aggregation occurs when a gold colloid in which the immobilization ratio between Compound 3 and Compound 4 is 30:70 to 90:10 is used for the SATIC reaction.

### [Example 19]

### Test of Detection Specificity and Detection Sensitivity

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Preparation of Modified Gold Colloid

### a) Providing and Washing of Gold Colloid before Use

A gold colloid (30 nm) was vortexed well, and 20 µL of the gold colloid was scooped up and placed in a 0.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice.

### b) Immobilization of ThT-Biotin (Compound 3) and PEG-Biotin (Compound 4) on Gold Colloid, and Washing

By centrifugation in a centrifuge, the gold colloid was separated from the supernatant. The supernatant was removed.

To the washed gold colloid, 2 µL of a mixed solution of ThT-biotin (Compound 3, 100 µM) and PEG-biotin (Compound 4, 100 µM) was added. The preparation was carried out such that the ratio of Compound 3 was 50%.

Incubation was carried out at 25°C for 30 minutes. During the incubation, vortexing was carried out at 5-minute intervals.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice. The prepared beads were stored under refrigeration until use.

### 3) Reaction Using Primer-Immobilized Nanoparticles and Coagulant (Modified Gold Colloid)

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM to 10 pM) were also added thereto. Alternatively, 2 µL of an off-target RNA (39-mer Arf; 10 a to 10 pM) were added thereto.

The reaction was allowed to proceed at 37°C for 2 hours.

**[Table 28]**

| Table 28. Final concentrations of the SATIC reagent and the primers in the reaction solution | |
|---|---|
| Component of the reaction solution | Concentration |
| cT₁ | 100nM |
| cT₂ | 400nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Compound 3 | 1µM |
| Compound 4 | 1µM |

### 4) Visual Observation

Changes in the nanoparticles were visually observed.

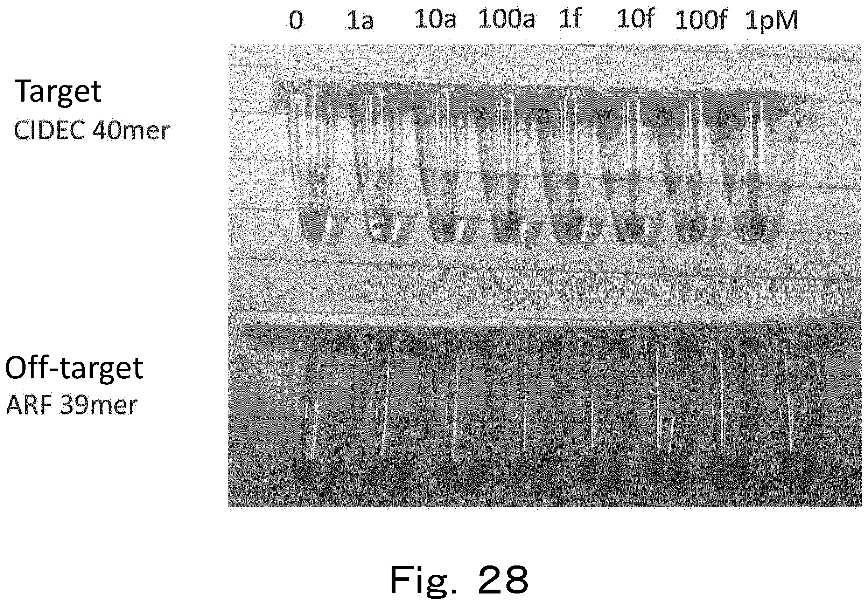

The results are shown in Fig. 28.

At a concentration of 1 aM (12 copies/tube), the 40-mer target (CIDEC) could be visually detected.

### [Example 20]

### Test of Detection Specificity and Detection Sensitivity (Continued)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Preparation of Modified Gold Colloid

### a) Providing and Washing of Gold Colloid before Use

A gold colloid (30 nm) was vortexed well, and 20 µL of the gold colloid was scooped up and placed in a 0.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice. Immobilization and Washing of: 10 µM)·PEG-Biotin (Compound 4)

By centrifugation in a centrifuge, the gold colloid was separated from the supernatant. The supernatant was removed.

To the washed gold colloid, 2 µL of a mixed solution of ThT-biotin (Compound 3, 100 µM) and PEG-biotin (Compound 4, 100 µM) was added. The preparation was carried out such that the ratio of Compound 3 was 50%.

Incubation was carried out at 25°C for 30 minutes. During the incubation, vortexing was carried out at 5-minute intervals.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice. The prepared colloid was stored under refrigeration until use.

### 3) Reaction Using Primer-Immobilized Nanoparticles and Coagulant (Modified Gold Colloid)

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (full-length CIDEC; 10 aM to 10 pM) were also added thereto. Alternatively, 2 µL of an off-target RNA (full-length Arf; 10 a to 10 pM) were added thereto.

The reaction was allowed to proceed at 37°C for 2 hours.

**[Table 29]**

| Table 29. Final concentrations of the SATIC reagent and the primers in the reaction solution | |
|---|---|
| Component of the reaction solution | Concentration |
| cT₁ | 100nM |
| cT₂ | 400nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Compound 3 | 1µM |
| Compound 4 | 1µM |

### 4) Visual Observation

Changes in the nanoparticles were visually observed.

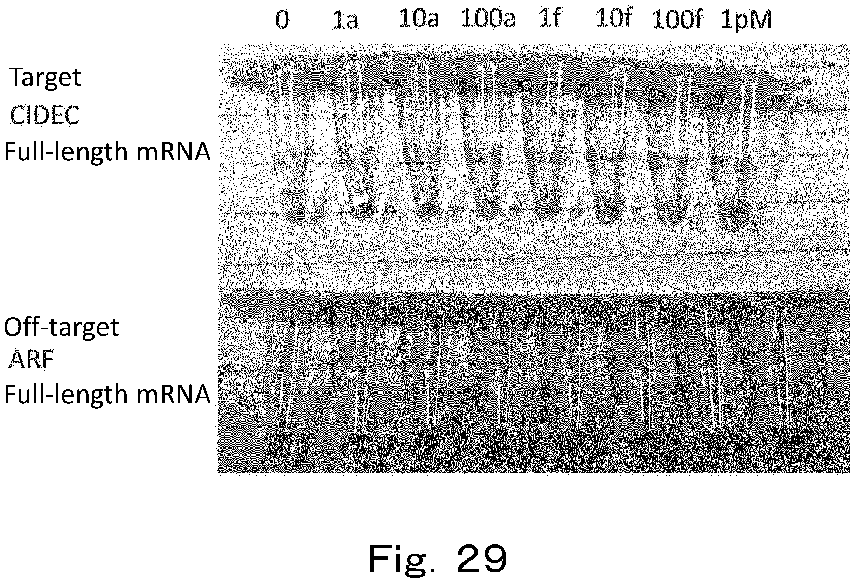

The results are shown in Fig. 29.

At a concentration of 1 aM (12 copies/tube), the full-length target (CIDEC) could be visually detected.

### [Example 21]

### Optimization of Rates of Modification of Streptavidin with Compounds 3 and 4

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Preparation of Streptavidin to Which Compounds 3 and 4 Are Bound

### a) Preparation of Streptavidin

A solution of streptavidin (400 µM) in 1×ϕ29 DNA polymerase reaction buffer was prepared.

### b) Immobilization of ThT-Biotin (Compound 3) and PEG-Biotin (Compound 4) on Streptavidin

To 50 µL of the streptavidin solution, 50 µL of a mixed solution of ThT-biotin (Compound 3, 0 to 400 µM) and PEG-biotin (Compound 4, 0 to 400) was added. The preparation of the mixed solution was carried out such that Compound 3 was contained at 0, 25, 50, 75, or 100%.

Incubation was carried out at 25°C for 30 minutes.

### 3) Reaction Using Primer-Immobilized Nanoparticles and Coagulant (Modified Gold Colloid)

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Fourteen microliters of SATIC reagent were added to the beads, and 2 µL of a target RNA (40-mer CIDEC; 10 pM) were also added thereto. Alternatively, 2 µL of an off-target RNA (40-mer Arf; 10 pM) were added thereto.

Four microliters of the modified streptavidin were added thereto.

The reaction was allowed to proceed at 37°C for 2 hours.

**[Table 30]**

| Table 30. Final concentrations of the SATIC reagent and the primers in the reaction solution | |
|---|---|
| Component of the reaction solution | Concentration |
| cT₁ | 100nM |
| cT₂ | 400nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Modified streptavidin | 40 µM |

### 4) Visual Observation

Changes in the nanoparticles were visually observed.

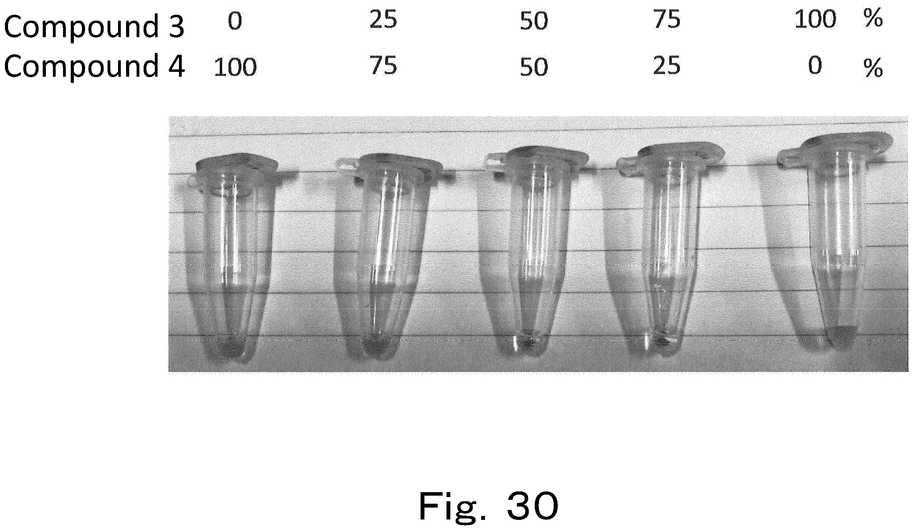

The results are shown in Fig. 30.

It was found that aggregation occurs when a streptavidin complex in which the modification rate between Compound 3 and Compound 4 is 50:50 to 25:75 is used for the SATIC reaction.

### [Example 22]

### Test of Detection Specificity and Detection Sensitivity (Coagulant: Modified Streptavidin)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Preparation of Streptavidin to Which Compounds 3 and 4 Are Bound

### a) Preparation of Streptavidin

A solution of streptavidin (400 µM) in 1×ϕ29 DNA polymerase reaction buffer was prepared.

### b) Immobilization of ThT-Biotin (Compound 3) and PEG-Biotin (Compound 4) on Streptavidin

To 50 µL of the streptavidin solution, 50 µL of a mixed solution of ThT-biotin (Compound 3, 200 µM) and PEG-biotin (Compound 4, 200 µM) was added.

Incubation was carried out at 25°C for 30 minutes.

### 3-3) Reaction Using Primer-Immobilized Nanoparticles and Coagulant (Modified Streptavidin)

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM to 10 pM) were also added thereto. Alternatively, 2 µL of an off-target RNA (40-mer Arf; 10 aM to 10 pM) were added thereto.

The reaction was allowed to proceed at 37°C for 2 hours.

**[Table 31]**

| Table 31. Final concentrations of the SATIC reagent and the primers in the reaction solution | |
|---|---|
| Component of the reaction solution | Concentration |
| cT₁ | 100nM |
| cT₂ | 400nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Modified streptavidin | 40 µM |

### 4) Visual Observation

Changes in the nanoparticles were visually observed.

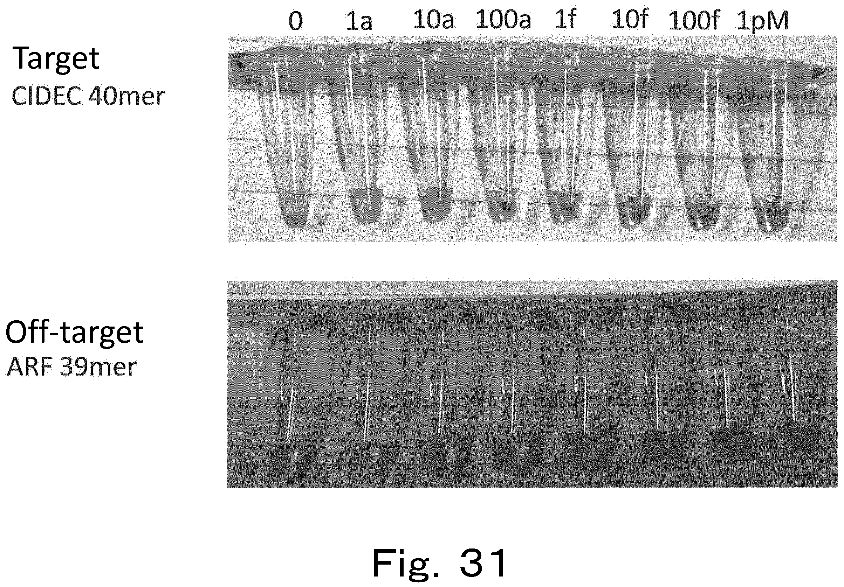

The results are shown in Fig. 31.

At a concentration of 100 aM (1200 copies/tube), the 40-mer target could be visually detected.

### [Example 23]

### Effect of Compound 5 (ThT-PEG-ThT, n=11) as Aggregation Promoter

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P1-P2-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 pM) were also added thereto. Alternatively, 2 µL of an off-target RNA (39-mer Arf; 10 pM) were added thereto.

The reaction was allowed to proceed at 37°C for 2 hours.

**[Table 32]**

| Table 32. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |

### 3) Visual Observation

Changes in the nanoparticles were visually observed.

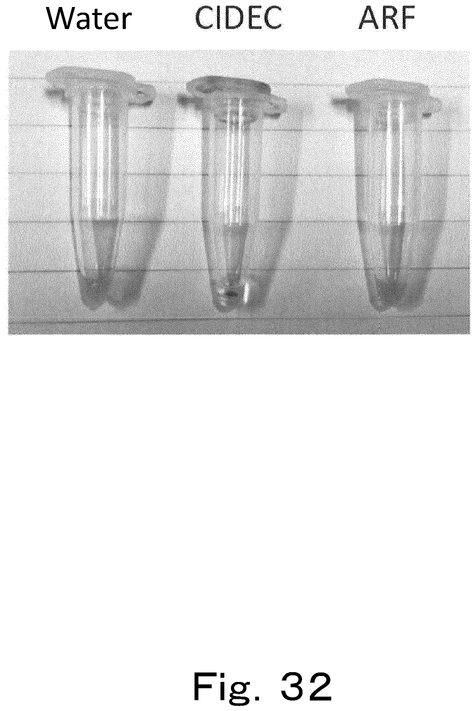

The results are shown in Fig. 32.

In the case where ThT-PEG-ThT was used as a coagulant, visual detection was possible specifically for the target.

### [Example 24]

### Test of Detection Specificity and Detection Sensitivity (ThT-PEG-ThT Compound 5)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM to 10 pM) were also added thereto. Alternatively, 2 µL of an off-target RNA (39-mer Arf; 10 aM to 10 pM) were added thereto.

The reaction was allowed to proceed at 37°C for 2 hours.

**[Table 33]**

| Table 33. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |

### 3) Visual Observation

Changes in the nanoparticles were visually observed.

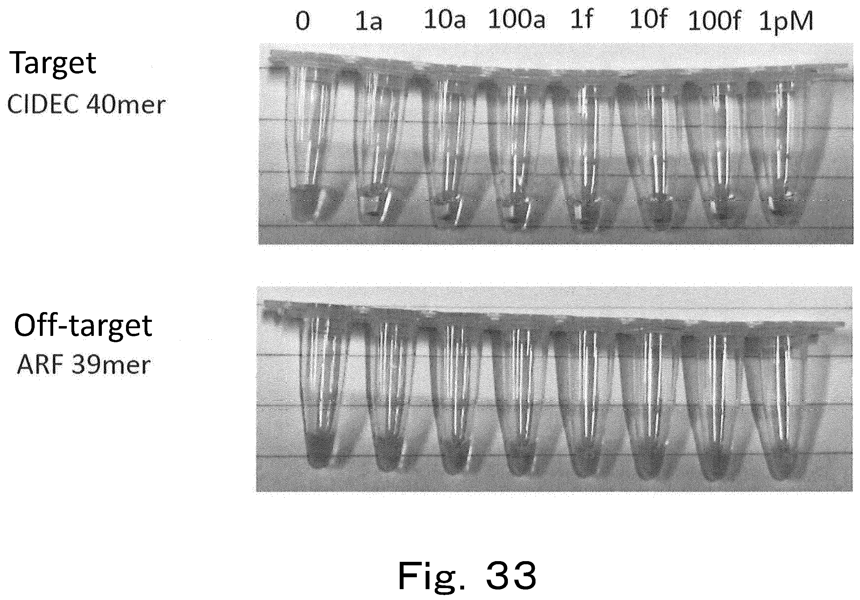

The results are shown in Fig. 33.

At a concentration of 1 aM (12 copies/tube), the 40-mer target could be visually detected.

### [Example 25]

### Test of Detection Specificity and Detection Sensitivity (Continued) (ThT-PEG-ThT Compound 5)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (full-length CIDEC; 10 aM to 10 pM) were also added thereto. Alternatively, 2 µL of an off-target RNA (full-length Arf; 10 aM to 10 pM) were added thereto.

The reaction was allowed to proceed at 37°C for 2 hours.

**[Table 34]**

| Table 34. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |

### 3) Visual Observation

Changes in the nanoparticles were visually observed.

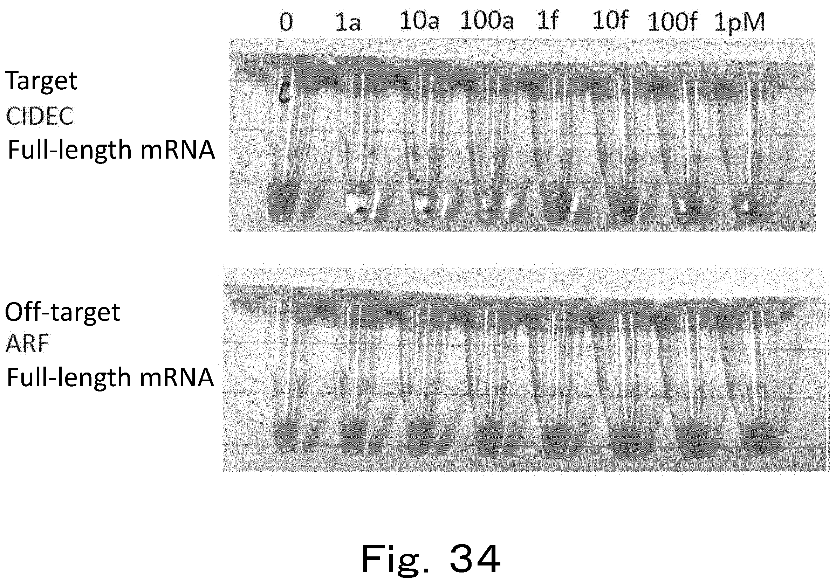

The results are shown in Fig. 34.

At a concentration of 1 aM (12 copies/tube), the full-length mRNA target could be visually detected.

### [Example 26]

### Effect of Mixed Coagulants

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Preparation of Modified Gold Colloid

### a) Providing and Washing of Gold Colloid before Use

A gold colloid (30 nm) was vortexed well, and 20 µL of the gold colloid was scooped up and placed in a 0.5-mL tube manufactured by Eppendorf.

By centrifugation in a centrifuge, the gold colloid was separated from the supernatant. After removing the supernatant, 40 µL of 1×ϕ29 DNA polymerase reaction buffer was added to the colloid, followed by pipetting.

The above operation was further carried out twice.

### b) Immobilization of ThT-Biotin (Compound 3) and PEG-Biotin (Compound 4) on Gold Colloid, and Washing

By centrifugation in a centrifuge, the gold colloid was separated from the supernatant. The supernatant was removed.

To the washed gold colloid, 2 µL of a mixed solution of ThT-biotin (100 µM) and PEG-biotin (100 µM) was added.

Incubation was carried out at 25°C for 30 minutes. During the incubation, vortexing was carried out at 5-minute intervals.

By centrifugation in a centrifuge, the gold colloid was separated from the supernatant. After removing the supernatant, 20 µL of 1×ϕ29 DNA polymerase reaction buffer was added to the colloid, followed by pipetting.

The above operation was further carried out twice. The prepared colloid was stored under refrigeration until use.

### 3) Reaction Using Primer-Immobilized Nanoparticles and Coagulant (Modified Gold Colloid)

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Fourteen microliters of SATIC reagent were added to the beads, and 2 µL of a target RNA (full-length CIDEC; 10 aM) were also added thereto. Alternatively, 2 µL of an off-target RNA (full-length Arf; 10 aM) were added thereto.

As a coagulant(s), 6 µL of a mixture of Compound 3, Compound 5, and/or the modified gold colloid was added. In the cases where one coagulant was used, 4 µL of water was added. In the cases where two coagulants were used, 2 µL of water was added.

The reaction was allowed to proceed at 37°C for 1 hour.

**[Table 35]**

| Table 35. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| Compound 2 | 10µM |
| Compound 3 | 1µM |
| Compound 4 | 1µM |
| Compound 5 | 10µM |

### 4) Visual Observation

Changes in the nanoparticles were visually observed. The observation was carried out over time.

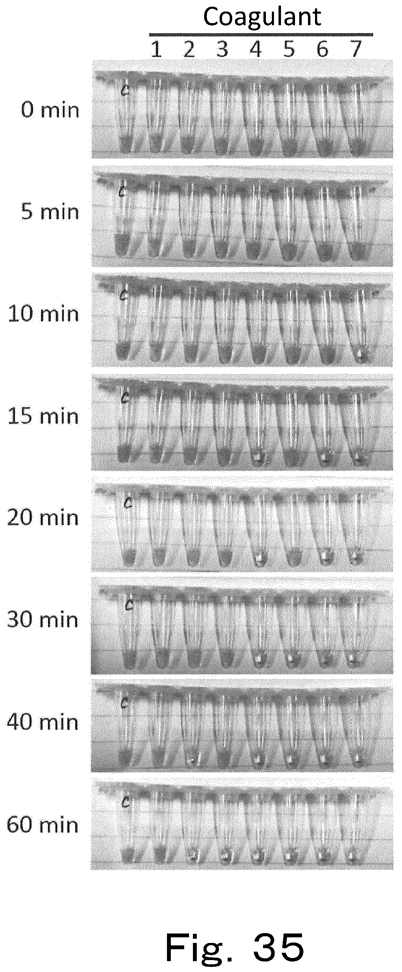

### The results are shown in Fig. 35.

When the reaction solution contained Coagulant 7, the full-length target at 1 aM (12 copies/tube) could be visually detected at about 5 minutes.

### [Synthesis Examples]

### Synthesis of ThT Derivatives

According to the following scheme, ThT-biotin and ThT-PEG-ThT were synthesized. The synthesis methods for ThT-PEG and ThT-AE were as described in a reference (Kataoka, Y; Fujita, H.; Afanaseva, A.; Nagao, C.; Mizuguchi, K.; Kasahara, Y; Obika, S.; Kuwahara, M. Biochemistry, 2019, 58, 493.).

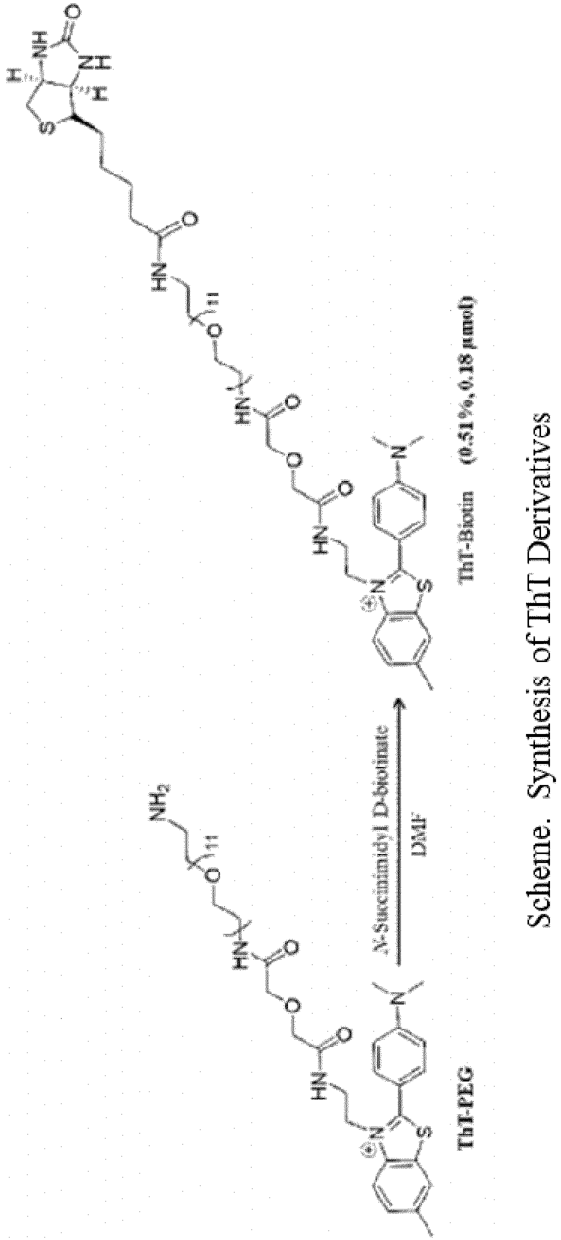

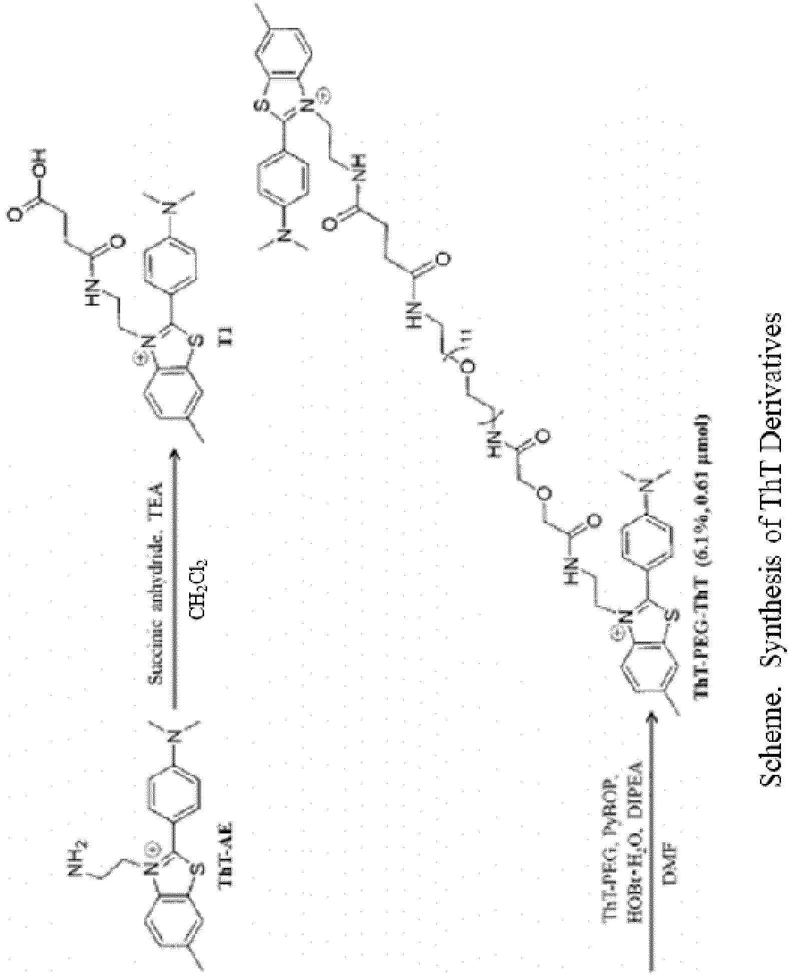

### [Synthesis of ThT-Biotin]

To ThT-PEG (33 mg, 35 µmol), dry N,N-dimethylformamide (DMF) (0.30 mL) was added, and the resulting mixture was stirred, followed by adding N-succinimidyl D-biotinate (12 mg, 35 µmol) thereto, and stirring the resulting mixture at 90°C for 2 hours. After evaporating the reaction mixture under reduced pressure, the mixture was purified using HPLC, to obtain ThT-biotin. Yield: 0.21 mg, Yield: 0.51%.

¹H NMR (500 MHz, Deuterium oxide) δ 8.01 (1H, d) 7.98 (1H, s) 7.76 (2H, d) 7.73 (1H, d) 6.97 (2H, d) 5.12 (2H, s) 4.58 (1H, q) 4.39 (1H, q) 3.87 (2H, s) 3.71 (2H, s) 3.68-3.66 (40H, m) 3.61 (3H, q) 4.41 (2H, t) 3.36 (2H, q) 3.29 (1H, q) 3. 20 (2H, q) 3.11 (6H, s) 3.04 (1H, q) 2.96 (1H, dd) 2.74 (1H, d) 2.57 (3H, s) 2.24 (2H, t) 1.72-1.49 (4H, m) 1.27 (2H, q); ESI-MS (positive ion mode) m/z, found = 1180.75, calculated for [M⁺] = 1180.59.

### [Synthesis of Compound T1]

To ThT-AE (32 mg, 0.10 mmol), dry dichloromethane (CH₂Cl₂) (0.30 mL) was added. After stirring the resulting mixture, triethylamine (TEA) (85 µL, 0.61 mmol) was added thereto, and then succinic anhydride (11 mg, 0.11 mmol) was added thereto, followed by stirring the resulting mixture at room temperature for 30 minutes. After evaporating the reaction mixture under reduced pressure, the residue was suspended in water, and washed with CH₂Cl₂. The aqueous layer was evaporated under reduced pressure, to quantitatively obtain Compound T1. ESI-MS (positive ion mode) m/z, found =412.15, calculated for [M⁺] =412.17.

### [Synthesis of ThT-PEG-ThT]

To ThT-PEG (10 mg, 10 µmol), dry DMF (0.3 mL) was added. After stirring the resulting mixture, HOBt·H₂O (4. 2 mg, 26 µmol) and PyBOP (14 mg, 26 µmol) were added thereto, and then DIPEA (14 µL, 80 µmol) was added thereto. To the resulting mixture, Compound T1 (5.6 mg, 13 µmol) dissolved in dry DMF (0.2 mL) was added, and the mixture was stirred at room temperature for 5 hours. After evaporating the reaction mixture under reduced pressure, the residue was dissolved in CH₂Cl₂, followed by washing with water. After evaporating the organic layer under reduced pressure, solid-liquid extraction was carried out with diethyl ether, followed by purification using HPLC, to obtain ThT-PEG-ThT. Yield: 0.82 mg, Yield ratio: 6.1%.

¹H NMR (500 MHz, Deuterium oxide) δ 8.01 (2H, d) 7.97 (2H, s) 7.75 (6H, t) 6.95 (4H, d) 5.12 (4H, t) 3.86 (4H, s) 3.68 (44H, q) 3.61 (4H, q) 3.42-3.35 (4H, m) 3.10 (12H, s) 2.65 (4H, t) 2.57 (3H, s) 2.54 (3H, t); ESI-MS (positive ion mode) m/z, found = 1348.51, calculated for [H⁺] = 1348.67.

### [Example 27]

### Effects of Compound 5 (ThT-PEG-ThT) and Various PEGs as Aggregation Promoters (Continued)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P1-P2-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM) were also added thereto.

The reaction was allowed to proceed at 37°C for 20 minutes.

**[Table 36]**

| Table 36. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |
| Various PEGs | 10w/v% |
| PEG200, PEG600, PEG1000 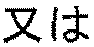 PEG6000) | |

### 3) Visual Observation

Changes in the nanoparticles were visually observed.

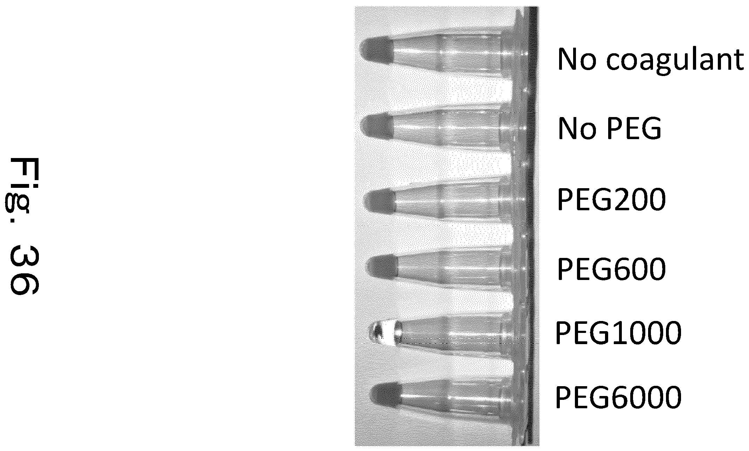

The results are shown in Fig. 36.

In the present Example, wherein ThT-PEG-ThT and various PEGs at 10 w/v% were used as coagulants, aggregation occurred in the case where PEG1000 was added.

### [Example 28]

### Effects of Compound 5 (ThT-PEG-ThT) and Various PEGs as Aggregation Promoters (Study on PEG Concentration)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P1-P2-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM) were also added thereto.

The reaction was allowed to proceed at 37°C for 20 minutes.

**[Table 37]**

| Table 37. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |
| Various PEGs | 0~20w/v% |

### 3) Visual Observation

Changes in the nanoparticles were visually observed.

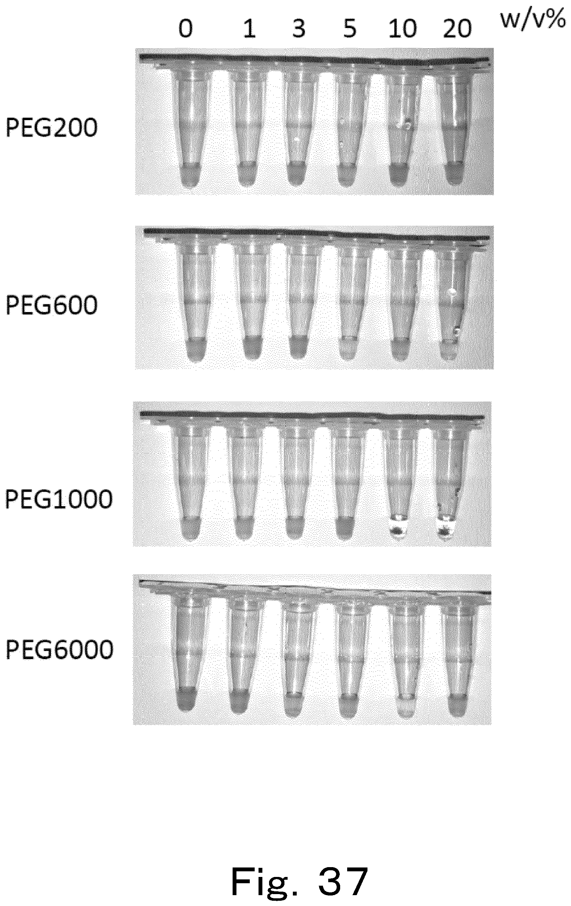

The results are shown in Fig. 37.

In the present Example, wherein ThT-PEG-ThT and PEG1000 were used as coagulants, the addition at 10 to 20 w/v% caused formation of an aggregate after a reaction time of about 20 minutes.

### [Example 29]

### Effects of Crown Ether and Its Salt Concentration on SATIC Reaction

### 1) Preparation of Primer-Immobilized Nanoparticles

### a) Preparation of Biotinylated Primers

A biotinylated capture probe (CS-thr) and a biotinylated primer (P2) were dissolved in 1×ϕ29 DNA polymerase reaction buffer, to prepare a CS-thr-P2 mixed solution of CS-thr (1 µM) and P2 (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the CS-thr-P2 mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

### 2) Reaction Using Primer-Immobilized Nanoparticles

Two microliters of the CS-thr-primer (P2)-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL (1 fM) of a target (thrombin) or a non-target (streptavidin) were further added thereto.

**[Table 38]**

| Table 38. Final concentrations of the SATIC reagent and the primers in the reaction solution | |
|---|---|
| Component of the reaction solution | Concentration |
| cT₁ | 100 nM |
| cT₂ | 400 nM |
| CS-thr | 0.20 pmol/tube |
| P₁-thr | 120 nM |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |
| PEG1000 | 10w/v% |
| Various crown ethers (12-crown-4, 15-crown-5, or 18-crown-6) | 0~300mM |

### 3) Visual Observation

Changes in the nanoparticles were visually observed.

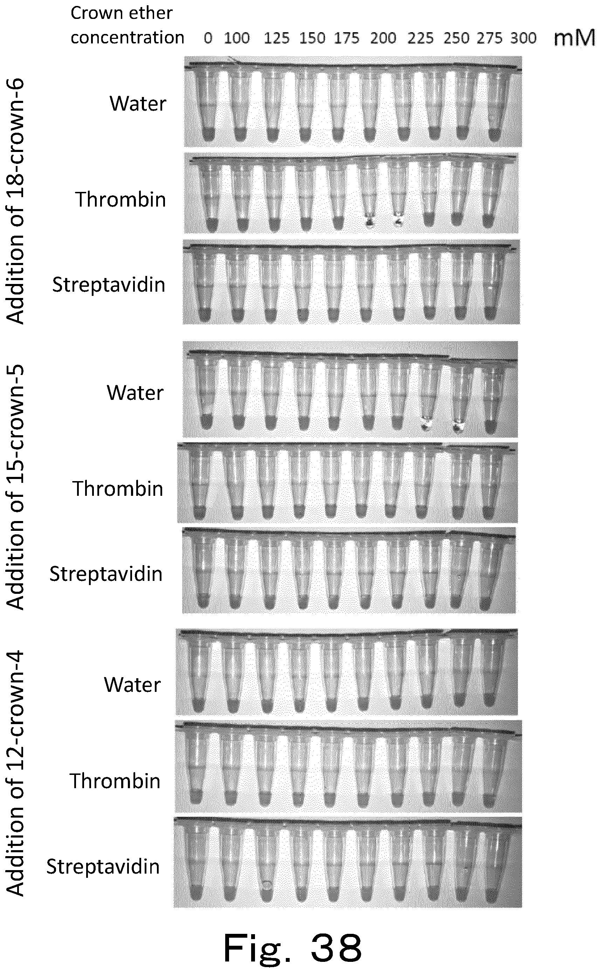

The results are shown in Fig. 38.

Normally, in the presence of a high concentration of sodium ions, the SATIC reaction hardly proceeds. It was found, however, that addition of 18-crown-6 or 15-crown-5 allows the SATIC reaction to proceed even in a case where 150 mM NaCl is added to the reaction solution.

### [Example 30]

### Study on Concentration of Surfactant Added

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles and Coagulant

Two microliters of the P₁-P₂-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM) were also added thereto.

The reaction was allowed to proceed at 37°C for 20.

**[Table 39]**

| Table 39. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |
| PEG1000 | 10w/v% |
| Various Surfactants (Tween 20 or Nonidet P-40) | 0~3v/v% |

### 3) Visual Observation

Changes in the nanoparticles were visually observed. The observation was carried out over time.

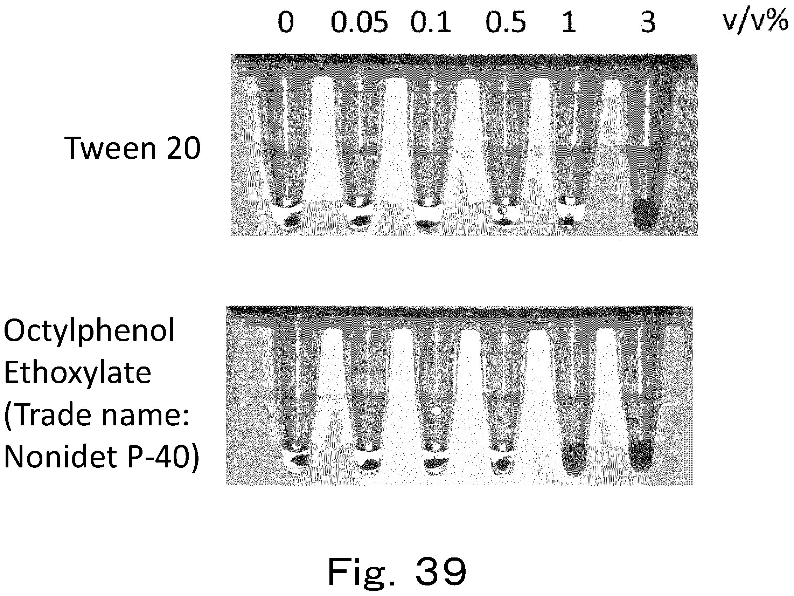

The results are shown in Fig. 39.

Nonionic surfactants are used for improvement of wettability and stability of nucleic acids and proteins. They are often added for treatment of a biological sample under stable conditions. In view of this, effects of representative surfactants on the SATIC reaction were investigated. As a result, it was found that Tween 20 is acceptable up to about 1%, and that Nonidet P-40 is acceptable up to about 0.5% in the reaction solution.

### [Example 31]

### Method of Aggregation after Reaction (Study on Temperature)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P1-P2-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM) were also added thereto.

The reaction was allowed to proceed at 37°C for 20 minutes.

### 3) Post-Reaction Treatment

One of the following treatments was carried out after the reaction.
1) The nanoparticles were accumulated by application of a magnet to the reaction solution.
2) The tube was shaken to uniformly distribute the nanoparticles.
3) The beads were left to stand as they were.

### 9-1-4) Cooling

After carrying out the above post-reaction treatment, the beads were left to stand for 5 minutes at one of the following temperatures.
1) 4°C
2) 0°C
3) -21°C
4) 25°C (room temperature)
5) Post-Cooling Treatment

One of the following treatments was carried out after the cooling.
1) The tube was shaken to uniformly distribute the nanoparticles, and then the nanoparticles were accumulated using a magnet.
2) After the cooling, the nanoparticles were accumulated as they were using a magnet.
6) The tube, containing ArfR, was shaken until the nanoparticles in the tube became uniform.

**[Table 40]**

| Table 40. Final concentrations of the SATIC reagent and the primers in the reaction solution | |
|---|---|
| Component of the reaction solution | Concentration |
| cT₁ | 100 nM |
| cT₂ | 400 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |
| PEG1000 | 10w/v% |
| Nonidet P-40 | 0.05% |

In the following Table, 1 to 24 represent Conditions 1 to 24.

**[Table 41]**

| Table 41 | | | | | | | |
|---|---|---|---|---|---|---|---|
| | Post-reaction treatment | Accumulation of nanoparticles using a magnet | | Uniform distribution of nanoparticles | | No treatment | |
| | Post-cool ing treatment | Accumulation using a magnet after uniform distribution | Direct accumulation using a magnet | Accumulation using a magnet after uniform distribution | Direct accumulation using a magnet | Accumulation using a magnet after uniform distribution | Direct accumulation using a magnet |
| Cooling temperature | 4°C | 1 | 2 | 3 | 4 | 5 | 6 |
| | 0°C | 7 | 8 | 9 | 10 | 11 | 12 |
| | -21°C | 13 | 14 | 15 | 16 | 17 | 18 |
| | 25°C | 19 | 20 | 21 | 22 | 23 | 24 |

### 7) Visual Observation

Changes in the nanoparticles were visually observed.

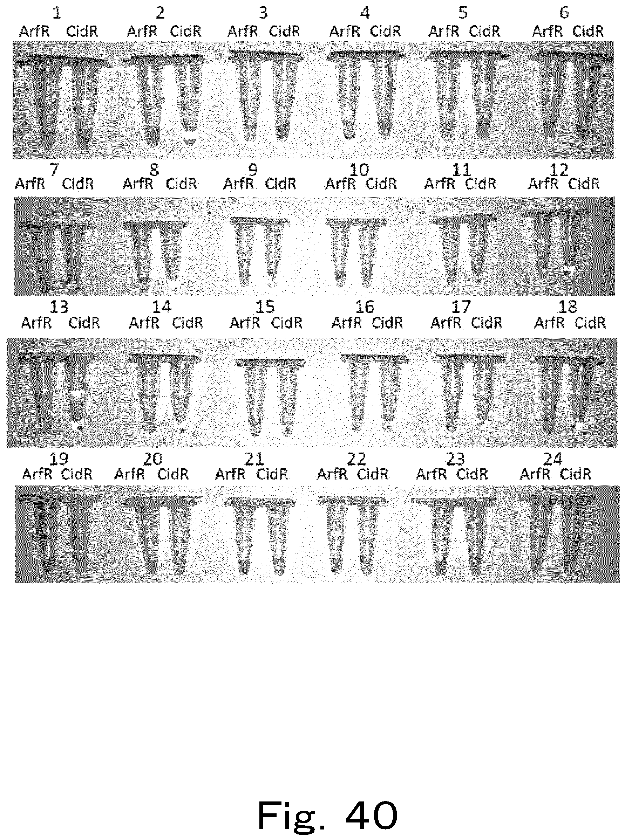

The results are shown in Fig. 40.

Aggregation quickly occurred under the conditions at cooling temperatures of 0°C and -21°C (especially under the conditions of 8 and 14).

### [Example 32]

### Method of Aggregation after Reaction (Study on Length of Time)

### 1) Preparation of Primer-Immobilized Nanoparticles

### a) Preparation of Biotinylated Primers

A biotinylated primer (P₁) and a biotinylated primer (P₂) were dissolved in 1×ϕ29 DNA polymerase reaction buffer, to prepare a P₁-P₂ mixed solution of P₁ (1 µM) and P₂ (20 µM).

### b) Providing and Washing of Nanoparticles before Use

FG beads (FG beads streptavidin) were stirred by vortexing well to obtain uniform particles. Four microliters of the resulting beads were scooped up and placed in a 1.5-mL tube manufactured by Eppendorf.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

### c) Immobilization of Primers on Nanoparticles, and Washing

The tube was placed in a magnetic rack (a tube stand with a magnet), to separate the magnetic beads from the supernatant (5 minutes). The supernatant was removed.

To the washed FG beads, 4 µL of the P₁-P₂ mixed solution was added.

Incubation was carried out at 25°C for 30 minutes. During the incubation, vortexing was carried out at 5-minute intervals.

The tube was placed in a magnetic rack (a tube stand with a magnet), to separate the magnetic beads from the supernatant (5 minutes). After removing the supernatant, 40 µL of 1×ϕ29 DNA polymerase reaction buffer was added to the beads, followed by pipetting.

The above operation was further carried out twice.

The tube was placed in a magnetic rack (a tube stand with a magnet), to separate the magnetic beads from the supernatant (5 minutes). After removing the supernatant, 40 µL of water was added to the beads, followed by pipetting.

The above operation was further carried out twice. The prepared beads were stored under refrigeration until use.

### 2) Reaction Using Primer-Immobilized Nanoparticles

Two microliters of the P1-P2-immobilized FG beads were scooped up and placed in a 0.5-mL tube manufactured by Eppendorf.

The tube was placed in a magnetic rack (a tube stand with a magnet), to separate the magnetic beads from the supernatant (1 minute). The supernatant was removed.

Eighteen microliters of SATIC reagent were added to the beads, and 2 µL of a target RNA (40-mer CIDEC; 10 aM) were also added thereto.

The reaction was allowed to proceed at 37°C for 20 minutes.

### 3) Post-Reaction Treatment

The nanoparticles were accumulated by application of a magnet to the reaction solution.

### 4) Cooling

After carrying out the above post-reaction treatment, the beads were left to stand for 0, 1, 3, 5, or 10 minutes at one of the following temperatures.
1) 0°C (Condition 8)
2) -21°C (Condition 14)

### 5) Post-Cooling Treatment

One of the following treatments was carried out after the cooling.
1) The tube was shaken to uniformly distribute the nanoparticles, and then the nanoparticles were accumulated using a magnet.
2) After the cooling, the nanoparticles were accumulated as they were using a magnet.
6) The tube, containing ArfR, was shaken until the nanoparticles in the tube became uniform.

**[Table 42]**

| Table 42. Final concentrations of the SATIC reagent and the primers in the reaction solution | |
|---|---|
| Component of the reaction solution | Concentration |
| cT₁ | 10 nM |
| cT₂ | 40 nM |
| P₁ | 0.20 pmol/tube |
| P₂ | 4.0 pmol/tube |
| *φ* 29 DNA polymerase reaction buffer | 1 × |
| BSA | 0.1 mg/µL |
| *φ* 29 DNA polymerase | 0.2 U/µL |
| ThT-PEG-ThT | 10 µM |
| PEG1000 | 10w/v% |
| Nonidet P-40 | 0.05% |

### 7) Visual Observation

Changes in the nanoparticles were visually observed.

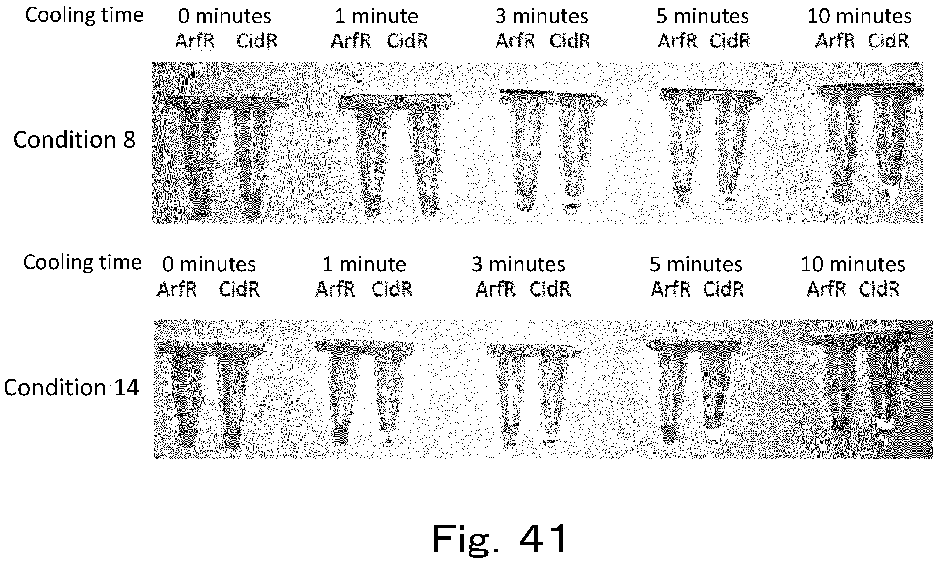

The results are shown in Fig. 41.

Under the condition at a cooling temperature of 0°C, aggregation occurred after a cooling time of 3 minutes. Under the condition at -21°C, aggregation occurred after a cooling time of 1 minute. It was thus found that faster observation of the aggregation is possible by cooling.

### DESCRIPTION OF SYMBOLS

20 ... Single-stranded circular DNA; 21 ... target nucleic acid; 22 ... first oligonucleotide primer; 23 ... first amplification product; 24 ... second single-stranded circular DNA; 25 ... second oligonucleotide primer; 26 ... second amplification product; 201 ... sequence complementary to the first site; 202 ... first-primer-binding sequence; 203 ... sequence complementary to a sequence that binds to the second single-stranded circular DNA; 204 ... site adjacent to the 5'-side of 203; 243 ... sequence complementary to the guanine-quadruplex-forming sequence; 261 ... sequence containing a guanine quadruplex; 262 ... guanine quadruplex detection reagent; 211 ... first site; 212 ... second site; 221 ... sequence complementary to the second site; 222 ... sequence complementary to the first-primer-binding site; 231 ... region complementary to 203; 232 ... region complementary to the site 204; 233 ... sequence complementary to the sequence 203; 234 ... region complementary to the site 204 of the first single-stranded circular DNA 20; 241 ... same sequence as the sequence 203 complementary to the sequence that binds to the second single-stranded circular DNA; 242 ... second-primer-binding sequence; 251 ... same sequence as the site 204; 252 ... sequence complementary to the second-primer-binding sequence 242 of the second single-stranded circular DNA; 27 ... target miRNA containing a mutation; 271 ... first site; 272 ... second site; 28 ... first oligonucleotide primer; 281 ... sequence complementary to the second site; 282 ... sequence complementary to the first-primer-binding site.
30 ... First single-stranded circular DNA; 31 ... capture oligonucleotide; 32 ... first oligonucleotide primer; 33 ... first amplification product (extended chain); 34 ... second single-stranded circular DNA; 35 ... second oligonucleotide primer; 36 ... second amplification product (extended chain); 37 ... target molecule; 38 ... guanine quadruplex detection reagent; 301 ... first region (primer-binding sequence); 302 ... second region; 303 ... sequence complementary to the sequence that binds to the second single-stranded circular DNA; 304 ... region adjacent to the 5'-side of 303; 311 ... sequence complementary to the second region; 312 ... second aptamer sequence; 321 ... first aptamer sequence; 322 ... sequence complementary to the first region; 331 ... region complementary to 303; 332 ... region complementary to the region 304; 341 ... same sequence as the sequence 303 complementary to the sequence that binds to the second single-stranded circular DNA; 342 ... second-primer-binding sequence; 343 ... sequence complementary to the guanine-quadruplex-forming sequence; 351 ... same sequence as the region 304; 352 ... sequence complementary to the second-primer-binding sequence 342 of the second single-stranded circular DNA; 361 ... sequence containing a guanine quadruplex.
40 ... Single-stranded circular DNA; 41 ... capture oligonucleotide; 42 ... miRNA; 43 ... first amplification product (extension product); 44 ... second single-stranded circular DNA; 45 ... second oligonucleotide primer; 46 ... second amplification product (extension product); 47 ... guanine quadruplex detection reagent; 401 ... sequence complementary to the second region of the miRNA; 402 ... second region of the single-stranded circular DNA; 403 ... sequence complementary to the sequence that binds to the second single-stranded circular DNA; 404 ... region adjacent to the 5'-side of 403; 411 ... sequence complementary to the second region of the single-stranded circular DNA; 412 ... sequence complementary to the first region of the miRNA; 421 ... first region of the miRNA; 422 ... second region containing the mutation of the miRNA; 431 ... region complementary to 403; 432 ... region complementary to the region 404; 433 ... sequence complementary to the sequence 403; 434 ... region complementary to the region 404 of the first single-stranded circular DNA 40; 441 ... same sequence as the sequence 403 complementary to the sequence that binds to the second single-stranded circular DNA; 442 ... second-primer-binding sequence; 443 ... sequence complementary to the guanine-quadruplex-forming sequence; 451 ... same sequence as the region 404; 452 ... sequence complementary to the second-primer-binding sequence 442 of the second single-stranded circular DNA; 461 ... guanine-quadruplex-forming sequence.

## Claims

1. A nucleic acid detection kit comprising:
(i) a first single-stranded circular DNA containing:
a sequence of 10 to 30 bases complementary to a first site of a target nucleic acid;
a first-primer-binding sequence of 7 to 8 bases adjacent to the 5'-side of this sequence; and
a sequence complementary to a sequence that binds to a second single-stranded circular DNA;
(ii) a first oligonucleotide primer containing:
a sequence of 8 to 15 bases complementary to a second site adjacent to the 3'-side of the first site of the target nucleic acid; and
a sequence of 7 to 8 bases adjacent to the 3'-side of this sequence and complementary to the first-primer-binding site of the first single-stranded circular DNA;
(iii) a second single-stranded circular DNA containing:
the same sequence as the sequence in the first single-stranded circular DNA, complementary to the sequence that binds to the second single-stranded circular DNA; and
a second-primer-binding sequence adjacent to the 5'-side of this sequence; and
(iv) a second oligonucleotide primer containing:
the same sequence as the site in the first single-stranded circular DNA, adjacent to the 5'-side of the sequence complementary to the sequence that binds to the second single-stranded circular DNA, wherein said same sequence as the site in the first single-stranded circular DNA is 8 to 15 bases; and
a sequence of 7 to 8 bases adjacent to the 3'-side of this sequence and complementary to the second-primer-binding sequence of the second single-stranded circular DNA,
wherein the first oligonucleotide primer is bound to a carrier through the 5'-end thereof, and the second oligonucleotide primer is bound, through the 5'-end thereof, to the carrier to which the first oligonucleotide primer is bound.

2. The nucleic acid detection kit according to claim 1,
wherein
the first oligonucleotide primer is modified with biotin at the 5'-end thereof, and bound, through the biotin, to a carrier on which avidin is immobilized, and
the second oligonucleotide primer is modified with biotin at the 5'-end thereof, and bound, through the biotin, to the carrier to which the first oligonucleotide primer is bound, and/or
wherein the ratio between the first oligonucleotide primer and the second oligonucleotide primer bound to the carrier is 1:10 to 1:30 in terms of the molar ratio.

3. The nucleic acid detection kit according to any one of claims 1 to 2, comprising (v) a detection reagent,
wherein the second single-stranded circular DNA contains a sequence complementary to a detection reagent-binding sequence, preferably wherein the detection reagent-binding sequence is a guanine-quadruplex-forming sequence, and
the detection reagent is a guanine-quadruplex-binding reagent, more preferably wherein the sequence complementary to the guanine-quadruplex-forming sequence contains a C₃N₁₋₁₀C₃N₁₋₁₀C₃N₁₋₁₀C₃ sequence.

4. The nucleic acid detection kit according to claim 3, wherein the guanine-quadruplex-binding reagent contains a compound represented by the following General Formula (I): 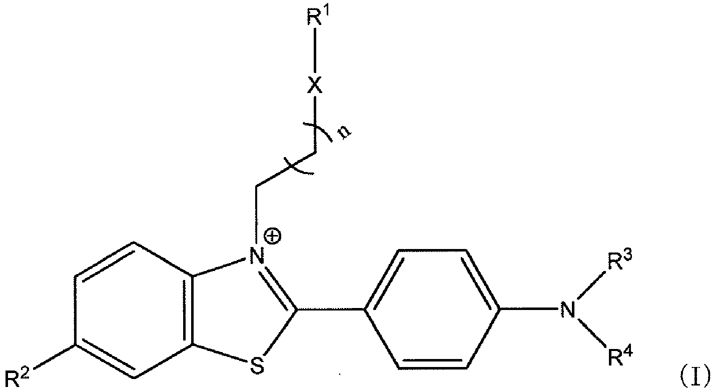 wherein
R¹ represents hydrogen, or a hydrocarbon group which optionally contains one or more selected from O, S, and N,
R², R³, and R⁴ each independently represent a C₁-C₅ hydrocarbon group;
n represents an integer of 0 to 5, and
X represents O, S, or NH, preferably
wherein the compound represented by General Formula (I) is represented by the following Formula (II) or (III)

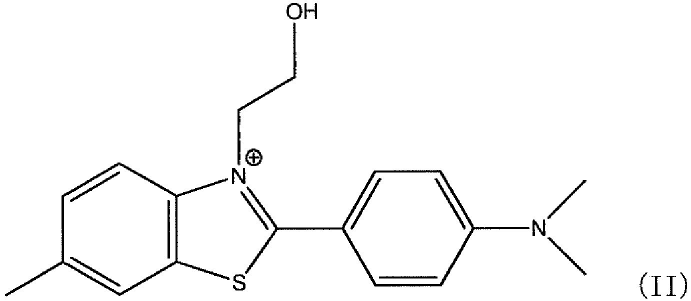

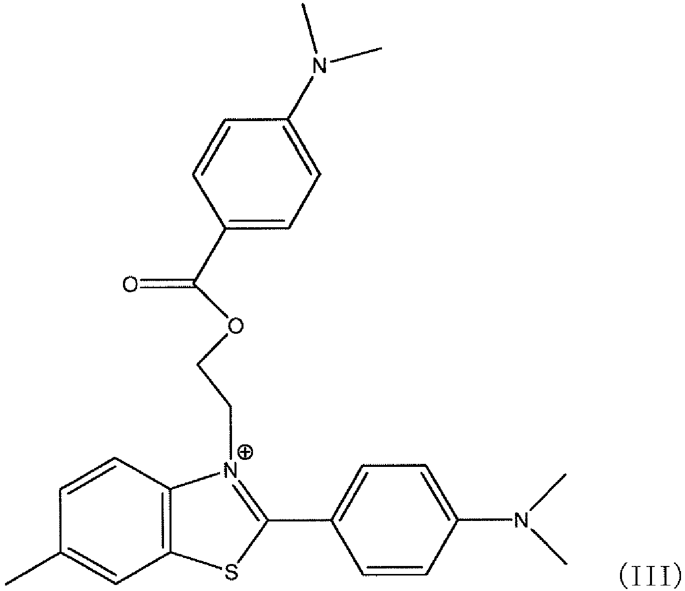

5. The nucleic acid detection kit according to claim 4, wherein the guanine-quadruplex-binding reagent is the following compound: 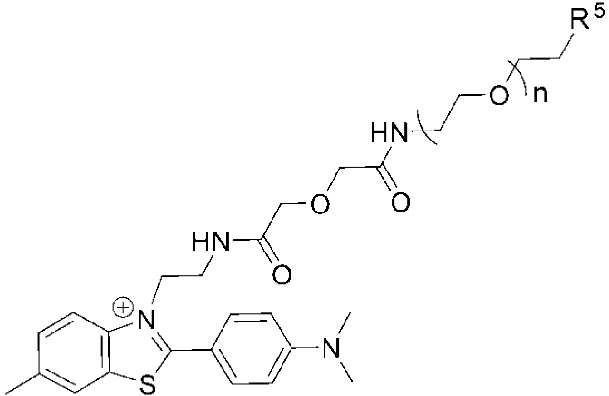
(wherein R⁵ represents an amino group, a hydroxyl group, an alkyl group, or a carboxyl group, and n represents an integer of 4 to 50), or
wherein the guanine-quadruplex-binding reagent is the following compound: 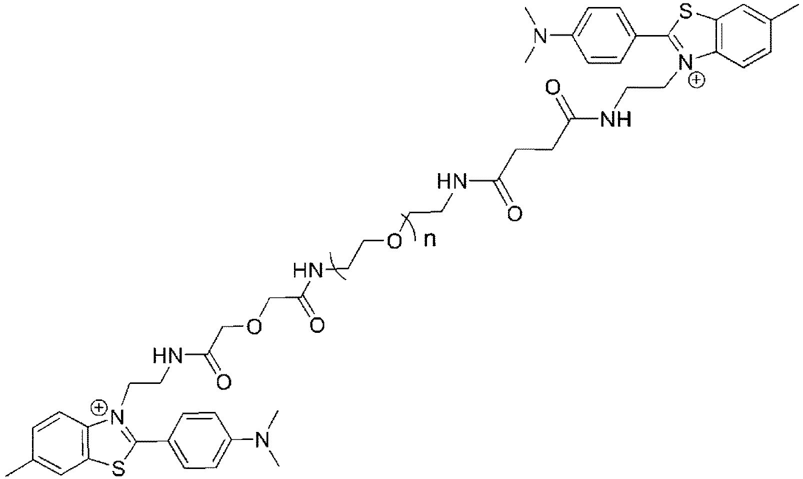
(wherein n represents an integer of 4 to 50).

6. The nucleic acid detection kit according to claim 5, wherein the compound is immobilized on a carrier together with a polyethylene glycol chain.

7. The nucleic acid detection kit according to any preceding claim, comprising a crown ether, preferably wherein the crown ether is 18-crown-6 or 15-crown-5.

8. The nucleic acid detection kit according to any one of claims 1 to 7, wherein said nucleic acid detection kit comprises a nonionic surfactant, preferably wherein the nonionic surfactant is polyoxyethylene sorbitan monolaurate or octylphenol ethoxylate.

9. The nucleic acid detection kit according to any preceding claim, wherein the target nucleic acid is viral RNA.

10. A method of detecting a target nucleic acid using the kit according to any one of claims 1 to 9, the method comprising the steps of:
hybridizing the first single-stranded circular DNA and the first oligonucleotide primer with the target nucleic acid;
performing a nucleic acid amplification reaction based on the target nucleic acid by rolling circle amplification from the first oligonucleotide primer;
hybridizing the second single-stranded circular DNA and the second oligonucleotide primer with the obtained amplification product;
performing a nucleic acid amplification reaction based on the amplification product by rolling circle amplification from the second oligonucleotide primer; and
detecting an amplified nucleic acid.

11. A nucleic acid detection kit comprising:
a short-chain target nucleic acid containing:
a first region; and
a second region in the 3'-side of the first region, the second region containing a mutation;
(i) a first single-stranded circular DNA containing:
a region that binds to the short-chain target nucleic acid, the region being complementary to the second region of the short-chain target nucleic acid;
a second region in the 3'-side thereof; and
a sequence complementary to a sequence that binds to a second single-stranded circular DNA;
(ii) a capture oligonucleotide containing:
a template-binding sequence complementary to the second region of the single-stranded circular DNA; and
a sequence that binds to the short-chain target nucleic acid, the sequence being complementary to the first region of the short-chain target nucleic acid;
(iii) a second single-stranded circular DNA containing:
the same sequence as the sequence in the first single-stranded circular DNA, complementary to the sequence that binds to the second single-stranded circular DNA;
a second-primer-binding sequence adjacent to the 5'-side of this sequence; and
a sequence complementary to a detection reagent-binding sequence; and
(iv) a second oligonucleotide primer containing:
the same sequence as the region in the first single-stranded circular DNA, adjacent to the 5'-side of the sequence complementary to the sequence that binds to the second single-stranded circular DNA, wherein said same sequence as the region in the first single-stranded circular DNA is 8 to 15 bases; and
a sequence of 7 to 8 bases adjacent to the 3'-side of this sequence and complementary to the second-primer-binding sequence of the second single-stranded circular DNA,
wherein the capture oligonucleotide is bound to a carrier through the 5'-end thereof, and the second oligonucleotide primer is bound, through the 5'-end thereof, to the carrier to which the capture oligonucleotide is bound.

12. A method of detecting a short-chain target nucleic acid using the kit according to claim 11, the method comprising the steps of:
hybridizing the first single-stranded circular DNA and the capture polynucleotide with the short-chain target nucleic acid containing: the first region; and the second region adjacent to the 3'-side of the first region and containing the mutation;
performing a nucleic acid amplification reaction by rolling circle amplification based on the formation of a complex of the short-chain target nucleic acid, the capture oligonucleotide, and the first single-stranded circular DNA;
hybridizing the second single-stranded circular DNA and the second oligonucleotide primer with an extended chain generated by the nucleic acid amplification reaction, and
performing a nucleic acid amplification reaction based on the formation of a complex of the extended chain, the second primer, and the second single-stranded circular DNA; and
detecting an amplified nucleic acid.

13. A kit for detecting a target molecule, the kit comprising:
(i) a first single-stranded circular DNA containing:
a first region;
a second region linked to the 3'-side thereof; and
a sequence complementary to a sequence that binds to a second single-stranded circular DNA;
(ii) a first oligonucleotide primer containing:
a first aptamer sequence which binds to the target molecule; and
a sequence linked to the 3'-side thereof and complementary to the first region of the first single-stranded circular DNA;
(iii) a capture oligonucleotide containing:
a sequence complementary to the second region of the first single-stranded circular DNA; and
a second aptamer sequence linked to the 3'-side thereof, which binds to the target molecule,
(iv) a second single-stranded circular DNA containing:
the same sequence as the sequence in the first single-stranded circular DNA, complementary to the sequence that binds to the second single-stranded circular DNA; and
a sequence which is adjacent to the 5'-side of this sequence and which binds to a second oligonucleotide primer; and
(v) a second oligonucleotide primer containing:
the same sequence as the region in the first single-stranded circular DNA, adjacent to the 5'-side of the sequence complementary to the sequence that binds to the second single-stranded circular DNA, wherein said same sequence as the site in the first single-stranded circular DNA is 8 to 15 bases; and
a sequence of 7 to 8 bases adjacent to the 3'-side of this sequence and complementary to the sequence in the second single-stranded circular DNA, that binds to the second oligonucleotide primer,
wherein the capture oligonucleotide and/or the first oligonucleotide primer is/are bound to a carrier through the 5'-end(s) thereof, and the second oligonucleotide primer is bound, through the 5'-end thereof, to the carrier to which the capture oligonucleotide and/or the first oligonucleotide primer is/are bound.

14. A method of detecting a target molecule using the kit according to claim 13, the method comprising the steps of:
forming a first complex containing the target molecule, the capture oligonucleotide, the first oligonucleotide primer, and the first single-stranded circular DNA;
performing a nucleic acid amplification reaction by rolling circle amplification based on the formation of the first complex;
hybridizing the second single-stranded circular DNA and the second oligonucleotide primer with an extended chain generated by the nucleic acid amplification reaction, to form a second complex containing the extended chain, the second oligonucleotide primer, and the second single-stranded circular DNA;
performing a nucleic acid amplification reaction by rolling circle amplification based on the formation of the second complex; and
detecting an amplified nucleic acid.

15. A compound represented by the following formula: 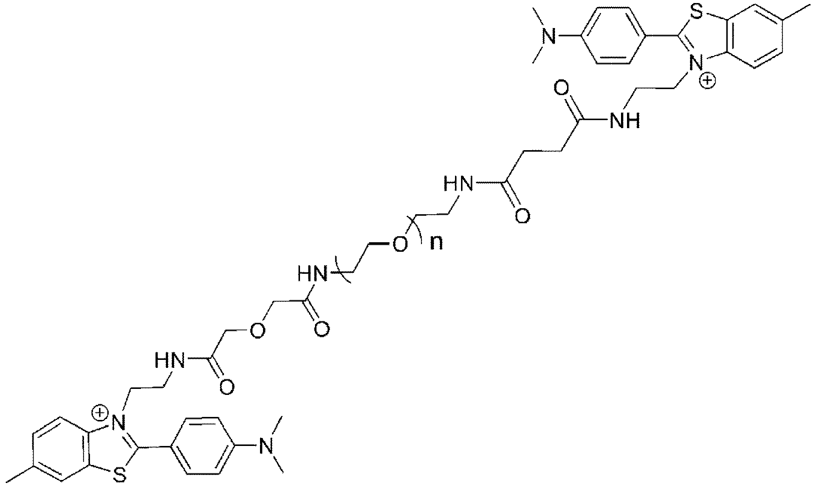 (wherein n represents an integer of 4 to 50).

## Patentansprüche

1. Nukleinsäurenachweiskit, umfassend:
(i) eine erste einzelsträngige zirkuläre DNA, die enthält:
eine Sequenz von 10 bis 30 Basen, die komplementär zu einer ersten Stelle einer Zielnukleinsäure ist;
eine erste Primer-Bindungssequenz von 7 bis 8 Basen, die an die 5'-Seite dieser Sequenz angrenzt; und
eine Sequenz, die komplementär zu einer Sequenz ist, die an eine zweite einzelsträngige zirkuläre DNA bindet;
(ii) einen ersten Oligonukleotid-Primer, der enthält:
eine Sequenz von 8 bis 15 Basen, die komplementär zu einer zweiten Stelle ist, die an die 3'-Seite der ersten Stelle der Zielnukleinsäure angrenzt; und
eine Sequenz von 7 bis 8 Basen, die an die 3'-Seite dieser Sequenz angrenzt und komplementär zu der ersten Primer-Bindungsstelle der ersten einzelsträngigen zirkulären DNA ist;
(iii) eine zweite einzelsträngige zirkuläre DNA, die enthält:
dieselbe Sequenz wie die Sequenz in der ersten einzelsträngigen zirkulären DNA, die komplementär zu der Sequenz ist, die an die zweite einzelsträngige zirkuläre DNA bindet; und
eine zweite Primer-Bindungssequenz, die an die 5'-Seite dieser Sequenz angrenzt; und
(iv) einen zweiten Oligonukleotid-Primer, der enthält:
dieselbe Sequenz wie die Stelle in der ersten einzelsträngigen zirkulären DNA, die an die 5'-Seite der Sequenz angrenzt, die komplementär zu der Sequenz ist, die an die zweite einzelsträngige zirkuläre DNA bindet, wobei dieselbe Sequenz wie die Stelle in der ersten einzelsträngigen zirkulären DNA 8 bis 15 Basen ist; und
eine Sequenz von 7 bis 8 Basen, die an die 3'-Seite dieser Sequenz angrenzt und komplementär zu der zweiten Primer-Bindungsstelle der zweiten einzelsträngigen zirkulären DNA ist,
wobei der erste Oligonukleotid-Primer über das 5'-Ende davon an einen Träger gebunden ist und der zweite Oligonukleotid-Primer über das 5'-Ende davon an den Träger gebunden ist, an den der erste Oligonukleotid-Primer gebunden ist.

2. Nukleinsäurenachweiskit nach Anspruch 1,
wobei
der erste Oligonukleotid-Primer an dem 5'-Ende davon mit Biotin modifiziert ist und über das Biotin an einen Träger gebunden ist, auf dem Avidin immobilisiert ist, und
der zweite Oligonukleotid-Primer an dem 5'-Ende davon mit Biotin modifiziert ist und über das Biotin an den Träger gebunden ist, an den der erste Oligonukleotid-Primer gebunden ist, und/oder
wobei das Verhältnis zwischen dem ersten Oligonukleotid-Primer und dem zweiten Oligonukleotid-Primer, die an den Träger gebunden sind, 1 : 10 bis 1 : 30 in Bezug auf das molare Verhältnis beträgt.

3. Nukleinsäurenachweiskit nach einem der Ansprüche 1 bis 2, umfassend (v) ein Nachweisreagenz,
wobei die zweite einzelsträngige zirkuläre DNA eine Sequenz enthält, die komplementär zu einer nachweisreagenzbindenden Sequenz ist, wobei die nachweisreagenzbindende Sequenz vorzugsweise eine Guanin-Quadruplexausbildende Sequenz ist, und
das Nachweisreagenz ein Guanin-Quadruplex-bindendes Reagenz ist, stärker bevorzugt wobei die zu der Guanin-Quadruplex-ausbildenden Sequenz komplementäre Sequenz eine C₃N₁₋₁₀C₃N₁₋₁₀C₃N₁₋₁₀C₃-Sequenz enthält.

4. Nukleinsäurenachweiskit nach Anspruch 3, wobei das Guanin-Quadruplex-bindende Reagenz eine Verbindung enthält, die durch die folgende allgemeine Formel (I) dargestellt wird: 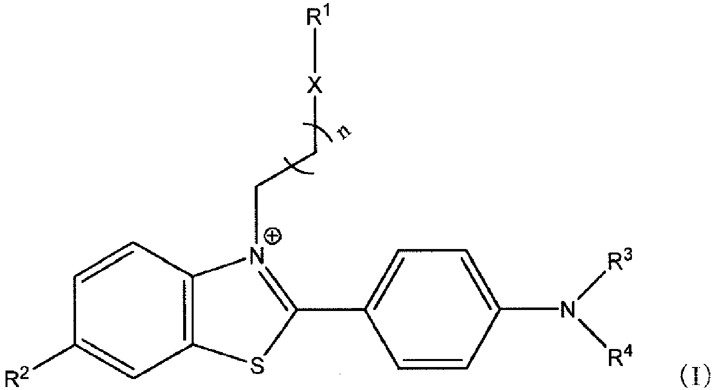 wobei
R¹ Wasserstoff oder eine Kohlenwasserstoffgruppe darstellt, die optional eine oder mehrere enthält, ausgewählt aus O, S und N,
R², R³ und R⁴ jeweils unabhängig voneinander eine C₁-C₅-Kohlenwasserstoffgruppe darstellen;
n eine ganze Zahl von 0 bis 5 darstellt, und
X O, S oder NH darstellt, vorzugsweise
wobei die durch die allgemeine Formel (I) dargestellte Verbindung durch die folgende Formel (II) oder (III) dargestellt wird

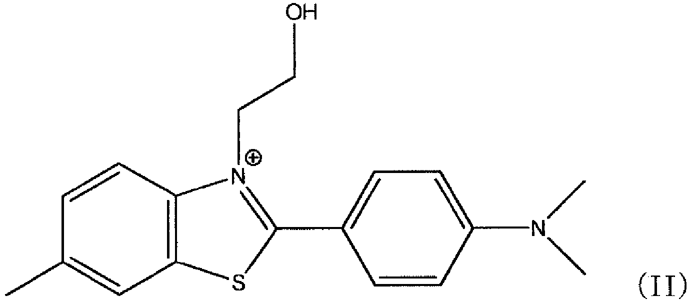

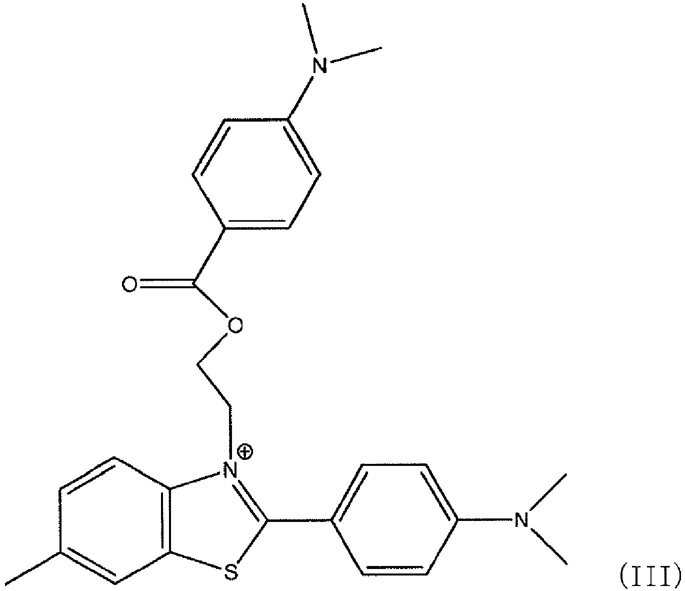

5. Nukleinsäurenachweiskit nach Anspruch 4, wobei das Guanin-Quadruplex-bindende Reagenz die folgende Verbindung ist: 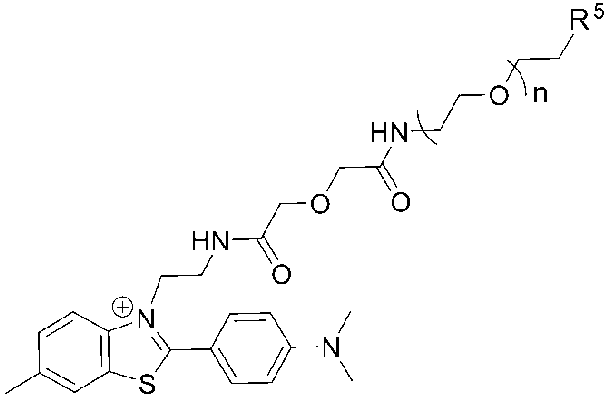
(wobei R⁵ eine Aminogruppe, eine Hydroxylgruppe, eine Alkylgruppe oder eine Carboxylgruppe darstellt und n eine ganze Zahl von 4 bis 50 darstellt), oder
wobei das Guanin-Quadruplex-bindende Reagenz die folgende Verbindung ist: 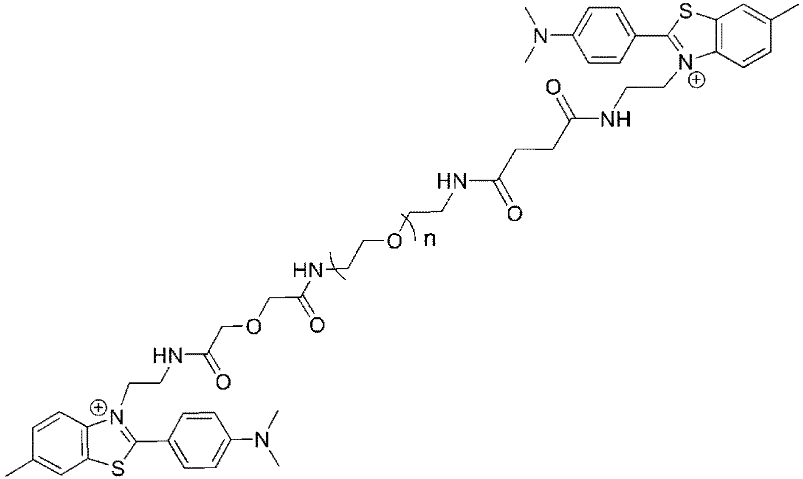
(wobei n eine ganze Zahl von 4 bis 50 darstellt).

6. Nukleinsäurenachweiskit nach Anspruch 5, wobei die Verbindung zusammen mit einer Polyethylenglykolkette auf einem Träger immobilisiert ist.

7. Nukleinsäurenachweiskit nach einem der vorhergehenden Ansprüche, umfassend einen Kronenether, wobei der Kronenether vorzugsweise 18-Krone-6 oder 15-Krone-5 ist.

8. Nukleinsäurenachweiskit nach einem der Ansprüche 1 bis 7, wobei das Nukleinsäurenachweiskit ein nichtionisches Tensid umfasst, wobei das nichtionische Tensid vorzugsweise Polyoxyethylensorbitanmonolaurat oder Octylphenolethoxylat ist.

9. Nukleinsäurenachweiskit nach einem der vorhergehenden Ansprüche, wobei die Zielnukleinsäure virale RNA ist.

10. Verfahren zum Nachweisen einer Zielnukleinsäure unter Verwendung des Kits nach einem der Ansprüche 1 bis 9, wobei das Verfahren die Schritte umfasst:
Hybridisieren der ersten einzelsträngigen zirkulären DNA und des ersten Oligonukleotid-Primers mit der Zielnukleinsäure;
Durchführen einer Nukleinsäureamplifikationsreaktion auf Grundlage der Zielnukleinsäure durch Rolling-Circle-Amplifikation von dem ersten Oligonukleotid-Primer;
Hybridisieren der zweiten einzelsträngigen zirkulären DNA und des zweiten Oligonukleotid-Primers mit dem erhaltenen Amplifikationsprodukt;
Durchführen einer Nukleinsäureamplifikationsreaktion auf Grundlage des Amplifikationsprodukts durch Rolling-Circle-Amplifikation von dem zweiten Oligonukleotid-Primer; und
Nachweisen einer amplifizierten Nukleinsäure.

11. Nukleinsäurenachweiskit, umfassend:
eine kurzkettige Zielnukleinsäure, die enthält:
eine erste Region; und
eine zweite Region auf der 3'-Seite der ersten Region, wobei die zweite Region eine Mutation enthält;
(i) eine erste einzelsträngige zirkuläre DNA, die enthält:
eine Region, die an die kurzkettige Zielnukleinsäure bindet, wobei die Region komplementär zu der zweiten Region der kurzkettigen Zielnukleinsäure ist;
eine zweite Region auf der 3'-Seite davon; und
eine Sequenz, die komplementär zu einer Sequenz ist, die an eine zweite einzelsträngige zirkuläre DNA bindet;
(ii) ein Fänger-Oligonukleotid, das enthält:
eine Matrizenbindungssequenz, die komplementär zu der zweiten Region der einzelsträngigen zirkulären DNA ist; und
eine Sequenz, die an die kurzkettige Zielnukleinsäure bindet, wobei die Sequenz komplementär zu der ersten Region der kurzkettigen Zielnukleinsäure ist;
(iii) eine zweite einzelsträngige zirkuläre DNA, die enthält:
dieselbe Sequenz wie die Sequenz in der ersten einzelsträngigen zirkulären DNA, die komplementär zu der Sequenz ist, die an die zweite einzelsträngige zirkuläre DNA bindet;
eine zweite Primer-Bindungssequenz, die an die 5'-Seite dieser Sequenz angrenzt; und
eine Sequenz, die komplementär zu einer nachweisreagenzbindenden Sequenz ist; und
(iv) einen zweiten Oligonukleotid-Primer, der enthält:
dieselbe Sequenz wie die Region in der ersten einzelsträngigen zirkulären DNA, die an die 5'-Seite der Sequenz angrenzt, die komplementär zu der Sequenz ist, die an die zweite einzelsträngige zirkuläre DNA bindet, wobei dieselbe Sequenz wie die Region in der ersten einzelsträngigen zirkulären DNA 8 bis 15 Basen ist; und
eine Sequenz von 7 bis 8 Basen, die an die 3'-Seite dieser Sequenz angrenzt und komplementär zu der zweiten Primer-Bindungssequenz der zweiten einzelsträngigen zirkulären DNA ist,
wobei der erste Fänger-Oligonukleotid über das 5'-Ende davon an einen Träger gebunden ist und der zweite Oligonukleotid-Primer über das 5'-Ende davon an den Träger gebunden ist, an den das erste Fänger-Oligonukleotid gebunden ist.

12. Verfahren zum Nachweisen einer kurzkettigen Zielnukleinsäure unter Verwendung des Kits nach Anspruch 11, wobei das Verfahren die Schritte umfasst:
Hybridisieren der ersten einzelsträngigen zirkulären DNA und des Fänger-Polynukleotids mit der kurzkettigen Zielnukleinsäure, die enthält: die erste Region; und die zweite Region, die an die 3'-Seite der ersten Region angrenzt und die Mutation enthält;
Durchführen einer Nukleinsäureamplifikationsreaktion durch Rolling-Circle-Amplifikation auf Grundlage der Ausbildung eines Komplexes aus der kurzkettigen Zielnukleinsäure, dem Fänger-Oligonukleotid und der ersten einzelsträngigen zirkulären DNA;
Hybridisieren der zweiten einzelsträngigen zirkulären DNA und des zweiten Oligonukleotid-Primers mit einer verlängerten Kette, die durch die Nukleinsäureamplifikationsreaktion erzeugt wurde, und
Durchführen einer Nukleinsäureamplifikationsreaktion auf Grundlage der Ausbildung eines Komplexes aus der verlängerten Kette, dem zweiten Primer und der zweiten einzelsträngigen zirkulären DNA; und
Nachweisen einer amplifizierten Nukleinsäure.

13. Kit zum Nachweisen eines Zielmoleküls, wobei das Kit umfasst:
(i) eine erste einzelsträngige zirkuläre DNA, die enthält:
eine erste Region;
eine zweite Region, die mit der 3'-Seite davon verbunden ist; und
eine Sequenz, die komplementär zu einer Sequenz ist, die an eine zweite einzelsträngige zirkuläre DNA bindet;
(ii) einen ersten Oligonukleotid-Primer, der enthält:
eine erste Aptamersequenz, die an das Zielmolekül bindet; und
eine Sequenz, die an der 3'-Seite davon gebunden ist und komplementär zu der ersten Region der ersten einzelsträngigen zirkulären DNA ist;
(iii) ein Fänger-Oligonukleotid, das enthält:
eine Sequenz, die komplementär zu der zweiten Region der ersten einzelsträngigen zirkulären DNA ist; und
eine zweite Aptamersequenz, die mit der 3'-Seite davon verbunden ist und an das Zielmolekül bindet,
(iv) eine zweite einzelsträngige zirkuläre DNA, die enthält:
dieselbe Sequenz wie die Sequenz in der ersten einzelsträngigen zirkulären DNA, die komplementär zu der Sequenz ist, die an die zweite einzelsträngige zirkuläre DNA bindet; und
eine Sequenz, die an die 5'-Seite dieser Sequenz angrenzt und die an einen zweiten Oligonukleotid-Primer bindet; und
(v) einen zweiten Oligonukleotid-Primer, der enthält:
dieselbe Sequenz wie die Region in der ersten einzelsträngigen zirkulären DNA, die an die 5'-Seite der Sequenz angrenzt, die komplementär zu der Sequenz ist, die an die zweite einzelsträngige zirkuläre DNA bindet, wobei dieselbe Sequenz wie die Stelle in der ersten einzelsträngigen zirkulären DNA 8 bis 15 Basen ist; und
eine Sequenz von 7 bis 8 Basen, die an die 3'-Seite dieser Sequenz angrenzt und komplementär zu der Sequenz in der zweiten einzelsträngigen zirkulären DNA ist, die an den zweiten Oligonukleotid-Primer bindet,
wobei das Fänger-Oligonukleotid und/oder der erste Oligonukleotid-Primer über das/die 5'-Ende(n) davon an einen Träger gebunden ist/sind, und der zweite Oligonukleotid-Primer über das 5'-Ende davon an den Träger gebunden ist, an den das Fänger-Oligonukleotid und/oder der erste Oligonukleotid-Primer gebunden ist.

14. Verfahren zum Nachweisen eines Zielmoleküls unter Verwendung des Kits nach Anspruch 13, wobei das Verfahren die Schritte umfasst:
Ausbildung eines ersten Komplexes, der das Zielmolekül, das Fänger-Oligonukleotid, den ersten Oligonukleotid-Primer und die erste einzelsträngige zirkuläre DNA enthält;
Durchführen einer Nukleinsäureamplifikationsreaktion durch Rolling-Circle-Amplifikation auf Grundlage der Ausbildung des ersten Komplexes;
Hybridisieren der zweiten einzelsträngigen zirkulären DNA und des zweiten Oligonukleotid-Primers mit einer verlängerten Kette, die durch die Nukleinsäureamplifikationsreaktion erzeugt wurde, um einen zweiten Komplex auszubilden, der die verlängerte Kette, den zweiten Oligonukleotid-Primer und die zweite einzelsträngige zirkuläre DNA enthält;
Durchführen einer Nukleinsäureamplifikationsreaktion durch Rolling-Circle-Amplifikation auf Grundlage der Ausbildung des zweiten Komplexes; und Nachweisen einer amplifizierten Nukleinsäure.

15. Verbindung, die durch die folgende Formel dargestellt wird: 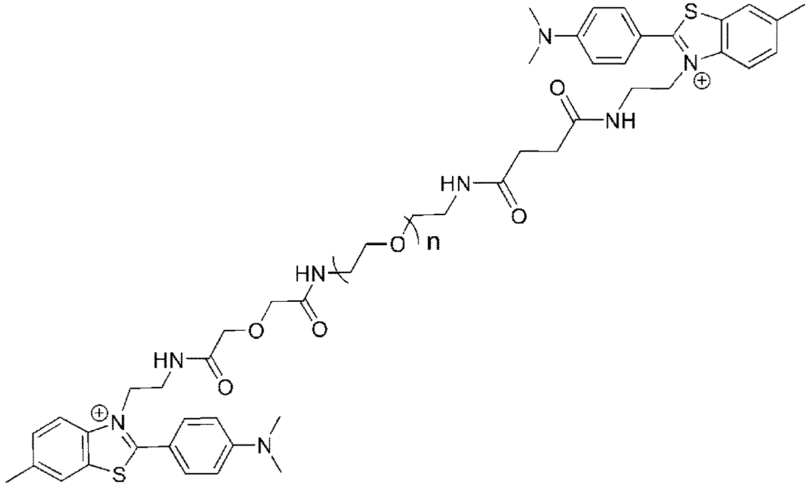 (wobei n eine ganze Zahl von 4 bis 50 darstellt).

## Revendications

1. Kit de détection d'acide nucléique comprenant :
(i) un premier ADN circulaire simple brin contenant :
une séquence de 10 à 30 bases complémentaire d'un premier site d'un acide nucléique cible ;
une première séquence de liaison d'amorce de 7 à 8 bases adjacente au côté 5' de cette séquence ; et
une séquence complémentaire d'une séquence qui se lie à un deuxième ADN circulaire simple brin ;
(ii) une première amorce oligonucléotidique contenant :
une séquence de 8 à 15 bases complémentaire d'un deuxième site adjacente au côté 3' du premier site de l'acide nucléique cible ; et
une séquence de 7 à 8 bases adjacente au côté 3' de cette séquence et complémentaire du premier site de liaison d'amorce du premier ADN circulaire simple brin ;
(iii) un deuxième ADN circulaire simple brin contenant :
la même séquence que la séquence dans le premier ADN circulaire simple brin, complémentaire de la séquence qui se lie au deuxième ADN circulaire simple brin ; et
une deuxième séquence de liaison d'amorce adjacente au côté 5' de cette séquence ; et
(iv) une deuxième amorce oligonucléotidique contenant :
la même séquence que le site dans le premier ADN circulaire simple brin, adjacente au côté 5' de la séquence complémentaire de la séquence qui se lie au deuxième ADN circulaire simple brin, dans lequel ladite même séquence que le site dans le premier ADN circulaire simple brin est 8 à 15 bases ; et
une séquence de 7 à 8 bases adjacente au côté 3' de cette séquence et complémentaire de la deuxième séquence de liaison d'amorce du deuxième ADN circulaire simple brin,
dans lequel la première amorce oligonucléotidique est liée à un support par l'intermédiaire de l'extrémité 5' de celle-ci, et la deuxième amorce oligonucléotidique est liée, par l'intermédiaire de l'extrémité 5' de celle-ci, au support auquel la première amorce oligonucléotidique est liée.

2. Kit de détection d'acide nucléique selon la revendication 1,
dans lequel
la première amorce oligonucléotidique est modifiée par une biotine à l'extrémité 5' de celle-ci, et liée, par la biotine, à un support sur lequel une avidine est immobilisée, et
la deuxième amorce oligonucléotidique est modifiée par une biotine à l'extrémité 5' de celle-ci, et liée, par la biotine, au support auquel la première amorce oligonucléotidique est liée, et/ou
dans lequel le rapport entre la première amorce oligonucléotidique et la deuxième amorce oligonucléotidique liée au support est 1:10 à 1:30 en termes de rapport molaire.

3. Kit de détection d'acide nucléique selon l'une quelconque des revendications 1 à 2, comprenant (v) un réactif de détection,
dans lequel le deuxième ADN circulaire simple brin contient une séquence complémentaire d'une séquence de liaison-réactif de détection, de préférence dans lequel la séquence de liaison-réactif de détection est une séquence de formation de guanine-quadruplex, et
le réactif de détection est un réactif de liaison de guanine-quadruplex, plus préférablement, dans lequel la séquence complémentaire de la séquence de formation de guanine-quadruplex contient une séquence C₃N₁₋₁₀C₃N₁₋₁₀C₃N₁₋₁₀C₃.

4. Kit de détection d'acide nucléique selon la revendication 3, dans lequel le réactif de liaison de guanine-quadruplex contient un composé représenté par la Formule générale (I) ci-après : 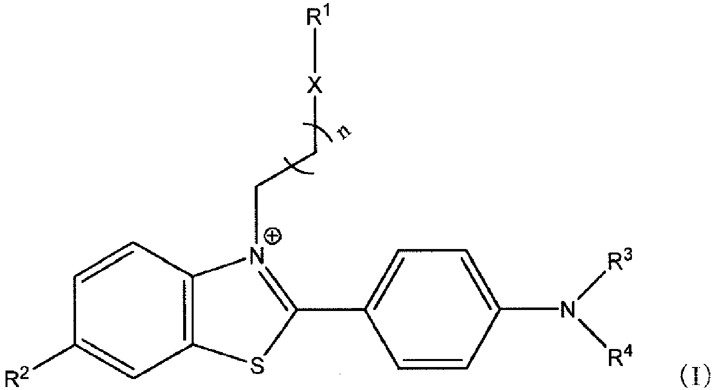 dans lequel
R¹ représente un hydrogène ou un groupe hydrocarboné qui contient en option un ou plusieurs éléments parmi O, S et N,
R², R³ et R⁴ représentent chacun indépendamment un groupe hydrocarboné en C₁-C₅ ;
n représente un nombre entier de 0 à 5, et
X représente O, S ou NH, de préférence
dans lequel le composé représenté par la Formule générale (I) est représenté par la Formule (II) ou (III) ci-après

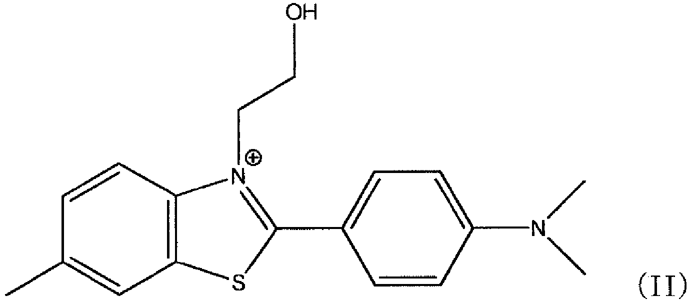

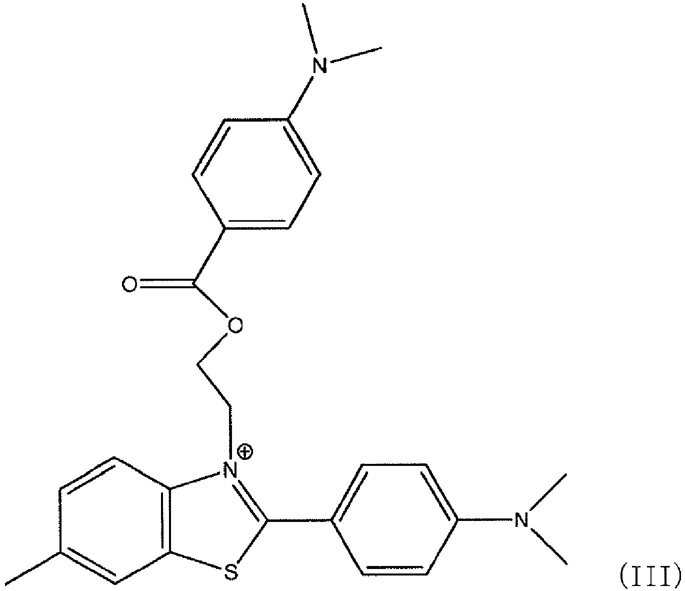

5. Kit de détection d'acide nucléique selon la revendication 4, dans lequel le réactif de liaison de guanine-quadruplex est le composé suivant : 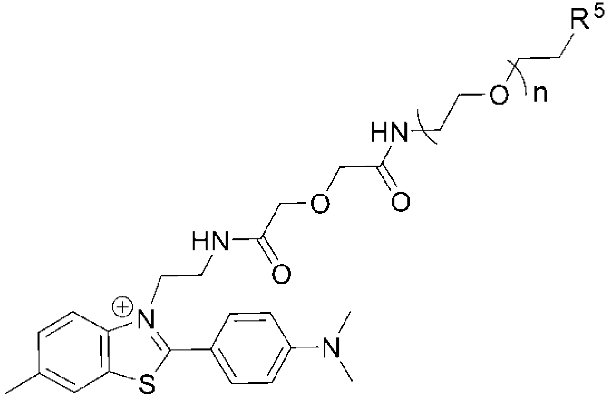
(dans lequel R⁵ représente un groupe amino, un groupe hydroxyle, un groupe alkyle ou un groupe carboxyle, et n représente un nombre entier de 4 à 50) ou
dans lequel le réactif de liaison de guanine-quadruplex est le composé suivant : 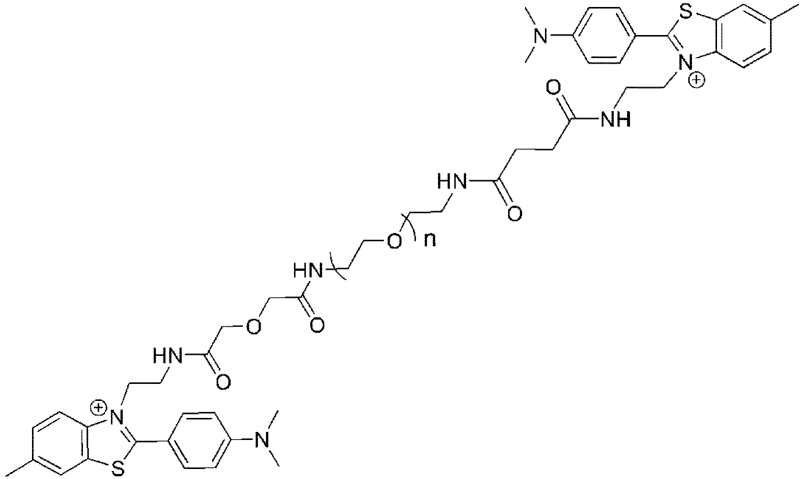
(dans lequel n représente un nombre entier de 4 à 50).

6. Kit de détection d'acide nucléique selon la revendication 5, dans lequel le composé est immobilisé sur un support avec une chaîne de polyéthylène glycol.

7. Kit de détection d'acide nucléique selon l'une quelconque des revendications précédentes, comprenant un éther couronne, de préférence dans lequel l'éther couronne est 18-couronne-6 ou 15-couronne-5.

8. Kit de détection d'acide nucléique selon l'une quelconque des revendications 1 à 7, dans lequel ledit kit de détection d'acide nucléique comprend un tensioactif non ionique, de préférence dans lequel le tensioactif non ionique est un monolaurate de sorbitane polyoxyéthylène ou un éthoxylate d'octylphénol.

9. Kit de détection d'acide nucléique selon l'une quelconque des revendications précédentes, dans lequel l'acide nucléique cible est un ARN viral.

10. Procédé de détection d'un acide nucléique cible utilisant le kit selon l'une quelconque des revendications 1 à 9, le procédé comprenant les étapes consistant à :
hybrider le premier ADN circulaire simple brin et la première amorce oligonucléotidique avec l'acide nucléique cible ;
effectuer une réaction d'amplification d'acide nucléique basée sur l'acide nucléique cible par amplification en cercle roulant depuis la première amorce oligonucléotidique ;
hybrider le deuxième ADN circulaire simple brin et la deuxième amorce oligonucléotidique avec le produit d'amplification obtenu ;
effectuer une réaction d'amplification d'acide nucléique basée sur la produit s par amplification en cercle roulant depuis la deuxième amorce oligonucléotidique ; et
détecter un acide nucléique amplifié.

11. Kit de détection d'acide nucléique comprenant :
un acide nucléique cible à chaîne courte contenant :
une première région ; et
une deuxième région dans le côté 3' de la première région, la deuxième région contenant une mutation ;
(i) un premier ADN circulaire simple brin contenant :
une région qui se lie à l'acide nucléique cible à chaîne courte, la région étant complémentaire de la deuxième région de l'acide nucléique cible à chaîne courte ;
une deuxième région dans le côté 3' de celle-ci ; et
une séquence complémentaire d'une séquence qui se lie à un deuxième ADN circulaire simple brin ;
(ii) un oligonucléotide de capture contenant :
une séquence de liaison de matrice complémentaire de la deuxième région de l'ADN circulaire simple brin ; et
une séquence qui se lie à l'acide nucléique cible à chaîne courte, la séquence étant complémentaire de la première région de l'acide nucléique cible à chaîne courte ;
(iii) un deuxième ADN circulaire simple brin contenant :
la même séquence que la séquence dans le premier ADN circulaire simple brin, complémentaire de la séquence qui se lie au deuxième ADN circulaire simple brin ;
une deuxième séquence de liaison d'amorce adjacente au côté 5' de cette séquence ; et
une séquence complémentaire d'une séquence de liaison-réactif de détection ; et
(iv) une deuxième amorce oligonucléotidique contenant :
la même séquence que la région dans le premier ADN circulaire simple brin, adjacente au côté 5' de la séquence complémentaire de la séquence qui se lie au deuxième ADN circulaire simple brin, dans lequel ladite même séquence que la région dans le premier ADN circulaire simple brin est 8 à 15 bases ; et
une séquence de 7 à 8 bases adjacente au côté 3' de cette séquence et complémentaire de la deuxième séquence de liaison d'amorce du deuxième ADN circulaire simple brin,
dans lequel l'oligonucléotide de capture est lié à un support par l'intermédiaire de l'extrémité 5' de celui-ci, et la deuxième amorce oligonucléotidique est liée, par l'intermédiaire de l'extrémité 5' de celle-ci, au support auquel l'oligonucléotide de capture est lié.

12. Procédé de détection d'un acide nucléique cible à chaîne courte utilisant le kit selon la revendication 11, le procédé comprenant les étapes consistant à :
hybrider le premier ADN circulaire simple brin et le polynucléotide de capture avec l'acide nucléique cible à chaîne courte contenant : la première région ; et la deuxième région adjacente au côté 3' de la première région et contenant la mutation ;
effectuer une réaction d'amplification d'acide nucléique par amplification en cercle roulant basée sur la formation d'un complexe de l'acide nucléique cible à chaîne courte, l'oligonucléotide de capture et le premier ADN circulaire simple brin ;
hybrider le deuxième ADN circulaire simple brin et la deuxième amorce oligonucléotidique avec une chaîne étendue générée par la réaction d'amplification d'acide nucléique, et
effectuer une réaction d'amplification d'acide nucléique basée sur la formation d'un complexe de la chaîne étendue, la deuxième amorce et du deuxième ADN circulaire simple brin ; et
détecter un acide nucléique amplifié.

13. Kit de détection d'une molécule cible, le kit comprenant :
(i) un premier ADN circulaire simple brin contenant :
une première région ;
une deuxième région liée au côté 3' de celle-ci ; et
une séquence complémentaire d'une séquence qui se lie à un deuxième ADN circulaire simple brin ;
(ii) une première amorce oligonucléotidique contenant :
une première séquence d'aptamère qui se lie à la molécule cible ; et
une séquence liée au côté 3' de celle-ci et complémentaire de la première région du premier ADN circulaire simple brin ;
(iii) un oligonucléotide de capture contenant :
une séquence complémentaire de la deuxième région du premier ADN circulaire simple brin ; et
une deuxième séquence d'aptamère liée au côté 3' de celle-ci, qui se lie à la molécule cible,
(iv) un deuxième ADN circulaire simple brin contenant :
la même séquence que la séquence dans le premier ADN circulaire simple brin, complémentaire de la séquence qui se lie au deuxième ADN circulaire simple brin ; et
une séquence qui est adjacente au côté 5' de cette séquence et qui se lie à une deuxième amorce oligonucléotidique ; et
(v) une deuxième amorce oligonucléotidique contenant :
la même séquence que la région dans le premier ADN circulaire simple brin, adjacente au côté 5' de la séquence complémentaire de la séquence qui se lie au deuxième ADN circulaire simple brin, dans lequel ladite même séquence que le site dans le premier ADN circulaire simple brin est 8 à 15 bases ; et
une séquence de 7 à 8 bases adjacente au côté 3' de cette séquence et complémentaire de la séquence dans le deuxième ADN circulaire simple brin, qui se lie à la deuxième amorce oligonucléotidique,
dans lequel l'oligonucléotide de capture et/ou la première amorce oligonucléotidique est/sont liés à un support par la ou les l'extrémités 5' de celle-ci, et la deuxième amorce oligonucléotidique est liée, par l'intermédiaire de l'extrémité 5' de celle-ci, au support auquel l'oligonucléotide de capture et/ou la première amorce oligonucléotidique est/sont liés.

14. Procédé de détection d'une molécule cible utilisant le kit selon la revendication 13, le procédé comprenant les étapes consistant à :
former un premier complexe contenant la molécule cible, l'oligonucléotide de capture, la première amorce oligonucléotidique et le premier ADN circulaire simple brin ;
effectuer une réaction d'amplification d'acide nucléique par amplification en cercle roulant basée sur la formation du premier complexe ;
hybrider le deuxième ADN circulaire simple brin et la deuxième amorce oligonucléotidique avec une chaîne étendue générée par la réaction d'amplification d'acide nucléique, pour former un deuxième complexe contenant la chaîne étendue, la deuxième amorce oligonucléotidique et le deuxième ADN circulaire simple brin ;
effectuer une réaction d'amplification d'acide nucléique par amplification en cercle roulant basée sur la formation du deuxième complexe ; et
détecter un acide nucléique amplifié.

15. Composé représenté par la formule suivante : 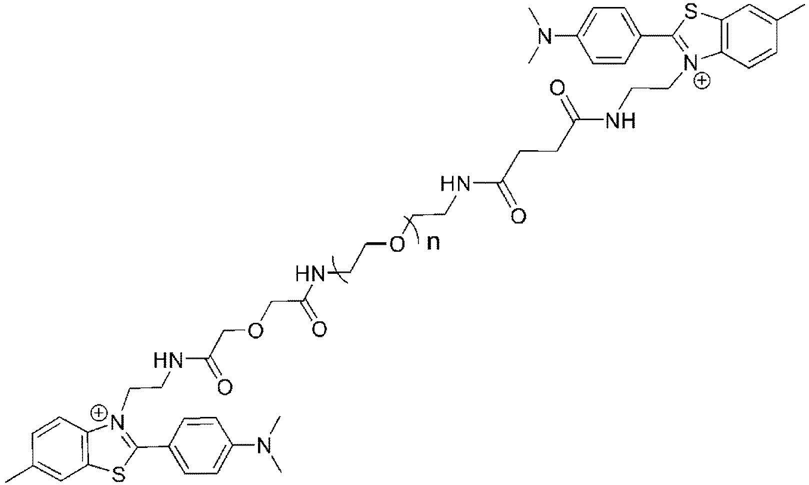 (dans lequel n représente un nombre entier de 4 à 50).
